(19) Europäisches Patentamt
European Patent Office
Office européen des brevets

(11) Numéro de publication: **0 005 659**
**B1**

(12) **FASCICULE DE BREVET EUROPEEN**

(45) Date de publication du fascicule de brevet: **25.11.81**

(51) Int. Cl.³: **A 61 K 7/46**

(21) Numéro de dépôt: **79400283.2**

(22) Date de dépôt: **03.05.79**

(54) **Procédé pour diluer des produits de parfumerie à l'aide de dimères d'alpha-méthylstyrène et/ou de dimères de terpènes, mélange pour la mise en oeuvre d'un tel procédé et composition de parfumerie ainsi diluée.**

(30) Priorité: **03.05.78 US 902436**
**03.05.78 US 902565**
**10.07.78 US 923183**

(43) Date de publication de la demande:
**28.11.79 Bulletin 79/24**

(45) Mention de la délivrance du brevet:
**25.11.81 Bulletin 81/47**

(84) Etats Contractants Désignés:
**AT BE CH DE FR GB IT LU NL SE**

(56) Documents cités:
**FR - A - 2 335 234**
**US - A - 3 502 769**

(73) Titulaire: **International Flavors & Fragrances Inc.**
**521 West 57th Street**
**New York, N.Y. 10019 (US)**

(72) Inventeur: **Wiegers, W.J.**
**72 Stephenville Bld**
**Red Bank New Jersey 07701 (US)**
Inventeur: **Hall J.B.**
**29 Buena Vista Avenue**
**Rumson New Jersey 07760 (US)**
Inventeur: **Hill I.D.**
**Clay Court**
**Locust New Jersey 07760 (US)**
Inventeur: **Novak R.M.**
**14 Oakland Avenue**
**Fords New Jersey 08863 (US)**
Inventeur: **Schmitt F.L.**
**29 McCampbell Road**
**Holmdel New Jersey 07733 (US)**
Inventeur: **Mookherjee, B.D.**
**46 Chestnut Ridge Road**
**Holmdel New Jersey 07733 (US)**
Inventeur: **Chi-Kuen Shu**
**305 Edgeview Road**
**Cliffwood New Jersey 07733 (US)**
Inventeur: **Schreiber, W.L.**
**33 Cambridge Drive**
**Jackson New Jersey 07733 (US)**

(74) Mandataire: **Rodhain, Claude**
**30, rue La Boétie**
**F-75008 Paris (FR)**

**EP 0 005 659 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé pour diluer des produits de parfumerie à l'aide de dimères d'alpha-méthylstyrène et/ou de dimères de terpènes, mélange pour la mise en oeuvre d'un tel procédé et composition de parfumerie ainsi diluée

L'invention concerne la découverte que l'on a faite que certains hydrocarbures liquides inodores, et spécialement: (I) les produits de la dimérisation de l'alpha-méthylstyrène, les produits de la dimérisation de ses homologues méthylés ou alcoylés par des alcoyles inférieurs en $C_2$ à $C_4$, et leurs dérivés hydrogénés, ou (II) les produits de la dimérisation (i) de terpènes monocycliques contenant deux doubles liaisons carbone-carbone, (ii) de terpènes bicycliques contenant une double liaison carbone-carbone et (iii) d'un terpène monocyclique contenant deux doubles liaisons carbone-carbone et d'un terpène bicyclique contenant une double liaison carbone-carbone, ou des mélanges de ces produits ou de produits d'hydrogénation de ceux-ci, ou encore des mélanges de ces produits de dimérisation et de ces produits d'hydrogénation, peuvent être utilisés comme diluants ou dilutifs pour étendre ou allonger différentes matières premières de parfumerie, sans que ces matières subissent une perte appréciable de leur effet odorant caractéristique.

Les compositions de parfumerie combinées contiennent un certain nombre d'ingrédients qui peuvent être d'origine naturelle ou synthétique. Les ingrédients sont mélangés dars la parfumerie pour créer l'effet odorant recherché. Les huiles essentielles qui contiennent des pourcentages élevés de constituants hydrocarburés, comme l'essence de patchouli (huile essentielle dérivée du Pogostemon Patchouli), ont par exemple de chaudes odeurs aromatiques épicées. Quand un parfumeur désire inclure ce type de note, par exemple dans und composition parfumée de type oriental, il utilise l'essence de patchouli. Toutefois les essences naturelles telles que l'essence de patchouli sont des huiles essentielles coûteuses et on ne peut les trouver qu'en quantités limitées. Des exemples encore plus extrêmes sont l'essence de bois de santal et l'essence de vétiver naturelles. Bien que des efforts aient faits pour reproduire l'odeur de l'essence de patchouli, de l'essence de bois de santal et de l'essence de vétiver en utilisant des mélanges de produits chimiques synthétiques de parfumerie, on n'a pas pu arriver à créer de telles essences possédant un aróme identique à celui des essences naturelles.

Dans le Brevet des Etats Unis d'Amérique n° 3 673 120 publié le 27 juin 1972, il est indiqué que le 8-camphène carbinol peut être utilisé comme dilutif de parfumerie pour l'essence de patchouli, dans des compositions de parfums, quand il est présent à la concentration de 1 à 200 parties en poids pour 100 parties en poids d'essence de patchouli. Toutefois, le 8-camphène carbinol à l'inconvénient de diminuer nettement l'intensité de l'arôme de l'essence de patchouli, et manque de souplesse pour l'utilisation avec des essences autres que celle de patchouli, par exemple l'essence de vétiver et l'essence de bois de santal dans le genre des essences naturelles, et avec les essences synthétiques, par exemple le géranonitrile et le cinnamonitrile.

Dans le Brevet des Etats Unis d'Amérique n° 2 422 145 publié le 10 juin 1947, on a trouvé que des éthers solubles à l'eau d'hydroxypolyéthylène d'esters partiels d'acides gras supérieurs de composés polyhydroxyliques à faible poids moléculaire pouvaient être utilisés pour former des solutions limpides allongées d'huiles essentielles que l'on peut utiliser telles que, ou que l'on peut diluer à l'eau pour former des dispersions ou des solutions stables d'huiles essentielles. On décrit particulièrement des compositions contenant des solutions limpides stables d'une quantité déterminée d'huile essentielle et d'une quantité au moins égale d'éthers tels que l'éther hydroxypolyéthylène de monopalmitate de mannitan avec environ 20 unités oxyéthylène par mole, cette solution étant capable, après dilution avec de l'eau de former une dispersion aqueuse limpide, stable, d'huile essentielle et d'éther d'hydroxypolyéthylène. Le Brevet des Etats Unis d'Amérique n° 2 422 145, toutefois, ne décrit pas la formation de solutions d'huiles essentielles dans des solvants organiques qui ne sont pas miscibles à l'eau. D'autre part, les éthers de ce Brevet diminuent nettement l'intensité des matières pour parfumerie que l'on utilise en conjonction avec eux.

On décrit comme susceptibles d'être utilisés comme "agents d'harmonisation de parfums", dans une demande japonaise publiée, sous le n° J 52136927, le 15 novembre 1977 au nom de Asahi Denka Kogyo, des esters d'acide dicarboxylique cyclohexane répondant à la formule générale:

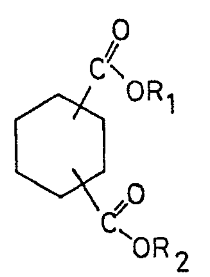

où $R_1$ et $R_2$ sont des groupements hydrocarbonés alicycliques ou aliphatiques à moins de 13 atomes de carbone. Toutefois, des matières telles que ces diesters d'acide dicarboxylique cyclohexane enlèvent de l'intensité aux matières pour parfums que l'on utilise avec eux.

Des procédés pour préparer des dimères de l'alpha-méthylstyrène et leurs homologues méthyle sont largement décrits dans la technique antérieure, par exemple:

Brevet français 1 317 412 délivré à Socony Mobil Oil Company en date du 8 février 1963;

Brevet U.S. 3 161 692 publié le 15 décembre 1964, délivré à Socony Mobil Oil Company;

Brevet japonais 78-21149 du 27 février 1978, basé sur une demande 76/94045 du 9 août 1976;

Brevet U.S.S.R. 191 511, publié le 26 janvier 1967;

Brevet U.S. 3 523 981 délivré à Olin Corporation, publié le 11 août 1970;

DT—OS 2 101 089 publiée le 10 août 1972;

Brevet U.S. 3 890 402 délivré à Phillips Petroleum Company, publié le 17 juin 1975;

Petropoulos and Fisher, J. Am. Chem. Soc. 80, 1938—41 (1958); et

Brevet U.S. 4 081 489 publié le 28 mars 1978.

Le Brevet des Etats Unis d'Amérique 4 081 489 décrit un procédé amélioré pour la production de composés répondant à la formule:

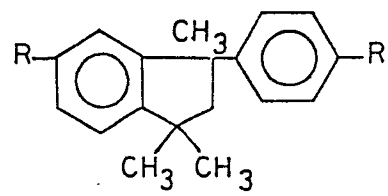

où R est, indépendamment, un hydrogène ou un méthyle, en mettant en contact un composé de la formula 1:

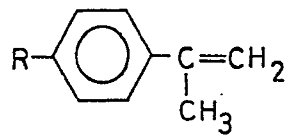 (I)

ou un mélange de composés de cette formule 1, où R est un hydrogène ou un méthyle, avec de l'acide sulfurique catalyseur à une température de 100 à 225°C, cette opération comprenant l'utilisation d'un catalyseur, constitué essentiellement d'environ 0,05 à 3% en poids, calculé sur le poids du composé ou du mélange de composés de formule 1, d'acide sulfurique concentré à 90 à 98%.

Le Brevet japonais 78/21149 décrit la production de dimères insaturés de l'alpha-méthylstyrène, par mise en contact d'alpha-méthylstyrène avec de la montmorillonite préchauffée à plus de 900°C pendant une durée d'environ 6 heures. On décrit aussi dans ce document japonais 78/21149 l'utilisation de bentonite comme catalyseur.

Rien dans la technique antérieure ne fait connaître l'utilisation de dimères de l'alpha-méthylstyrène, de leurs homologues méthylés ou de leurs dérivés hydrogénés comme diluants ou dilutifs dans les parfums. De plus, rien dans la technique antérieure ne donne d'indication sur l'utilisation des dérivés hydrogénés de ces dimères de l'alpha-méthylstyrène ou de leurs homologues méthylés.

Dans le Brevet des Etats Unis d'Amérique n° 3 415 893, publié le 10 décembre 1968, on a indiqué que l'on peut synthétiser l'essence de pin, matière bien connue pour son utilisation dans les techniques de la parfumerie, en agitant de l'alphapinène et de l'acide sulfurique aqueux contenant un émulsionnant, dans des conditions de température contrôlées, jusqu'à ce que la teneur en alcools terpèniques atteigne un maximum. Les phases huileuse et aqueuse sont ensuite, dans ce Brevet des Etats Unis d'Amérique n° 3 415 893, séparées et la phase huile est lavée avec de l'eau contenant une matière basique pour neutraliser tout ce qui resterait d'acide résiduel. On distille ensuite la phase huile pour séparer l'essence de pin de 1' "alpha-pinène qui n'a pas réagi et des autres terpènes, s'il s'en trouve, ainsi que des autres sous-produits de la réaction". Il est indiqué que les sous-produits sont principalement des hydrocarbures monocycliques, contenant certains cinéols, des éthers cycliques et autres produits indésirables de la réaction, et que cette portion des sous-produits est un solvant utilisable. Rien dans le Brevet des Etats Unis d'Amérique n° 3 415 893 ne décrit la possibilité d'utiliser les mélanges de diterpènes-dimères de l'alphaméthylstyrène de l'invention, ni leurs avantages comme dilutifs dans la parfumerie.

Les diterpènes et les diterpènes hydrogénés, résultant de l'action de différents acides sur les monoterpènes ont été étudiés par différents chercheurs depuis la découverte, il y a plus d'un siècle, du dipinène, par Deville, Ann. Chim. Phys. (2) *75,* 66 (1840) et Ann. Chim. *37,* 192 (1840), qui obtenait le dipinène à partir de l'essence de térébenthine et de l'acide sulfurique. Ces expériences de Deville ont été reprises, en ce qui concerne la littérature des dipinènes synthétiques en général, par Dulou, Chimie et Industrie, 27 (numéro spécial) 651 (1932), où l'on dit que l'on peut produire des composés ayant les structures de dipinène, indiquées comme étant:

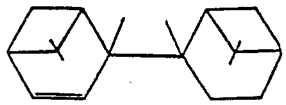 et 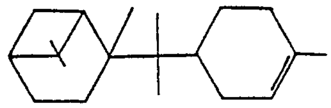

à partir d'alpha-pinène ayant la structure: 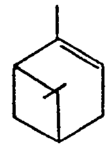

Le di-alpha-pinène a aussi été produit par Kuwata, J. Faculty Eng. Tokyo Imp. Univ., 18 117—24 (1929) par l'action d'une argile acide japonaise sur le d-alpha-pinène. Dans une réaction en deux étapes, Venable, J. Am. Chem. Soc. *45*, 728—34 (1923), traite l'alpha-pinène avec de la terre à foulon, l'amenant d'abord à subir un réarrangement moléculaire, et provoquant ensuite une polymérisation du dipinène. Kuwata, dans le J. Soc. Chem. Ind. Japan 36, volume supplé. 256—8 (1933) (résumé dans Chem. Abstracts 27: 3927) décrit le traitement du camphène en solution benzénique avec une argile acide japonaise donnant un dimère. On décrit aussi la production des dimères du camphène dans le Japanese Kokai 73 92 355 du 30 novembre 1973, où l'on fait passer le camphène sur une résine échangeuse de cations fortement acide, sous 1 kg/cm$^2$ et sous vide, à une température qui ne dépasse pas 130°C, pour produire des dimères et trimères du camphène. Le Japanese Kokai 73 92, 355 (demande de Brevet n° 27686/72) contient les revendications suivantes:

"Procédé pour la fabrication d'oligomères du camphène par polymérisation du camphène, en dessous de 130°C, et à la pression atmosphérique ou sous pression réduite, avec catalyse par des résines échangeuses d'ions du type fortement acide."

De plus ce Japanese Kokai 73 92 355 contient aussi l'affirmation afférente suivante:

"Grâce à cette invention, les oligomères produits sont éliminés du système réactionnel, supprimant en conséquence les risques de réactions secondaires telles qu'isomérisation, hydrolyse, déshydrogénation et oxydation. Les produits peuvent être obtenus avec un rendement élevé sans tétramères ni oligomères plus élevés, et sont des produits incolores et inodores qui conviennent pour être utilisés dans des parfums, cosmétiques et additifs alimentaires".

Les propriétés spécifiques des dimères du camphène et leur utilité dans les conditions décrites ici, comme dilutifs et diluants dans la parfumerie ne sont toutefois pas décrites dans le Japanese Kokai 73 92 355.

Il est indiqué que l'on peut préparer le dimère du limonène à partir du d-limonène, dans Beilstein V. 509, page 246 (N° 9). La présence du dimère du limonène est indiquée comme existant dans l'essence de Dictamnus Hispanicus dans Chem. Abstracts 45 : 5880 (Résumé de "The Essence of Dictamnus Hispanicus", J. Sistare (Inst. "Alonso Barba" Barcelona, Spain) Anales Real Soc. Espan. Fis. Ey. Quim.47B, 171—4 (1951).

Dans le Brevet des Etats Unis d'Amérique n° 2 249 112, publié le 15 juillet 1941, on indique que l'on peut utiliser les polymères hydrogénés du pinène pour leurs "caractéristiques de solubilité-viscosité" qui les rendent utilisables comme produits d'imprégnation, matières adhésives et comme véhicules pour des peintures métalliques". Ils sont aussi indiqués pour entrer en compétition avec de nombreuses essences minérales, et peuvent être mélangés avec du caoutchouc pour produire des compositions collantes élastiques. Les polymères hydrogénés du pinène dont on indique qu'ils peuvent être produits dans le Brevet des Etats Unis d'Amérique n° 2 249 112, sont produits suivant le schéma de réactions suivant:

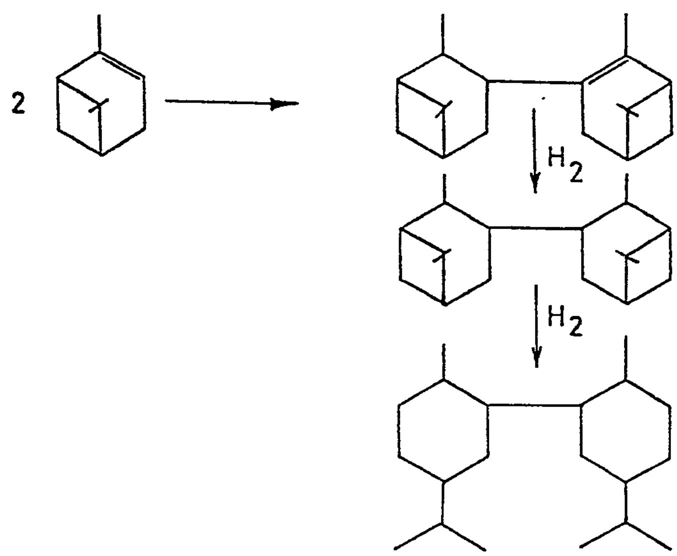

et sont considérés comme ayant les structures:

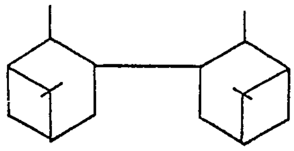

et

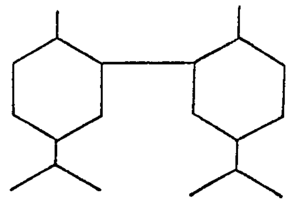

On ne dit pas que les polymères hydrogénés du terpène du Brevet des Etats Unis d'Amérique n° 2 249 112 soient utilisables comme dilutifs pour parfums, et ils ne sont pas indiqués comme ayant, en conjonction avec certaines huiles essentielles pour parfums, les propriétés qui sont décrites et revendiquées dans la présente demande.

Les dilutifs pour parfums ont été largement utilisés comme "adultérants" dans la technique de la parfumerie. Ainsi, par exemple, dans le texte intitulé "The Art of Parfumery and Method of Obtaining the Odors of Plants", dont l'auteur est Piesse (Lindsay and Blakiston Publishers, Philadelphia, 1856), la térébenthine et l'essence de spic sont indiquées à la page 255 comme étant des adultérants de l'essence de lavande. Dans Poucher, "Perfumes and Cosmetics", Van Nostrand Company 1923, les résidus terpèniques obtenus au cours de la fabrication d'essence de limon concentrée sont indiqués comme étant des adultérants de l'essence de limon. Dans le volume 1 de "The Essentials Oils", Günther, Krieger Publishing Company 1975, l'acétate de terpinyle et l'essence de térébenthine (contenant du d-alpha-pinène sont indiquées comme pouvant être utilisés comme adultérants. Dans le volume 2 de "The Essentials Oils", le camphorène, qui est un diterpène est indiqué comme étant largement appliqué comme fixatifs dans l'expédition des savons. L'alpha-camphorène a la structure suivante:

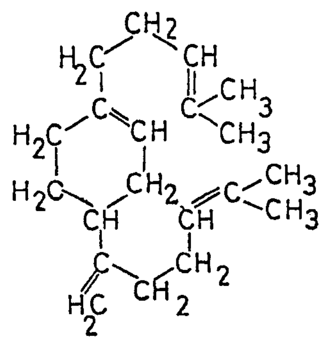

Le Brevet des Etats Unis d'Amérique n° 3 502 769, délivré le 24 mars 1970 (Fukuhara) décrit une préparation pour la toilette contenant une quantité, suffisante pour la stabilité au magasinage, d'un hydrocarbure qui peut être un terpène polymère monocyclique hydrogéné, répondant à la formule $(C_{10}H_{19})_n$ et/ou un terpène polymère bicyclique hydrogéné, répondant à la formule $(C_{10}H_{17})_n$, où n est un nombre entier de 2 à 4, et une base cosmétique, telle qu'un cold-cream ou une base de crème démaquillante. Dans la colonne 2 du Brevet des Etats Unis d'Amérique n° 3 502 769, à la ligne 15, il est indiqué que les terpènes hydrogénés sont produits en partant d'hydrocarbures terpéniques alicycliques. Il est en outre indiqué que, par exemple, les terpènes monocycliques tels que les menthadiènes, (par exemple alpha-terpinène, gamma-terpinène, alpha-phellandrène, bêta-phellandrène, terpinolène, limonène, etc.) et les terpènes bicycliques tels que les camphènes sont d'abord polymérisés de façon à former des composés répondant à la formule $(C_{10}H_{16})_n$, où n est un nombre entier positif entre 2 et 4. Il est en outre spécifié que ces polymères sont, soit des dimères, soit des trimères, soit des tétramères, et que les polymères résultants sont ensuite complètement hydrogenés, et dans le cas des menthadiènes, forment des polymères monocycliques répondant à la formule $(C_{10}H_{19})_n$, où n est un nombre entier de 2 à 4, et dans le cas de terpènes bicycliques, il est indiqué qu'il se forme des polymères bicycliques répondant à la formule $(C_{10}H_{17})_n$, où n est un nombre entier de 2 à 4. A titre d'exemple, on a constaté que si l'on utilise un dipentène comme produit de départ, il peut se produire la succession de réactions suivantes:

(1) 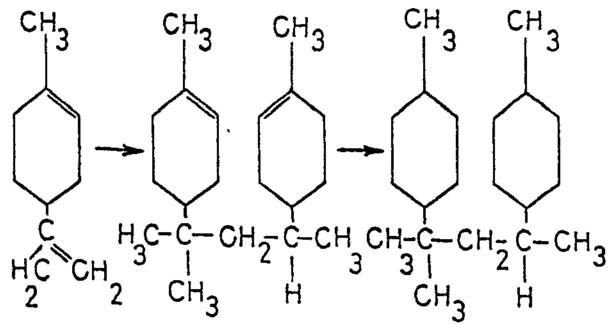

Di-pentène Dimère Dimère réduit

(2) 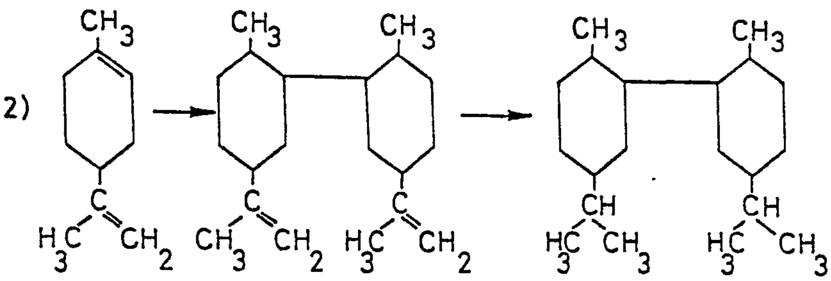

Di-pentène Dimère Dimère réduit

Bien qu'il soit ainsi montré que des parfums peuvent être utilisés en association avec des terpènes polymères hydrogénés, qui sont en outre utilisés aux fins d'assurer la stabilisation au magasinage, il n'est aucunement dit, dans ce Brevet des Etats Unis d'Amérique n° 3 502 769, que, dans les proportions indiquées, les mélanges de (I), ces produits de la dimérisation des terpènes comme les produits de la dimérisation du camphène, de l'alpha-pinène, ou du bêtapinène, et (II) de produits de la dimérisation de l'alpha-méthylstyrène suivant l'invention peuvent être utilisés dans les conditions décrites ici, comme dilutifs et diluants dans la parfumerie.

Plyusnin et al., dans U.S.S.R. Journal Of Applied Chemistry, 29, 1363 à 1367, décrivent aussi la polymérisation de terpènes isolés (alpha-pinène, dipentène et camphène), en présence de fluorures d'hydrogène, mais ne parlent pas de l'utilisation du mélange suivant l'invention, de la manière décrite ici, comme dilutifs et diluants dans la parfumerie.

Ainsi, il n'est nullement suggéré, dans la technique antérieure que des mélanges de (I), ces produits de la dimérisation des terpènes tels que les produits de la dimérisation du camphène ou de l'alpha-pinène ou du bêta-pinène et (II) de dimères de l'alpha-méthylstyrène suivant l'invention peuvent être utilisés de la manière décrite ici comme dilutifs et diluants dans la parfumerie.

On a été surpris de constater que soit (I) en variante les produits séparés, soit (II) des mélanges de (A) produits de la dimérisation de (i) des terpènes monocycliques contenant deux doubles liaisons carbone-carbone, (ii) des terpènes bicycliques contenant une double liaison carbone-carbone, et (iii) un terpène monocyclique contenant deux doubles liaisons carbone-carbone et un terpène bicyclique contenant une double liaison carbon-carbone, ou leurs mélanges, ou des mélanges des produits de leur hydrogénation et des produits de leur dimérisation et/ou (B) de produits de la dimérisation de l'alpha-méthylstyrène, ou de ses homologues méthylés ou alcoylés inférieurs en $C_2$ à $C_4$ et leurs dérivés hydrogénés, peuvent être utilisés comme diluants ou dilutifs de différentes matières premières de parfumerie sans que ces dernières subissent une perte appréciable de l'effet de leur caractéristique d'odeur.

Les produits (A) de la dimérisation sont produits en dimérisant des composés tels que les camphènes présentant la structure:

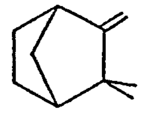

ou de l'alpha-pinène ayant la structure:

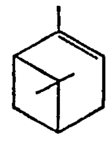

ou du d-limonène ayant la structure:

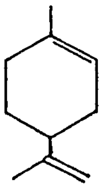

ou de l'alpha-phellandrène ayant la structure:

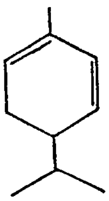

ou du gamma-terpinène ayant la structure:

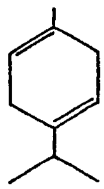

ou du delta $^{3}$-carène ayant la structure:

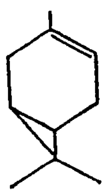

ou du bêta-phellandrène ayant la structure:

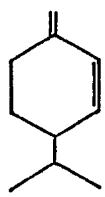

ou du bêta-terpinène ayant la structure:

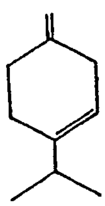

ou de l'alpha-terpinène ayant la structure:

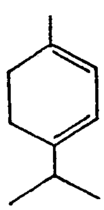

ou du terpinolène ayant la structure:

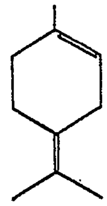

ou du bêta-pinène ayant la structure:

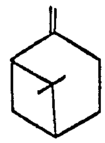

ou en "dimérisant" des mélanges de deux de ces composés ou plus, tels que par exemple un mélange de terpènes en $C_{10}$, connu habituellement sous la désignation de "térébenthine de pâte au sulfate", ou en "dimérisant" un terpène en $C_{10}$, et un terpène déshydrogéné (par exemple le cymène) en présence de catalyseurs acides, tels que l'acide sulfurique et l'acide fluorhydrique, ou en présence de catalyseurs faits d'une argile acide telle que l'argile acide japonaise ou la terre à foulon, ou des résines échangeuses de cations pouvant servir de catalyseurs. Les produits de cette "dimérisation" sont des composés qui peuvent avoir des structures telles que:

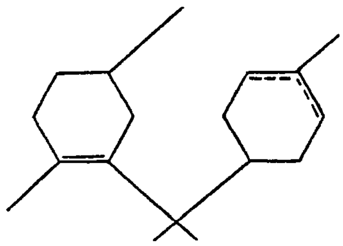

et des produits d'une hydrogénation qui peuvent avoir une structure telle que:

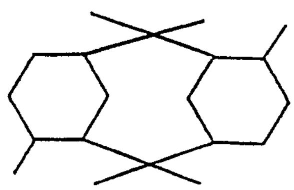

Pour autant qu'il s'agisse de produits de la dimérisation, (A) les termes "produits de la dimérisation" et "dimère" se proposent de désigner des produits de réaction contenant 20 atomes de carbone, résultant de la réaction d'un composé terpénique à 10 atomes de carbone (monocyclique avec deux doubles liaisons carbone-carbone, ou bicyclique avec une double liaison carbone-carbone) avec lui-même ou avec un autre composé terpénique qui sera monocyclique ou bicyclique, sans que l'on tienne compte du nombre d'atomes d'hydrogène contenus dans une molécule quelconque de ces produits de réaction.

Les produits (B) de la dimérisation sont obtenus en dimérisant l'alpha-méthylstyrène ayant la structure:

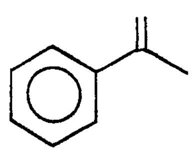

ou en dimérisant un de ses homologues méthylés ou alcoylés inférieurs en $C_2$ à $C_4$, ayant, par exemple la structure:

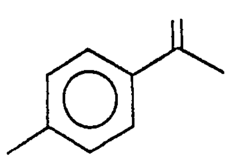

en présence d'acide de Lewis, d'acide de Bronstedt catalyseurs tels que l'acide sulfurique, ou en présence d'argiles acides catalyseurs, telles que l'argile acide japonaise ou la terre à foulon, ou de résines échangeuses de cations catalyseurs. Les produits de la dimérisation de l'alpha-méthylstyrène que l'on peut ainsi utiliser ont les structures:

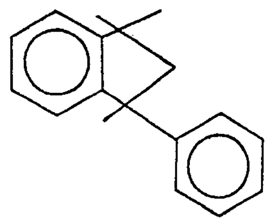

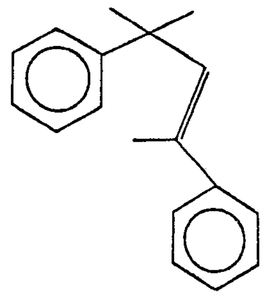

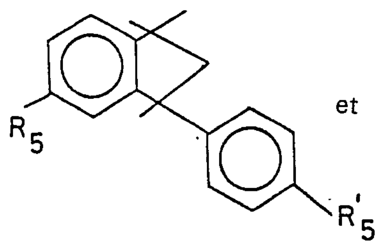

et

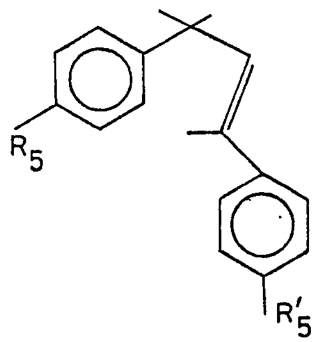

où l'un des deux groupes $R_5$ et $R'_5$ est un méthyle ou un autre alcoyle inférieur en $C_2$ à $C_4$ et l'autre est un hydrogène, ou $R_5$ et $R'_5$ sont tous deux des alcoyles inférieurs en $C_1$ à $C_4$, semblables ou différents, méthyle par exemple. Les produits d'hydrogénation ont les structures:

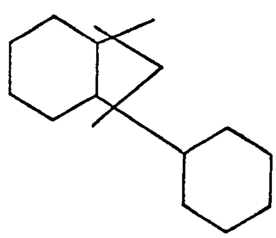

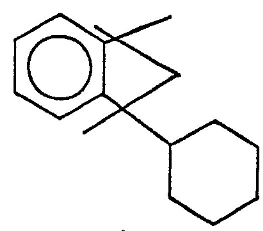

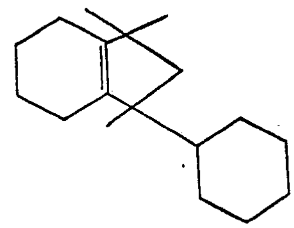

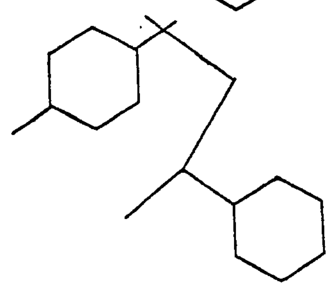

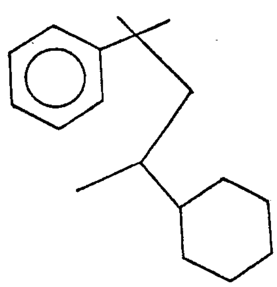

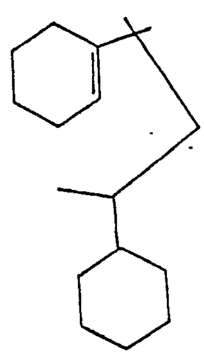

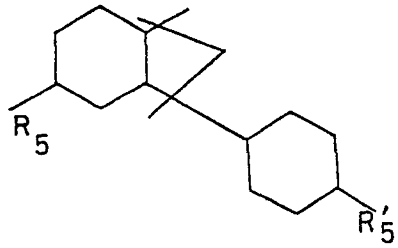

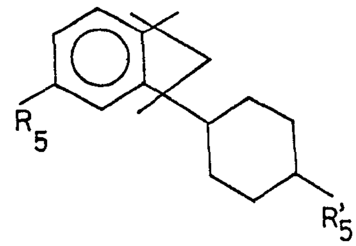

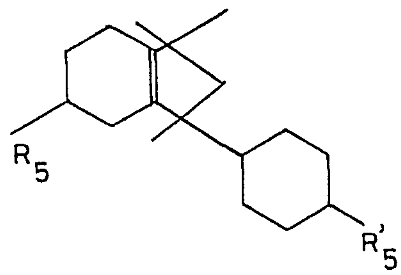

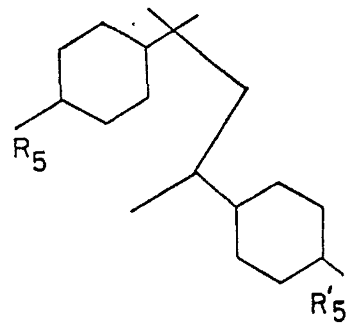

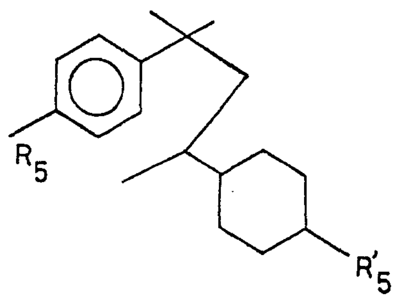

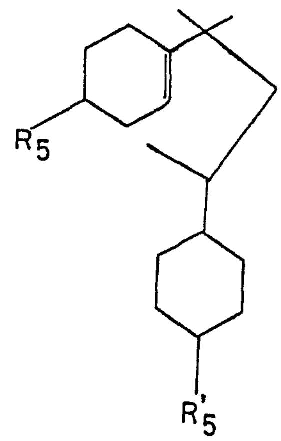

où l'un des deux groupes $R_5$ et $R'_5$ est un méthyle et ou un autre alcoyle inférieur en $C_2$ à $C_4$ et l'autre est un hydrogène, ou $R_5$ et $R'_5$ sont tous deux des alcoyles inférieurs en $C_1$ à $C_4$ semblables ou différents, méthyle par exemple. Ces produits d'hydrogénation peuvent être représentés par les structures génériques:

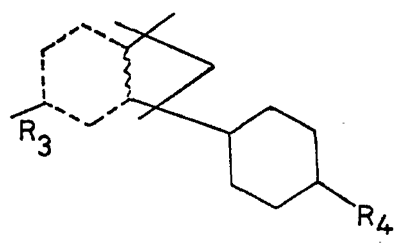

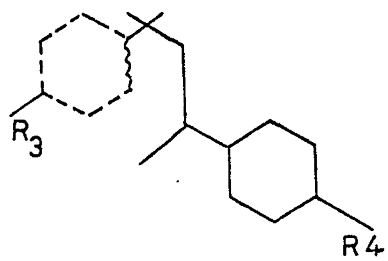

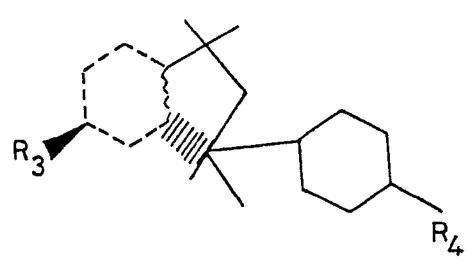

où $R_3$ et $R_4$ sont semblables ou différents et représentent un hydrogène ou un méthyle, ou un autre alcoyle inférieur en $C_2$ à $C_4$, les lignes en tirets et la ligne ondulée représentent des liaisons uniques

carbone-carbone, ou des doubles liaisons carbone-carbone, avec cette condition que, s'il se présente une double liaison, seule la ligne ondulée est une double liaison, et s'il existe plus d'une double liaison, le cycle contenant les lignes en tirets et la ligne ondulée est un cycle benzénique où la ligne ⦙⦙⦙⦙ représente soit une simple liaison carbone-carbone ou aucune liaison.

Dans le cas des produits d'hydrogénation, quand $R_3$ et/ou $R_4$ sont des alcoyles inférieurs, méthyle par exemple, les groupes méthyle peuvent se trouver en cis ou en trans l'un par rapport à l'autre et par rapport aux groupements cyclohexyl.

Une importante propriété des mélanges ci-dessus de produits de dimérisation et de produits de dimérisation hydrogénés est qu'ils présentent un large éventail de solubilité pour différents types de matières pour parfumerie y compris une solubilité totale pour certains alcools, esters, pyranes, aldéhydes, cétones, éthers cycliques, amines cycliques, nitriles et essences naturelles. Ainsi par exemple les matières suivantes sont complètement miscibles avec les dimères qui font l'objet de l'invention:

Alcool de di-phényl éthyl
Géraniol
Terpinéol
Acétate de citronellyl
Acétate de dl-décyle
Oxyde rose
n-décanal
Citral
Alpha-ionone
Eugénol
Galaxolide
Acide 2-méthyl-2-penténoïque
Isobutyl quinoline
Essence de limon
Essence de romarin
Essence de patchouli
Cinnamonitrile
Géranonitrile

Ainsi, on a constaté que les dimères suivant l'invention peuvent être utilisés pour remplacer patiellement certaines huiles essentielles ou leurs substituts synthétiques, dans des compositions pour parfumerie constituées par une phase liquide unique.

En conséquence, l'invention comprend une composition constituée d'une phase liquide unique, qui contient une ou plusieurs essences parfumées synthétiques ou essences parfumées naturelles, ou des mélanges d'essences parfumées naturelles et d'essences parfumées synthétiques, à laquelle on a incorporé de 1 à 30 parties en poids environ, soit (i) en variante ces produits séparés, soit (ii) en mélange, un mélange de (A) un produit de dimérisation d'un alpha-méthylstyrène, ou d'un homologue alpha-méthylstyrèhe méthylé ou autrement alcoylé inférieur en $C_2$ à $C_4$, ou un ou plusieurs de leurs dérivés hydrogénés qui présentent au moins une des structures suivantés:

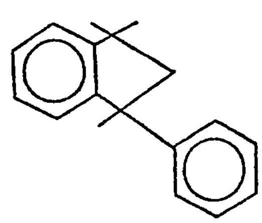

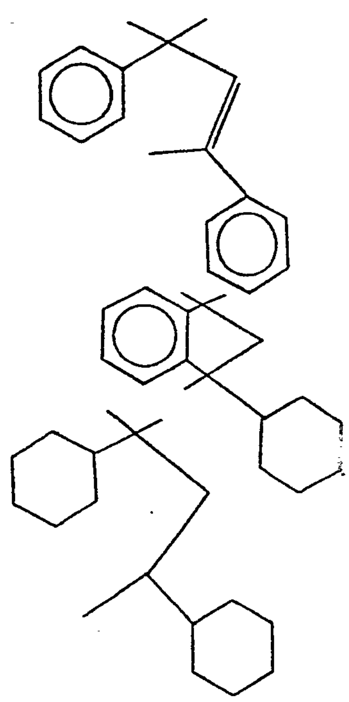

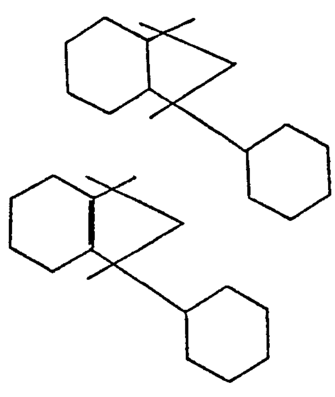

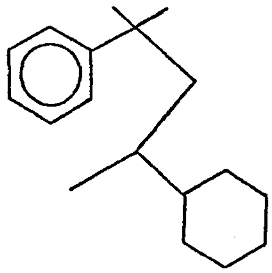

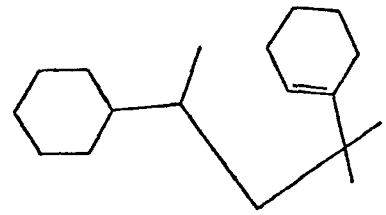

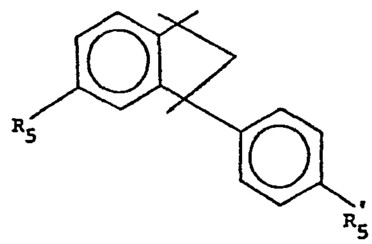

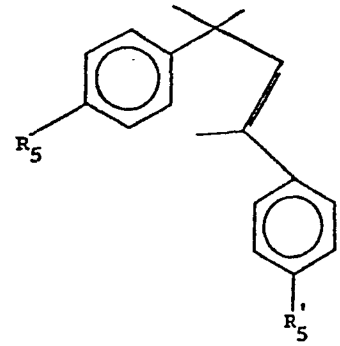

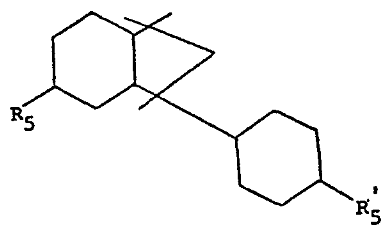

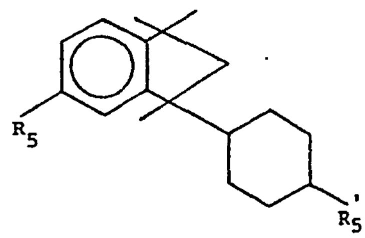

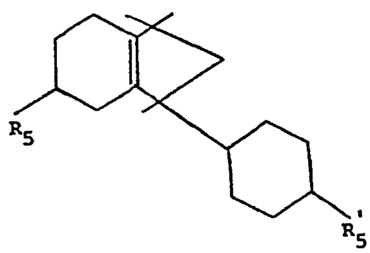

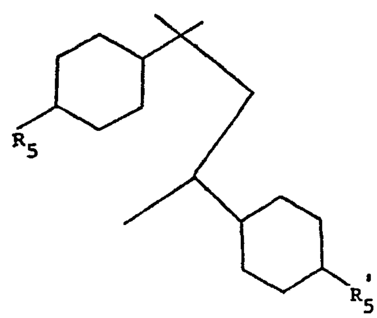

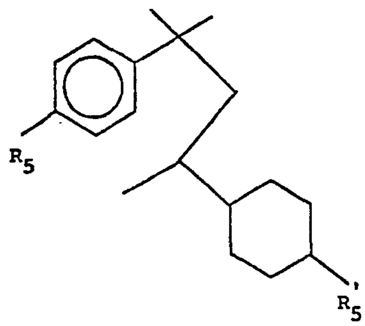

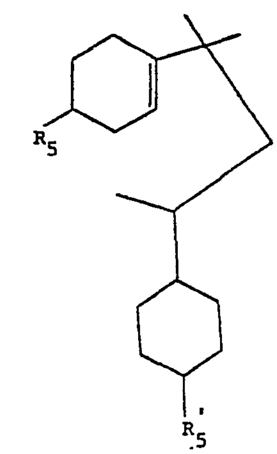

où un de $R_5$ et $R'_5$, ou tous les deux sont constitués par un méthyle ou un autre alcoyle inférieur en $C_2$ à $C_4$. Ces composés etant représentés collectivement par les structures génériques:

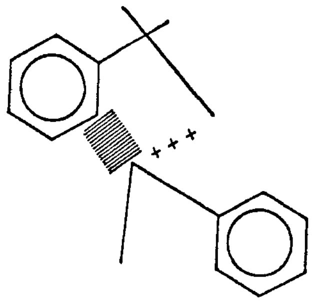

et

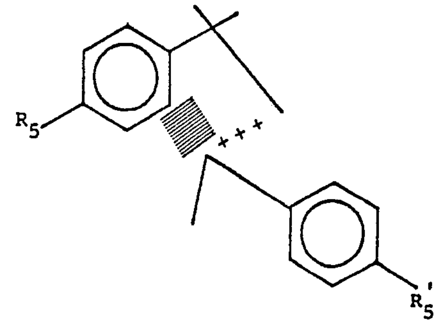

dans le cas de produits de la dimérisation de l'alpha-méthylstyrène et de produits de la dimérisation de ses homologues méthylés ou autrement alcoylés inférieurs en $C_2$ à $C_4$, et les structures:

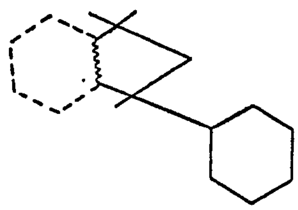

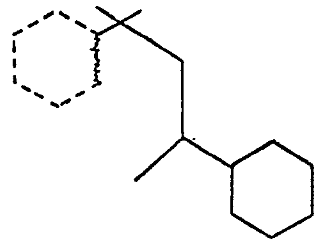

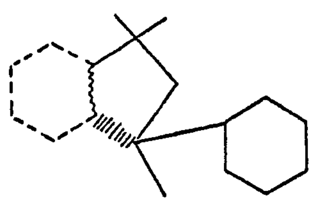

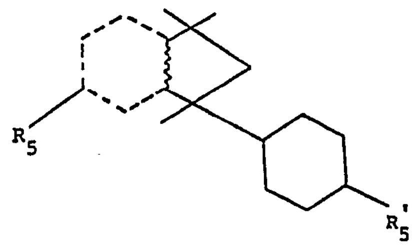

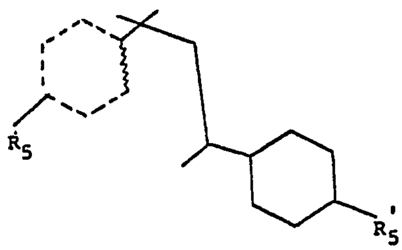

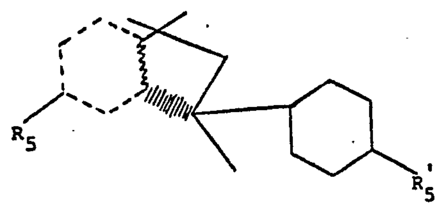

où l'un de $R_5$ et $R'_5$ est un méthyle ou un autre alcoyle inférieur en $C_2$ à $C_4$ et l'autre est un hydrogène, ou $R_5$ et $R'_5$ sont tous deux un méthyle ou un autre alcoyle inférieur en $C_2$ à $C_4$, s'il s'agit de leurs dérivés hydrogénés, dans lesquelles les lignes en tirets et les lignes ondulées représentent des simples liaisons carbone-carbone ou des doubles liaisons carbone-carbone, avec cette condition que s'il existe une double liaison dans le cycle contenant les lignes en tirets et les lignes ondulées, seule la ligne ondulée est une double liaison, et s'il existe plus d'une double liaison, le cycle contenant les lignes en tirets et les lignes ondulées et un noyau benzénique, et la ligne |||||||||| représente soit une simple liaison carbone-carbone, soit aucune liaison, et dans lesquelles la ligne ++++++ est représente soit une simple liaison carbone-carbone, soit une double liaison carbone-carbone, avec cette condition que si la ligne ++++++ est une double liaison carbone-carbone, la ligne |||||||||| n'est pas une liaison, et si la ligne +++++ est une simple liaison carbone-carbone, la ligne |||||||||| est une simple liaison carbone-carbone, et (B) un produit de la dimérisation de (I) un terpène monocyclique contenant deux doubles liaisons carbone-carbone ou (II) un terpène bicyclique contenant une double liaison carbone-carbone ou (III) un produit de la réaction d'un terpène monocyclique contenant deux doubles liaisons carbone-carbone et d'un terpène bicyclique contenant une double liaison carbone-carbone et/ou des dérivés hydrogénés de ces prcduits pour 100 parties de la composition de parfumerie constituée d'une unique phase liquide.

Comme exemples spécifiques de tels produits de la dimérisation et de leurs dérivés hydrogénés, que l'on peut utiliser dans la pratique de l'invention, on peut citer:

Produits de la dimérisation de l'alpha-pinène;
Produits de la dimérisation de la bêta-pinène;
Produits de la dimérisation de la camphène;

Produits de la dimérisation de la d-limonène;

Produits de la dimérisation de la gamma-terpinène;
Produits de la dimérisation de l'alpha-phellandrène;
Produits de la dimérisation du $\Delta^3$-carène;
Produits de la dimérisation de la bêta-phellandrène;
Produits de la dimérisation de la terpinolène;
Produits mixtes de la dimérisation de l'alpha-phellandrène et de $\Delta^3$-carène où un mélange de ces deux produits est soumis à une réaction de dimérisation;
Produits mixtes de la dimérisation de l'alpha-phellandrène et de la gamma-terpinène;
Produits mixtes de la dimérisation de la gamma-terpinène et de la bêta-phellandrène;
Produits mixtes de la dimérisation des alpha-pinène, bêta-pinène et camphène;
Produits mixtes de la dimérisation des alpha-pinene et $\Delta^3$-carène;
Produits mixtes de la dimérisation de la térébenthine de pâte au sulfate;
Produits mixtes de la dimérisation du terpinolène et de la gamma-terpinène;
Dérivés hydrogénés d'un quelconque des produits ci-dessus ou de leurs mélanges.

Les produits de la dimérisation (A) et/ou (B) et/ou leurs dérivés hydrogénés pris seuls ou ensemble, ne contiennent en eux-mêmes aucune odeur, et aucun d'eux ne peut par lui même causer aucune altération de l'odeur d'une quelconque des matières pour parfumerie auxquelles on les ajoute.

Les produits de dimérisation indiqués ci-dessus, leurs dérivés hydrogénés et leurs mélanges, peuvent être produits suivant l'un quelconque des procédés connus dans la technique antérieure. Ainsi, on donne ci-dessous un schéma de réaction par lequel on peut produire un produit de dimérisation (B) utilisable dans l'invention et par lequel on peut produire ses dérivés hydrogénés qui sont aussi utilisables dans l'invention, et enfin par lequel on produit des mélanges de ces dérivés hydrogénés et de produits de la dimérisation:

*Schéma de réaction # 1:*

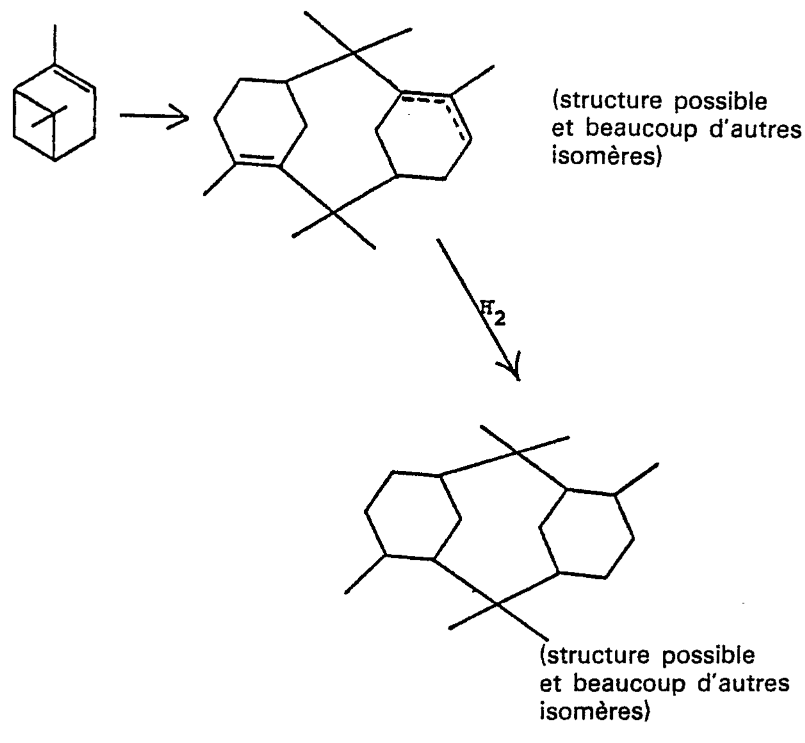


Les catalyseurs utilisables pour produire les produits de la dimérisation de terpènes contenant 10 atomes de carbone suivant l'invention sont les acides de Lewis tels que le trifluorure de bore, le trichlorure d'aluminium, les acides de Bronstedt tels que l'acide sulfurique et l'acide phosphorique, les résines échangeuses d'ions catalyseurs, comme l'Amberlyst 15, les argiles acides telles que la terre à foulon et l'argile acide japonaise. Ces réactions de dimérisation s'opérent en présence de solvants tels que la cyclohexane, le toluène et l'hexahydro-1,1,2,3,3-pentaméthylindane, ou en l'absence de solvants. La température de dimérisation peut varier de 20 jusqu'à 250°C. Un catalyseur recommandé avec un ordre de grandeur de température recommandé sera par exemple l'éthérate de trifluorure de bore à 0 à 100°C, ou l'argile acide à 80 à 175°C. On peut utiliser la pression atmosphérique dans la réaction de dimérisation, bien que l'on puisse utiliser des pressions plus élevés ou moins élevées sans que le rendement en soit défavorablement affecté. Le rapport en poids entre le terpène monomère

contenant 10 atomes de carbone et le catalyseur peut varier de 1:0,005 et 1:0,2, avec un rapport en poids préféré de 1:0,05 à 0,01.

Les "térébenthines" y compris la térébenthine de pâte au sulfate, sous-produit de l'opération de formation d'une pulpe pour Kraft (sulfate) pour la fabrication du papier sont décrites dans un article de John M. Derfer, intitulé: "Térébenthine comme source de matières pour les parfums et arômes", Perfumer and Flavorist International, Vol. 3, n° 1, aux pages 45 à 50. La composition des "térébenthines", y compris la térébenthine au sulfate, mentionnées ci-dessus, sont décrites comme suit:

"Dans les trois types de térébenthine produits dans le Sud des Etats-Unis (qui est la plus important région productrice du monde) l'alpha-pinène est le constituant le plus abondant, sa proportion variant de 60 à 80% (voir tableau 1). Le bêta-pinène est le second par sa teneur dans la gomme, et est suivi par la térébenthine du sulfate variant de 25 à 35% pour le premier et de 20 à 25% pour ce dernier. La térébenthine du bois contient peu de bêta-pinène si elle en contient. Des deux pinènes, la forme bêta est chimiquement la plus réactive. La résine de térébenthine contient de 5 à 8% de p-menthadiènes monocycliques, auxquelles on donne couramment le nom de dipentènes, nom commun du dl-limonène.

Tableau 1 — Composition des térébenthines du Sud (%)

| Composant | Sulfate | Gomme | Bois |
|---|---|---|---|
| Alpha-pinène | 60—70 | 60—65 | 75—80 |
| Bêta-pinène | 20—25 | 25—35 | 0—2 |
| Camphène | Trace | Trace | 4—8 |
| Autres | 6—12 | 5—8 | 15—20 |

"Le dl-limonène est le composant principal de ce mélange de p-menthadiènes. La térébenthine du sulfate contient de 6 à 12% de ce mélange pendant que la térébenthine du bois en contient un peu plus. La térébenthine du sulfate contient de 5 à 10% de matières oxygénées d'où l'on sépare l'essence de pin "naturelle", pour la plus grande partie, des alcools terpéniques. L'essence de pin "naturelle" est aussi produite quand on traite le bois des souches pour produire la térébenthine de bois. On rencontre aussi, dans le térébenthine du sulfate, en petites proportions, du caryophyllène, méthylchavicol et anéthol. La composition de la térébenthine ne dépend pas seulement du procédé par lequel on l'isole, mais aussi de l'espèce et de l'emplacement géographique des arbres. Par exemple certaines térébenthines de l'Ouest, ainsi que certaines térébenthines étrangères contiennent des proportions appréciables de $\Delta^3$-carène, qui trouve peu d'usages autres que comme solvant."

En conséquence, et plus spécifiquement, les variétés de térébenthines que l'on peut utiliser dans la mise en pratique de l'invention, ingrédients à base de terpène contenant 10 atomes de carbone qui sont susceptibles d'être dimérisés pour former les produits de la dimérisation utilisables dans l'invention sont les suivants:

| *Espèce de pin d'où la térébenthine est dérivée* | *Composition chimique de la térébenthine* | |
|---|---|---|
| (1) Pinus albicaulis Engelmann | $\Delta^3$-carène | (35%) |
| | autres terpènes | (10%) |
| | sesquiterpène | (7%) |
| | diterpène | (30%) |
| (2) Pinus aristata Engelmann | dl et alpha-pinène | (96%) |
| | sesquiterpène tricylique | (4%) |
| (3) Pinus attenuata lemmon | dl-alpha-pinène | (+ de 95%) |
| | ni béta-pinène | |
| | ni camphène | |

| *Espèce de pin d'ou la térébenthine est dérivée* | *Composition chimique de la térébenthine* | |
|---|---|---|
| (4) Pinus balfouriana Grev. and Balif. | dl-et 1-alpha-pinène | (90%) |
| | dl-et 1-bêta-pinène | (2%) |
| | dl-et 1-limonène | (2%) |
| | sesquiterpène tri-cyclique | (4%) |
| (5) Pinus banksiana Lambert | dl-et 1 -alpha-pinène | (85%) |
| | d1-et 1-bêta-pinène) | 10%) |
| (6) Pinus caribaea Morelet | 1-alpha-pinène | (61,5%) |
| | 1-bêta-pinène | (34%) |
| | queues | (4,5%) |
| (7) Pinus clausa Engelmann Vasey | 1-alpha-pinène | (10%) |
| | 1-camphène | (10%) |
| | 1-bêta-pinène | (75%) |
| (8) Pinus contorta var. latifolia Engelmann | 1-bêta-phellandrène | |
| (9) Pinus coulteri D. Don | n-heptane | (5%) |
| | 1-alpha-pinène | (30—35%) |
| | 1-bêta-phellandrène | (35—45%) |
| | n-undécane | (10%) |
| (10) Pinus echinata Miller | d-alpha-pinène | (85%) |
| | 1-bêta-pinène | (11%) |
| | limonène | |
| (11) Pinus edulis Engelmann | alpha-pinène | (70—75%) |
| | bêta-pinène | (5%) |
| | alpha-cadiène | (15—20%) |
| (12) Pinus flexilis James | dl-et 1-alpha-pinène albicaulène | (80%) |
| | sesquiterpène mono-cyclique | (13%) |
| | sesquiterpène bi-cyclique | (7%) |
| (13) Pinus glabra Walter | 1-limonène | |

| *Espèce de pin d'ou la térébenthine est dérivée* | *Composition chimique de la térébenthine* | |
|---|---|---|
| (14) Pinus lambertiana Douglas | 1-alpha-pinène | (65%) |
| | 1-bêta-pinène | (13%) |
| | sesquiterpène bicyclique du type cadalène | (10%) |
| | Lambertol (sesquiterpène alcool $C_{15}H_{26}O$) | (2%) |
| (15) Pinus monophylla Torrey et Fremont | d-alpha-pinène | (85%) |
| | 1 limonène ou dipentène | (4—5%) |
| | d-cadinène | (4—6%) |
| (16) Pinus monticola Douglas | d-alpha-pinène | (60%) |
| | bêta-pinène | (26%) |
| | n-undécane | (1—2%) |
| | sesquiterpènes et peut-être limonène | |
| (17) Pinus muricata D. Don | d-alpha-pinène | (98—99%) |
| | camphène | (— de 1%) |
| (18) Pinus palustris Miller | d-alpha-pinène | (65%) |
| | 1-bêta-pinène | (31,5%) |
| | queues | (3%) |
| (19) Pinus ponderosa Lawson | 1-bêta-pinène | (50%) |
| | 1-$\Delta^3$-carène | (20%) |
| | 1-limonène et dipentène | (25%) |
| | d-cadinène | (3%) |
| | pondérène | (— de 1%) |
| (20) Pinus ponderosa var. scopulorum Engelmann | d-alpha-pinène | (60—70%) |
| | bêta-pinène | (5%) |
| | limonène | (20—25%) |
| (21) Pinus radiata D. Don | dl-alpha-pinène | (75%) |
| | 1-bêta-pinène | (22%) |
| (22) Pinus resinosa Ait. | alpha-pinène | |
| (23) Pinus rigida var. serotina Michaux Loudon | limonène | |

| *Espèce de pin d'ou la térébenthine est dérivée* | *Composition chimique de la térébenthine* | |
|---|---|---|
| (24) Pinus strobus Linnaeus | dl-alpha-pinène | (75%) |
| | 1-bêta-pinène | (15%) |
| | alcools terpéniques et cétones | (4%) |
| | sesquiterpène tri-cyclique | (0,3%) |
| (25) Pinus taeda Linnaeus | d-alpha-pinène | (85%) |
| | 1-bêta-pinène | (12%) |
| (26) Pinus torreyana Parry | 1-limonène | (75%) |
| | n-décylaldéhyde | (10%) |
| | n-undécane | (5%) |
| | longifolène | (4%) |
| | laurylaldéhyde | (0,2%) |
| | heptane et nonale, de chaque | (— de 0,1%) |
| (27) Pinus virginiana Miller | dl-alpha-pinène | (90%) |
| | 1-alpha-pinène | (8%) |
| (28) Pinus washoensis Mason et Stockwell | d-$\Delta^3$-carène (principalement: alpha-pinène, dipentène, sesquiterpène cyclique) 1-bêta-pinène (s'il existe du $\Delta^3$-carène, la teneur est faible | |

Les produits (A) de la dimérisation suivant l'invention et leurs dérivés hydrogénés peuvent être produits suivant l'un quelconque des procédés connus dans la technique actuelle et suivant l'un des schémas de réaction suivants:

*Schéma de réaction # 1*

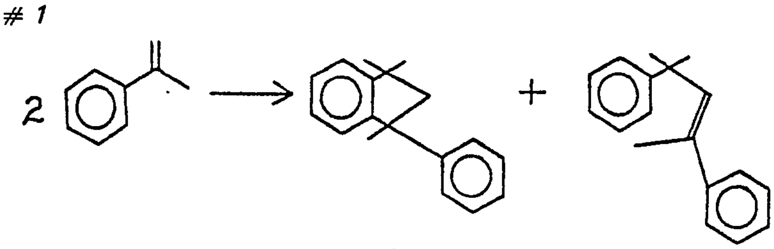

(quantité mineure d'un mélange d'isomères cis et trans)

*Schéma de réaction # 2*

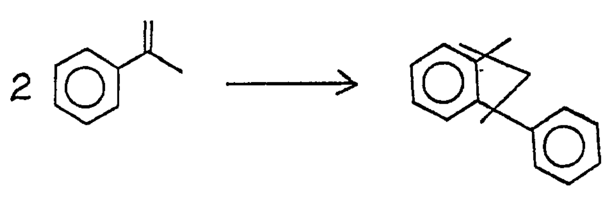

*Schéma de réaction # 3*

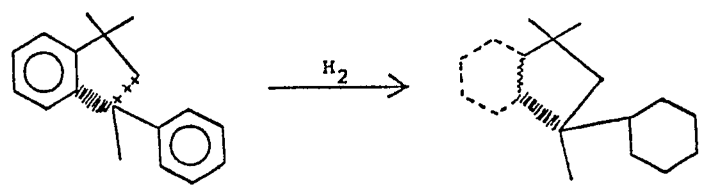


*Schéma de réaction # 4*

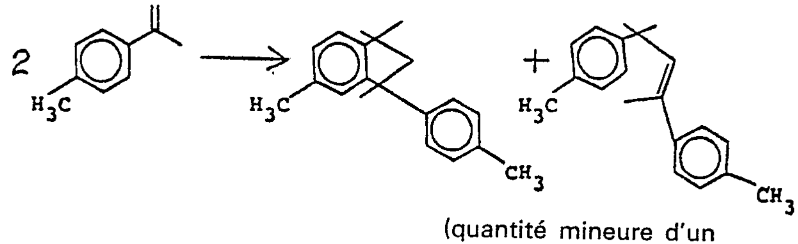


(quantité mineure d'un mélange d'isomères cis et trans)

*Schéma de réaction # 5*

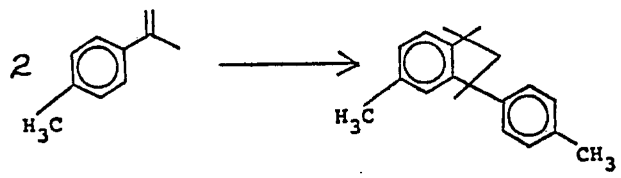


*Schéma de réaction # 6*

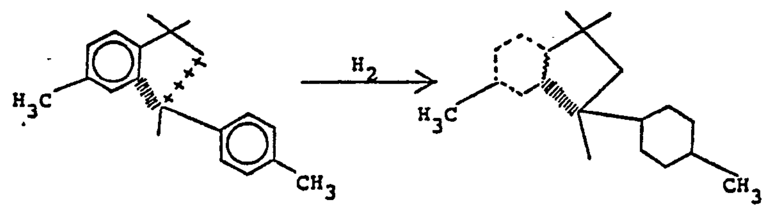


*Schéma de réaction # 7*

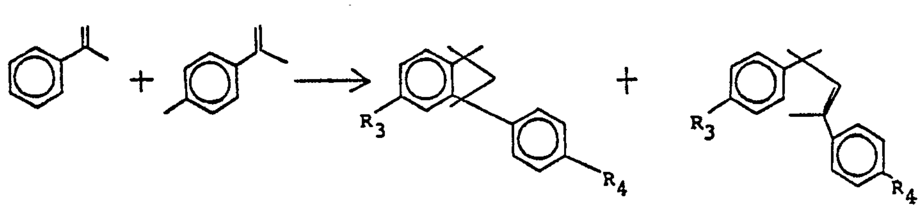

(mélanges)

(quantité mineure d'un mélange d'isomères cis et trans)

*Schéma de réaction # 8*

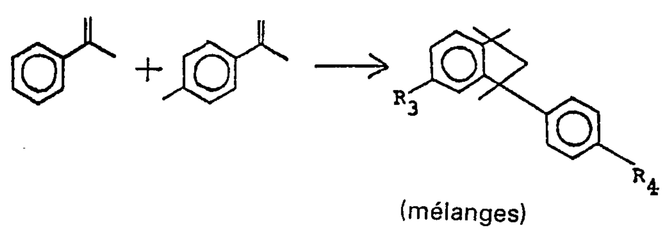

(mélanges)

*Schéma de réaction # 9*

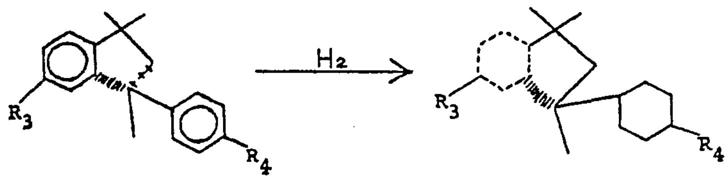


(mélange) (mélange)

où $R_3$ et $R_4$ sont semblables ou différents et représentent un hydrogène ou un méthyle ou un autre alcoyle inférieur en $C_2$ à $C_4$, et où les lignes en tirets, les lignes ondulées, la ligne et la ligne +++++ sont telles qu'elles sont définies plus haut.

Dans cette réaction de dimérisation, les catalyseurs que l'on peut utiliser sont les acides de Lewis, tels que le trifluorure de bore et le trichlorure d'aluminium, et les acides de Bronstedt, tels que les acides sulfurique et phosphorique, ou ces acides sur des véhiculeurs comme l'alumine, la silice, ou les résines échangeuses de cations catalyseurs, comme l'Amberlyst $®_{15}$, ou les argiles acides catalyseurs tels que l'argile acide japonaise ou la terre à foulon. La réaction de dimérisation est effectuée en présence d'un solvant tel que le cyclohexane ou en l'absence de solvant. L'ordre de grandeur de la température de dimérisation peut se situer d'environ 20°C jusqu'à environ 250°C, l'ordre de grandeur préféré étant, quand on utilise l'éthérate de trifluorure de bore, de 0 à 100°C, et, quand on utilise des argiles acides, de 80 à 200°C. La pression à laquelle on effectue la réaction pourra être convenablement la pression atmosphérique, mais on pourra aussi utiliser, sans effet défavorable sur le rendement du produit, des pressions plus hautes ou plus basses que la pression atmosphérique. Le rapport en poids entre l'alpha-méthylstyrène ou l'homologue alpha-méthylstyrène méthylé et le catalyseur se situera entre environ 1:0,005 et 1:0,2, quand on utilisera une argile acide catalyseur, et environ 1:0,1 à 1:3 quand on utilisera, par exemple, un acide de Bronstedt catalyseur, tel que l'acide sulfurique.

La réaction d'hydrogénation peut être opérée dans les conditions d'hydrogénation standard, en utilisant les catalyseurs d'hydrogénation standard. Ainsi, par exemple, la réaction d'hydrogénation s'effectue en présence de catalyseur au palladium ou au carbone, ou de nickel de Raney catalyseur, à des températures pouvant aller de 80 à 150°C environ, et sous des pressions pouvant aller de 4 jusqu'à 30 kg/cm² environ.

Les essences pour parfumerie allongées et les produits chimiques allongés suivant l'invention peuvent être utilisés dans des compositions où l'on devra utiliser des essences naturelles ou des produits chimiques, par exemple, en combinaison avec de l'essence de bois de santal, essence de vétiver, mousse de chêne, ionone, labdanum, méthyl-ionone, essence de patchouli, et autres substituts synthétiques.

Les matières premières pour parfumerie allongées pourront être utilisées comme constituants de compositions de parfumerie combinées où l'on mélange ensemble un certain nombre de matières premières pour parfumerie, d'origine naturelle et/ou synthétique pour produire un effet d'odeur particulier recherché. Ces compositions peuvent être utilisées dans des pulvériseurs, d'ambiance, ou peuvent être mélangées dans du savon, des compositions détergentes ou désordorisantes (déodorants), y compris les sels pour bains, shampooings, eau de toilette, crèmes pour le visage, poudre de talc, lotions corporelles, préparations anti-solaires, et lotions et crèmes à raser. Les compositions de parfumerie peuvent aussi être utilisées pour parfumer des substrats tels que des fibres, des tissus, et des papiers.

Les exemples suivants sont donnés pour illustrer des modes de réalisation de l'invention tels qu'il est recommandé actuellement de la mettre en pratique. On comprendra facilement que ces exemples ne constituent que des illustrations de l'invention, et sont donnés sans aucune idée de limitation.

Exemple 1

*Préparation du produit de la dimérisation de l'alpha-méthyl-styrène*
*Réaction:*

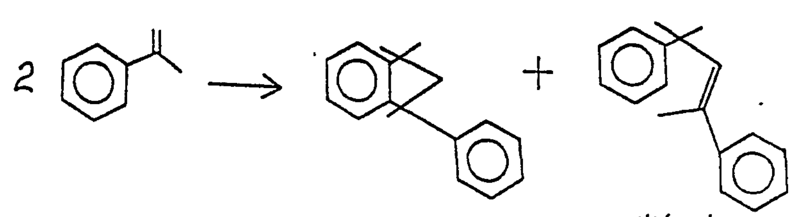

quantité mineure d'un mélange d'isomères cis et trans

Dans un ballon à réaction de deux litres, équipé d'un thermomètre, d'un condenseur à reflux, d'un bain de refroidissement, d'un entonnoir d'addition, d'un agitateur, et d'un souffleur de gaz, on introduit 100 g d'eau. En seize minutes, on ajoute à l'eau 318 g d'acide sulfurique concentré. On chauffe alors le contenu du ballon à 30°C. On ajoute alors, dans un intervalle de deux heures après avoir ajouté l'acide sulfurique, et tout en maintenant la température de la masse réactionnelle à 20 à 33°C, 500 g d'alpha-méthylstyrène. Après addition de l'alpha-méthylstyrène, on maintenant la masse réactionnelle à une température de 30°C pendant une durée de quatre heures. On ajoute ensuite 500 g d'eau que l'on fait suivre de 250 g de cyclohexane. On agite alors la masse réactionnelle pendant une durée de quinze minutes et chauffe à 70°C. On sépare les couches et lave la couche organique jusqu'à neutralité (à 70°C) avec une solution d'hydroxyde de sodium à 5% (deux fois 250 ml) et avec une solution de chlorure de sodium à 5% (trois fois 250 ml). On recueille 650 g de produit brut, et distille après addition au mélange de 15 g de Primol (huile minérale produite per Exxon Inc. de Linden)® et 0,2 g d'ionox® (2,6-di-t-butyl-4-méthylphénol), au moyen d'une colonne de Vigreux de 30 cm, dans les conditions suivantes:

| Fraction | Température de la vapeur | Température du liquide | Vide Pascals | Poids de la fraction |
|---|---|---|---|---|
| 1 | 69 | 101/135 | $10^5/10^5$ | 102,2 |
| 2 | 132 | 145 | 306 | 2,8 |
| 3 | 132 | 145 | 306 | 26,4 |
| 4 | 132 | 147 | 306 | 16,0 |
| 5 | 132/134 | 148/148 | 293/293 | 19,4 |
| 6 | 132 | 149 | 293 | 26,2 |
| 7 | 132 | 149 | 293 | 21,6 |
| 8 | 132 | 142 | 293 | 25,4 |
| 9 | 132 | 150 | 293 | 24,2 |
| 10 | 133 | 150 | 293 | 29,6 |
| 11 | 133 | 151 | 333 | 28,2 |
| 12 | 133 | 151 | 319 | 25,5 |
| 13 | 133 | 152 | | 25,0 |
| 14 | 133 | 152 | 306 | 23,7 |
| 15 | 133 | 155 | | 31,5 |
| 16 | 135 | 162 | 306 | 29,4 |
| 17 | 137 | 169 | 306 | 23,1 |
| 18 | 139 | 176 | 306 | 12,5 |
| 19 | 142 | 202 | | 13,2 |
| 20 | 141 | 221 | 306 | 10,3 |
| 21 | 188 | 230 | | 10,5 |
| 22 | 187 | 242 | 306 | 8,1 |

La Figure 1 annexée donne le profil de la chromatographie gaz-liquide (GLC) de la fraction 19. Cette fraction est principalement constituée par le dimère de l'alpha-méthylstyrène ayant la structure suivante:

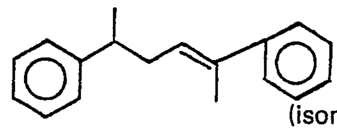

(isomères cis et trans)

La Figure 2 donne le spectre infra-rouge de la fraction 19. La Figure 3 est le spectre NMR (résonance magnétique nucléaire) de cette fraction 19. Le spectre de masse de cette fraction de ce composé qui a la structure:

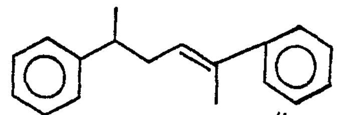

(isomères cis et trans)

est le suivant:

| M/E | Intensité relative | M/E | Intensité relative- |
|---|---|---|---|
| 39 | 23[5] | 103 | 14 |
| 41 | 27[4] | 119 | 100[1] |
| 51 | 18 | 143 | 20 |
| 77 | 19 | 221 | 32[3] |
| 91 | 42[2] | 236 | 20[6] |

Le profil GLC (chromatographie gaz-liquide- de la même fraction qui est principalement le composé ayant la structure:

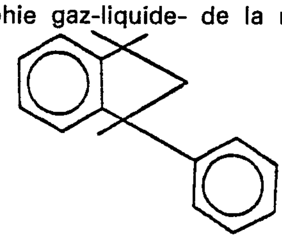

est illustré dans la Figure 4. La Figure 5 donne le spectre IR de la fraction 3. La Figure 6 donne le spectre NMR de la fraction 3.

Les Figures 4, 5 et 6, respectivement, représentent aussi les spectres GLC, IR et NMR des produits obtenus suivant l'exemple III que l'on trouvera plus loin.

Exemple II

*Préparation du produit de la dimérisation de l'alpha-méthyl styrène*
*Réaction:*

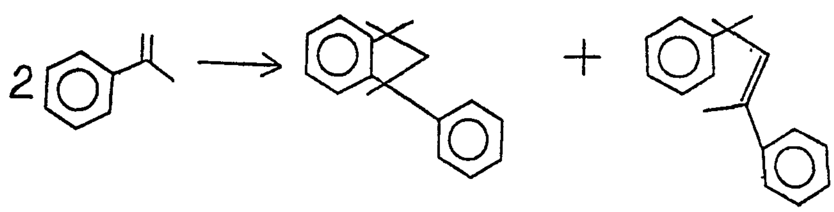

(quantité mineure) (quantité majeure des isomères mélangés cis et trans)

Dans un ballon à réaction de un litre, pourvu d'un thermomètre, d'un entonnoir d'addition, d'une

jaquette chauffante, d'un condenseur à reflux, d'un agitateur, d'un tube en Y, et d'une tête de distillation, on ajoute 100 g de cyclohexane suivi de 5 g d'acide para-toluène sulfonique. On chauffe le mélange obtenu à 50°C, et en une heure, on ajoute 500 g d'alpha-méthylstyrène dans le ballon de réaction. On chauffe ensuite la masse réactionnelle à 100°C, et on maintient à cette température pendant une durée de quatre heures. On obtient ainsi 529,3 g de produit brut que l'on mélange ensuite avec 15 g de Primol® et 0,2 g d'Ionox®. Le mélange résultant est distillé dans une colonne de distillation à tube adapteur Y, ce qui donne les chiffres de distillation suivants:

| Fraction | Température de la vapeur | Température du liquide | Vide Pascals | Poids de la fraction |
|---|---|---|---|---|
| 1 | 21/80 | 90/149 | 346/333 | 6,4 |
| 2 | 139 | 155 | 333 | 7,0 |
| 3 | 139 | 155 | 333 | 21,4 |
| 4 | 140 | 157 | 333 | 34,0 |
| 5 | 142 | 160 | 319 | 49,6 |
| 6 | 144 | 170 | 319 | 100,4 |
| 7 | 164 | 195 | 319 | 58,5 |
| 8 | 174 | 203 | 293 | 8,0 |
| 9 | 200 | 215 | 466 | 70,0 |
| 10 | 202 | 215 | 293 | 31,3 |
| 11 | 206 | 215 | 293 | 27,1 |
| 12 | 214 | 225 | 293 | 18,1 |
| 13 | 210 | 250 | 293 | 41,6 |

Exemple III

*Préparation de l'alpha-méthyl-styrène dimère*

*Réaction:*

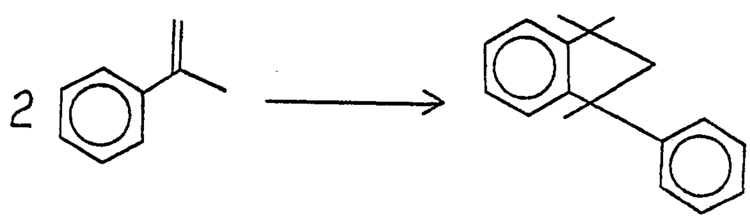

Dans un ballon à réaction de un litre équipé d'un thermomètre, d'un entonnoir d'addition, d'une jaquette de chauffage, d'un condenseur à reflux et d'un agitateur, on ajoute 20 g de Filtrol 25® (argile acide activée, granulée, passant au tamis de 10 à 20 mailles, produite par le Filtrol Corporation de 5959 West Century Brd., Los Angeles, California 90 045) offrant les propriétés suivantes:

Analyse granulométrique au tamis standard Tyler:

| | | |
|---|---|---|
| passant au tamis de 10 mailles, | poids % | 100 |
| passant au tamis de 20 mailles, | poids % | 5 |
| Humidité libre, | poids % | 10 |
| Humidités libre et combinée, (perte à 925°C) | poids % | 15 (maximum) |
| Densité apparente, | $kg/m^3$ | 700 |
| Densité des particules | | 1,3 |
| Surface, absorption de $N_2$ (méthode BET) | $m^2/g$ | 280 à 300 |

On ajoute 50 g d'alpha-méthylstyrène au Filtrol® et on chauffe la masse réactionnelle à 100°C. On ajoute encore lentement 450 g d'alpha-méthylstyrène à la masse réactionnelle, sur une durée de deux heures. On chauffe ensuite la masse réactionnelle à 150°C et maintient à cette température pendant une durée de quatre heures. La masse réactionnelle est ensuite filtrée, ce qui donne 470 g de produit brut que l'on mélange ensuite avec 12 g de Primol® et 0,3 g d'Ionox®, et distille sur une colonne Vigreux de 30 cm, ce qui donne les fractions suivantes et les chiffres de distillation suivants:

| Fraction | Température de la vapeur | Température du liquide | Vide Pascals | Poids de la fraction |
|---|---|---|---|---|
| 1 | 38/88 | 135/140 | 333/333 | 1,2 |
| 2 | 133 | 142 | 266 | 7,0 |
| 3 | 134 | 142 | 266 | 12,0 |
| 4 | 134 | 142 | 266 | 17,1 |
| 5 | 134 | 145 | 239 | 53,5 |
| 6 | 134 | 146 | 239 | 31,0 |
| 7 | 135 | 147 | 239 | 49,2 |
| 8 | 135 | 148 | 239 | 51,5 |
| 9 | 136 | 149 | 239 | 46,2 |
| 10 | 137 | 151 | 239 | 52,3 |
| 11 | 137 | 159 | 239 | 43,2 |
| 12 | 139 | 170 | 239 | 17,5 |
| 13 | 185 | 225 | 239 | 21,6 |

La Figure 4 est le profil GLC des fractions 9 à 12. La Figure 5 donne le spectre infra-rouge des fractions 9 à 12. La Figure 6 donne le spectre NMR des fractions 9 à 12. La Figure 7 donne le spectre de masse des fractions 9 à 12. Enfin la Figure 8 donne un second profil GLC des fractions 9 à 12.

Exemple IV (A)

*Préparation de l'alpha-pinène dimère*
*Réaction:*

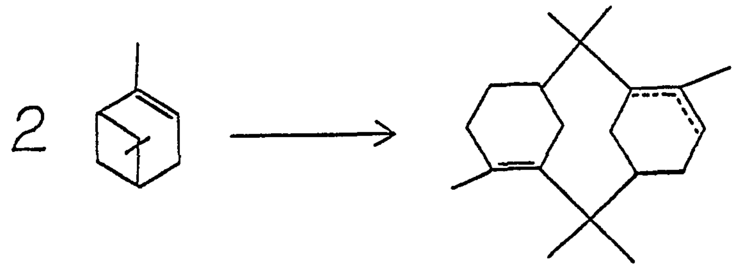

(structure possible et beaucoup d'autres isomères)

Dans un ballon à réaction de deux litres équipé d'un agitateur, d'un thermomètre, d'un entonnoir d'addition et d'un condenseur à reflux, on introduit 100 g d'alpha-pinène et 40 g de Filtrol 25® qui est une argile acide activée, granulée, passant au tamis de 20 mailles (produite par The Filtrol Corporation of 5959, West Century Boulevard, Los Angelés, California 90 045) ayant les propriétés suivantes:

| | | |
|---|---|---|
| Analyse granulométrique au tamis standard Tyler | | |
| passant au tamis de 10 mailles, | poids% | 100 |
| passant au tamis de 20 mailles, | poids% | 5 |
| Humidité libre, | poids% | 10 |
| Humidités libre et combinée, (perte à 925°C) | poids % | 15 (maximum) |
| Densité apparente, | kg/m³ | 688 |
| Densité des particules | | 1,3 |
| Surface, (absorption d'azote) (méthode BET) | m2/g | 280 à 300 |

La masse réactionnelle est chauffée à 150°C pendant que l'on agite, et on ajoute un supplément de 900 g d'alpha-pinène, en deux heures, tout en maintenant la masse réactionnelle à 150°C. On continue ensuite à agiter cette masse réactionnelle à 150°C, jusqu'à ce que l'analyse par GLC d'échantillons indique que la réaction est complète (il ne reste alors que peu ou pas d'alpha-pinène).

On refroidit alors la masse réactionnelle à 80°C et la filtre en utilisant une cellule filtrante.

On distille le filtrat sous un vide de 3 mm Hg, en utilisant une colonne Godloe de 30 cm, et en partant d'un rapport de reflux de 9:1 et en allant ensuite jusqu'à 4:1. Immédiatement avant la distillation on ajoute à la matière que l'on se propose de distiller, 30 g de Primol®.

Les chiffres donnés par la distillation sont les suivants:

| Fraction | Température de la vapeur | Température du liquide | Vide en Pascals | Rapport de reflux | Poids de la fraction |
|---|---|---|---|---|---|
| 1 | 35/75 | 72/115 | 6.650/4.655 | 9:1/9:1 | 44,1 |
| 2 | 39 | 95 | 399 | 9:1 | 53,9 |
| 3 | 41 | 103 | 399 | 9:1 | 46,0 |
| 4 | 41 | 165 | 399 | 4:1 | 68,7 |
| 5 | 31/128 | 152/168 | 133/106 | 4:1/4:1 | 38,0 |
| 6 | 133 | 175 | 106 | 4:1 | 43,0 |
| 7 | 133 | 179 | 106 | 4:1 | 51,1 |
| 8 | 133 | 180 | 106 | 4:1 | 49,4 |
| 9 | 133 | 181 | 106 | 4:1 | 44,5 |
| 10 | 133 | 185 | 106 | 4:1 | 49,5 |
| 11 | 133 | 187 | 106 | 4:1 | 47,5 |
| 12 | 136 | 193 | 106 | 4:1 | 42,7 |
| 13 | 140 | 204 | 106 | 4:1 | 44,5 |

La Figure 9 représente le profil GLC du dimère de l'alpha-pinène, fractions 9 à 11. (Conditions: 2% de Carbowax® dans la colonne de 65 cm x 6,30 mm, programmée à 80 à 220°C, à raison de 10°C par minute).

La Figure II est le spectre NMR du produit de l'exemple IV (A). La Figure 12 est le spectre IR de ce même produit.

Exemple IV (B)

*Dimères du camphène*

Dans un ballon à réaction de deux litres équipé d'un agitateur, d'un thermomètre, d'un entonnoir d'addition et d'un condenseur à reflux comportant une Bidwell Trap, on introduit:

| | |
|---|---|
| Hexahydropentaméthylindane | 336 g |
| Filtrol 25® (dont les propriétés ont été indiquées dans l'exemple I (A)) | 32 g |

et chauffe ce mélange à 155°C tout en l'agitant. Sur un intervalle de temps de 2 heures 1/4, on ajoute 547 g de camphène, tout en maintenant la masse réactionnelle à 155°C. On agite ensuite cette masse pendant 7 h 1/2 à 115 à 158°C, et l'on surveille la progression de la dimérisation sur l'appareil de GLC, (conditions: collonne à 5% de SE 30, dimensions de la colonne:3 m:6,35 mm, programmée à 80 à 240°C à raison de 8°C par minute). L'analyse GLC ne montre qu'un très faible changement après deux heures. On filtre alors la masse réactionnelle. On lave le gâteau de filtre avec 200 g d'hexahydropentaméthylindane. Le poids du filtrat est de 1056 g.

On distille le filtrat obtenu en présence de Primol® (30 g) et d'Ionox® 1 g, dans une colonne Vigreux de 45 cm, équipée d'une tête de reflux. On obtient les chiffres de distillation suivants:

| Fraction N°s | Température de la vapeur | Température de la masse | Pression Pascals | Poids de la fraction | Analyse GLC de la fraction |
|---|---|---|---|---|---|
| 1—6 | 45—100 | 87—160 | 346 | 631,9 | hexahydropenta-méthylindane récupérée |
| 7 | 149 | 170 | 466 | 10,5 | Section intermédiaire |
| 8 | 150 | 170 | 372 | 40,7 | Dimères pratiquement purs |
| 9—11 | 152—168 | 176—230 | 333 | 214,8 | Dimères purs |
| 12 | 215 | 275 | 333 | 22,9 | |
| 13—14 | 225—250 | 290—306 | 333 | 62,7 | Très faible élution en GLC |

Résidu — 41,7
Capté — 18,0

La Figure 10 donne les profils GLC des fractions 9 à 11, le camphène dimère (colonne SE 30 à 5%, 3 m x 6,35 mm, programmée à 80 à 240°C à raison de 8°C par minute).

La structure de l'hexahydropentaméthylindane, utilisé comme solvant est la suivante:

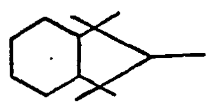

Exemple IV (C)

*Préparation du d-limonène dimère*

Dans un ballon à réaction de 500 ml, pourvu d'un thermomètre, d'un agitateur, d'un condenseur à reflux et d'un entonnoir d'addition, on introduit 5 g de Primol® et 2 g de Filtrol 25®. On chauffe le mélange réactionnel à 150°C et ajoute goutte à goutte, sur un intervalle de temps de 80 minutes, 40 g de limonène. On chauffe ensuite la masse réactionnelle à 150°C pendant 3 heures.

On refroidit ensuite la masse réactionnelle, filtre et distille. Le produit obtenu est le dimère de la d-limonène, ce que confirment les analyses par GLC, NMR et IR.

Le profil GLC est donné dans la Figure 13. Le spectre NMR est donné dans la Figure 14, et le spectre IR, dans la Figure 15.

Exemple V

On mélange de l'essence de patchouli (80 parties), provenant des îles Seychelles, avec 10 parties d'alpha-méthylstyrène dimère produit suivant l'un quelconque des exemples I, II ou III, et 10 parties de l'alpha-pinène de l'exemple IV (A). On constate que le mélange des dimères de l'alpha-méthylstyrène et de l'alpha-pinène agit comme dilutif vis-à-vis de l'essence de patchouli, du fait que l'odeur effective caractéristique de cette dernière n'est pas sensiblement modifiée.

Exemple VI

L'essence de patchouli allongée, préparée suivant l'exemple V, est introduite avec avantage dans une composition combinée du type Chypre, en mélangeant les ingrédients suivants:

| | Parties |
|---|---|
| Aldéhyde cinnamique | 1 |
| Glycidate d'éthyl-méthyl-phényl | 1 |
| Nonyl acétaldéhyde de méthyle | 2 |
| Mouse de chêne (absolu) | 20 |
| Essence de bois de santal (des Indes orientales) | 20 |
| Acétate de vétiveryl | 20 |
| Essence d'ylang n° 1 | 20 |
| Résine de benjoin (Sumatra) | 30 |
| Alpha-ionone (100%) | 30 |
| Essence de girofle (Zanzibar) | 36 |
| Essence de bergamote | 40 |
| Hydroxycitronellal | 40 |
| Iso Eugénol | 40 |
| Essence de patchouli allongée (Exemple V) | 40 |
| Coumarine | 50 |
| Musc cétone | 50 |
| Salicylate d'amyle | 60 |
| Essence de bois de cèdre (américaine) | 60 |
| Citronellol | 60 |
| Acétate de benzyle | 80 |
| Alcool de phényl-éthyl | 150 |
| Acétate de terpinyle | 150 |
| | 1000 |

Exemple VII

On prépare un dilutif de base pour l'essence de patchouli en mélangeant les ingrédients suivants:

| | Parties |
|---|---|
| Mélange de 10 parties d'alpha-méthylstyrene dimère produit suivant l'un quelconque des exemples I, II ou III et 28 parties de d-limonène dimère produit suivant l'exemple IV (C) | 38 |
| Galaxolide | 27 |
| Oxydate d'isolongifolène | 20 |
| Omége-hydroxyméthyl longifolène | 10 |
| Cédrol | 3 |
| Essence de bois de santal (Indes orientales) | 2 |
| | 100 |

Ce mélange, 46 parties, est ensuite mélangé avec de l'essence de patchouli naturelle (Seychelles) 60 parties pour réaliser une essence de patchouli allongée satisfaisante.

Exemple VIII

L'essence de patchouli allongée, préparée dans l'exemple VII, est incorporée dans une composition de parfumerie combinée du type fougère, contenant les ingrédients suivants:

| | Parties |
|---|---|
| Baume du Pérou | 30 |
| Résine de labdanum | 30 |
| Mousse de chêne (absolu) | 30 |
| Essence de bois de santal (Indes orient.) | 30 |
| Acétaté de linalyle | 40 |
| Acétate de terpinyle | 40 |
| Essence de géranium (Bourbon) | 50 |
| Musc ambrette | 50 |
| Coumarine | 60 |
| Salicylate d'amyle | 60 |
| Méthyl-ionone | 70 |
| Essence de bois de cèdre (américaine) | 80 |
| Essence de girofle (Zanzibar) | 80 |
| Essence de vétiver (Bourbon) | 80 |
| Essence de patchouli allongée | 130 |
| Essence de lavandin | 140 |
| | 1000 |

Exemple IX

On mélange de l'essence de patchouli (85 parties) provenant des Iles Seychelles avec 10 parties en poids de camphène-dimère, produit suivant l'exemple IV (B) et 5 parties d'alpha-méthylstyrène dimère de l'exemple II. Ce mélange de camphène dimère — alpha-méthylstyrène dimère agit, comme on le constate, comme dilutif pour allonger l'essence de patchouli du fait que l'effet odorant caractéristique de cette dernière n'est pas modifié sensiblement.

Exemple X

On incorpore avantageusement l'essence de patchouli allongée préparée suivant l'exemple IX, dans une composition combinée du type Chypre en mélangeant les ingrédients suivants:

| | Parties |
|---|---|
| Aldéhyde cinnamique | 1 |
| Glycidate d'éthylméthylphényl | 1 |
| Nonyl acétaldéhyde de méthyle | 2 |
| Mousse de chêne (absolu) | 20 |
| Essence de bois de santal (Indes orientales) | 20 |
| Acétate de vétiveryl | 20 |
| Essence d'ylang n° 1 | 20 |
| Résine de benjoin (Sumatra) | 30 |
| Alpha ionone (100%) | 30 |
| Essence de girofle (Zanzibar) | 36 |
| Essence de bergamote | 40 |
| Hydroxycitronellal | 40 |
| Iso eugénol | 40 |
| Essence de patchouli allongée (exemple IX) | 40 |
| Coumarine | 50 |
| Musc cétone | 50 |
| Salicylate d'amyle | 60 |
| Essence de bois de cèdre (américaine) | 60 |
| Citronellol | 60 |
| Acétate de benzyle | 80 |
| Alcool de phényl éthyl | 150 |
| Acétate de terpinyl | 150 |
| | 1000 |

Exemple XI

On mélange de l'essence de patchouli (80 parties), provenant des Iles Seychelles, avec l'alpha-pinène dimère produit suivant l'exemple IX (A) (20 parties). On constate que l'alpha-pinène dimère agit comme dilutif pour allonger l'essence de patchouli, du fait que l'effet odorant caractéristique de cette dernière n'est pas sensiblement modifié.

Exemple XII

On incorpore avantageusement l'essence de patchouli allongée, préparée suivant l'exemple XI, dans une composition combinée du type Chypre, en mélangeant les ingrédients suivants:

| | Parties |
|---|---|
| Aldéhyde cinnamique | 1 |
| Glycidate d'éthyl méthyl | 1 |
| Nonyl acétaldéhyde de méthyle | 2 |
| Mousse de chêne (absolu) | 20 |
| Essence de bois de santal (Indes. orient.) | 20 |
| Acétate de vétiveryl | 20 |
| Essence d'ylang n° 1 | 20 |
| Résine de benjoin (Sumatra) | 30 |
| Alpha ionone (100%) | 30 |
| Essence de girofle (Zanzibar) | 36 |
| Essence de bergamote | 40 |
| Hydroxycitronellal | 40 |
| Iso eugénol | 40 |
| Essence de patchouli allongée (exemple II) | 40 |
| Coumarine | 50 |
| Musc cétone | 50 |
| Salicylate d'amyle | 60 |
| Essence de bois de cèdre (américaine) | 60 |
| Citronellol | 60 |
| Acétate de benzyle | 80 |
| Alcool de phényl éthyl | 150 |
| Acétate der terpinyl | 150 |
| | 1000 |

Exemple XIII

On prépare un dilutif de base pour allonger l'essence de patchouli, en mélangeant les ingrédients suivants:

| | Parties |
|---|---|
| Alpha-pinène dimère produit suivant l'exemple IV (A) | 38 |
| Guaioxyde | 27 |
| Oxydate d'isolongifolène | 20 |
| Oméga-hydroxyméthyl longifolène | 10 |
| Cédrol | 3 |
| Essence de bois de santal (Indes orientales) | 2 |
| | 100 |

On incorpore ce mélange (46 parties) dans de l'essence de patchouli naturelle (Seychelles) (60 parties), pour réaliser une essence de patchouli allongée satisfaisante.

Exemple XIV

On incorpore l'essence de patchouli allongée, préparée dans l'exemple XIII, dans une composition de parfumerie combinée du type fougère, contenant les ingrédients suivants:

| | Parties |
|---|---|
| Baume du Pérou | 30 |
| Résine de labdanum | 30 |
| Mousse de chêne (absolu) | 30 |
| Essence de bois de santal (Indes orient.) | 30 |
| Acétate de linalyle | 40 |
| Acétate de terpinyle | 40 |
| Essence de géranium (Bourbon) | 50 |
| Musc ambrette | 50 |
| Coumarine | 60 |
| Salicylate d'amyle | 60 |
| Méthyl ionone | 70 |
| Essence de bois de cèdre (américaine) | 80 |
| Essence de girofle (Zanzibar) | 80 |
| Essence de vétiver (Bourbon) | 80 |
| Essence de patchouli allongée | 130 |
| Essence de lavandin | 140 |
| | 1000 |

Exemple XV

On mélange de l'essence de patchouli (85 parties), provenant des Iles Seychelles, avec le camphène dimère produit suivant l'exemple IV (B) (15 parties). On constate que le camphène dimère agit comme dilutif pour allonger l'essence de patchouli du fait que l'effet odorant caractéristique de cette dernière n'est pas sensiblement modifié.

Exemple XVI

On incorpore avantageusement l'essence de patchouli allongée préparée suivant l'exemple XV, dans une composition combinée du type Chypre, en mélangeant les ingrédients suivants:

| | Parties |
|---|---|
| Aldéhyde cinnamique | 1 |
| Glycidate d'éthyl méthyl phényl | 1 |
| Nonyl acétaldéhyde de méthyle | 2 |
| Mousse de chêne (absolu) | 20 |
| Essence de bois de santal (Indes orient.) | 20 |
| Acétate de vétiveryle | 20 |
| Essence d'ylang n° 1 | 20 |
| Résine de benjoin (Sumatra) | 30 |
| Alpha ionone (100%) | 30 |
| Essence de girofle (Zanzibar) | 36 |
| Essence de bergamote | 40 |
| Hydroxycitronellal | 40 |
| Iso eugénol | 40 |
| Essence de patchouli allongée (ex. XI) | 40 |
| Coumarine | 50 |
| Musc cétone | 50 |
| Salicylate d'amyle | 60 |
| Essence de bois de cèdre (américaine) | 60 |
| Citronellol | 60 |
| Acétate de benzyle | 80 |
| Alcool de phényl éthyl | 150 |
| Acétate de terpinyle | 150 |
| | 1000 |

Exemple XVII

On prépare un dilutif de base pour allonger l'essence de patchouli en mélangeant les ingrédients suivants:

| | Parties |
|---|---|
| Camphène dimère produit suivant l'exemple IV (B) | 38 |
| Guaioxyde | 27 |
| Oxydate d'isolongifolène | 20 |
| Oméga-hydroxyméthyl longifolène | 10 |
| Cédrol | 3 |
| Essence de bois de santal (Indes orient.) | 2 |
| | 100 |

On mélange 46 parties de ce mélange avec 60 parties d'essence de patchouli (Seychelles) pour réaliser une essence de patchouli allongée satisfaisante.

Exemple XVIII

On incorpore l'essence de patchouli allongée, préparée suivant l'exemple XVII, dans une composition combinée de parfumerie du type fougère, contenant les ingrédients suivants:

| | Parties |
|---|---|
| Baume du Pérou | 30 |
| Résine de labdanum | 30 |
| Mousse de chêne (absolu) | 30 |
| Essence de bois de santal (Indes orient.) | 30 |
| Acétate de linalyle | 40 |
| Acétate de terpinyle | 40 |
| Essence de géranium (Bourbon) | 50 |
| Musc ambrette | 50 |
| Coumarine | 60 |
| Salicylate d'amyle | 60 |
| Méthyl Ionone | 70 |
| Essence de bois de cèdre (américaine) | 80 |
| Essence de girofle (Zanzibar) | 80 |
| Essence de vétiver (Bourbon) | 80 |
| Essence de patchouli allongée | 130 |
| Essence de lavandin | 140 |
| | 1000 |

Exemple XIX

On mélange 85 parties d'essence de patchouli (Seychelles) avec le dimère du camphène, produit suivant l'exemple IV (B), 15 parties; On constate que le dimère du camphène agit comme dilutif pour l'essence de patchouli du fait que l'effet odorant caractéristique de cette dernière n'est essentiellement pas modifié.

Exemple XX

On incorpore l'essence de patchouli allongée, préparée suivant l'exemple XIX, avantageusement, dans une composition combinée du type Chypre, en mélangeant les ingrédients suivants:

| | Parties |
|---|---|
| Aldéhyde cinnamique | 1 |
| Glycidate d'éthyl-méthyl-phényl | 1 |
| Nonyl acétaldèhyde de méthyle | 2 |
| Mousse de chêne (absolu) | 20 |
| Essence de bois de santal (Indes orient.) | 20 |
| Acétate de vétivéryl | 20 |
| Essence d'ylang n° 1 | 20 |
| Résine de benjoin (Sumatra) | 30 |
| Alpha Ionone (100%) | 30 |
| Essence de girofle (Zanzibar) | 36 |
| Essence de bergamote | 40 |
| Hydroxycitronellal | 40 |
| Iso Eugénol | 40 |
| Essence de patchouli allongée (Ex. XI) | 40 |
| Coumarine | 50 |
| Musc cétone | 50 |
| Salicylate d'amyle | 60 |
| Essence de bois de cèdre (américaine) | 60 |
| Citronellol | 60 |
| Acétate de benzyle | 80 |
| Alcool de phényl-éthyl | 150 |
| Acétate de terpinyle | 150 |
| | 1000 |

Exemple XXI

On prépare un dilutif de base pour allonger l'essence de patchouli en mélangeant les ingrédients suivants:

| | Parties |
|---|---|
| di-limonène dimère, produit suivant l'exemple IV (C) | 38 |
| Guaioxyde | 27 |
| Oxydate d'isolongifolène | 20 |
| Oméga-hydroxyméthyl longifolène | 10 |
| Cédrol | 3 |
| Essence de bois de santal (Indes orient.) | 2 |
| | 100 |

On mélange ensuite 46 parties de ce mélange avec 60 parties d'essence de patchouli naturelle (Seychelles) pour réaliser une essence de patchouli allongée satisfaisante.

Exemple XXII

L'essence de patchouli allongée préparée suivant l'exemple XVII est incorporée dans une composition de parfumerie combinée du type fougère, contenant les ingrédients suivants:

| | Parties |
|---|---|
| Baume du Pérou | 30 |
| Résine de labdanum | 30 |
| Mousse de chêne (absolu) | 30 |
| Essence de bois de santal (Indes orient.) | 30 |
| Acétate de linalyle | 40 |
| Acétate de terpinyle | 40 |
| Essence de géranium (Bourbon) | 50 |
| Musc ambrette | 50 |
| Coumarine | 60 |
| Salicylate d'amyle | 60 |
| Méthyl Ionone | 70 |
| Essence de bois de cèdre (américaine) | 80 |
| Essence de girofle (Zanzibar) | 80 |
| Essence de vétiver (Bourbon) | 80 |
| Essence de patchouli allongée | 130 |
| Essence de lavandin | 140 |
| | 1000 |

Exemple XXIII

*Préparation de produits de la dimérisation de térébenthine de pâte au sulfate*

Dans un ballon à réaction de 500 ml équipé d'un thermomètre, d'un agitateur, d'un condenseur et d'un entonnoir d'addition, on introduit 5 g de Primol® et 2 g d'un catalyseur à base d'acide phosphorique à 20% sur silice, produit par la Chemtron Corporation. La masse réactionnelle est chauffée à 150°C et on y ajoute, goutte à goutte, 50 g de térébenthine de pâte au sulfate, sur un intervalle de 2 heures tout en agitant. On continue ensuite à chauffer la masse réactionnelle pendant encore 2 heures à 150°C.

On refroidit ensuite la masse réactionnelle et la filtre; le produit de la dimérisation obtenu est un mélange de composés contenant des terpènes monomères qui n'ont pas réagi. Ces terpènes monomères sont distillés et dimérisés à nouveau en utilisant un catalyseur à base d'éthérate de trifluorure de bore. On combine ensuite les produits de la dimérisation obtenus, les distille et les utilise dans les exemples suivants:

Exemple XXIV

On mélange de l'essence de vétiver (70 parties), provenant de Haïti, avec le produit de la dimérisation obtenu suivant l'exemple XXIII. On constate que le produit de la dimérisation ainsi obtenu agit comme dilutif de l'essence de vétiver du fait que l'effet odorant caractéristique de ce dernier n'est essentiellement pas modifié.

Exemple XXV

On mélange de l'essence de bois de santal (75 parties) provenant d'Indonésie, avec le produit de dimérisation obtenu suivant l'exemple XXIII (25 parties). On constate que le produit de la dimérisation ainsi obtenu agit comme dilutif pour allonger l'essence de bois de santal du fait que l'effet odorant caractéristique de cette essence n'est essentiellement pas modifié.

Exemple XXVI

On mélange de l'essence de patchouli (80 parties), provenant des Iles Seychelles, avec le produit de la dimérisation de l'alpha-méthylstyrène obtenu suivant l'un des exemples I, II ou III (20 parties). On constate que ce produit de la dimérisation de l'alpha-méthylstyrène agit comme dilutif pour allonger l'essence de patchouli du fait que l'effet odorant caractéristique de cette dernière n'est essentiellement pas modifié.

Exemple XXVII

On introduit avantageusement l'essence de patchouli allongée, préparée suivant l'exemple XXVI dans une composition combinée du type Chypre, en mélangeant les ingrédients suivants:

| | Parties |
|---|---|
| Aldéhyde cinnamique | 1 |
| Glycidate d'éthyl-méthyl-phényl | 1 |
| Nonyl acétaldéhyde méthyle | 2 |
| Mousse de chêne (absolu) | 20 |
| Essence de bois de santal (Indes orient.) | 20 |
| Acétate de vétiveryl | 20 |
| Essence d'ylang n° 1 | 20 |
| Résine de benjoin (Sumatra) | 30 |
| Alpha Ionone (100%) | 30 |
| Essence de girofle (Zanzibar) | 36 |
| Essence de bergamote | 40 |
| Hydroxycitronellal | 40 |
| Iso Eugénol | 40 |
| Essence de patchouli allongée (Ex. XXVI) | 40 |
| Coumarine | 50 |
| Musc cétone | 50 |
| Salicylate d'amyle | 60 |
| Essence de bois de cèdre (américaine) | 60 |
| Citronellol | 60 |
| Acétate de benzyle | 80 |
| Alcool de phényl-éthyle | 150 |
| Acétate de terpinyle | 150 |
| | 1000 |

Exemple XXVIII

On prépare un dilutif de base pour allonger l'essence de patchouli en mélangeant les ingrédients suivants:

| | Parties |
|---|---|
| Produits de la dimérisation de l'alpha-méthylstyrène obtenus suivant les exemples I, II ou III | 38 |
| Galaxolide | 27 |
| Oxydate d'isolongifolène | 20 |
| Oméga-hydroxyméthyl longifolène | 10 |
| Cédrol | 3 |
| Essence de bois de santal (Indes orient.) | 2 |
| | 100 |

On mélange ensuite ce mélange (46 parties) avec de l'essence naturelle de patchouli (Seychelles) (60 parties) pour réaliser une essence de patchouli allongée satisfaisante.

Exemple XXIX

On incorpore l'essence de patchouli allongée préparée suivant l'exemple XXVIII dans une composition de parfumerie combinée du type fougère, contenant les ingrédients suivants:

| | Parties |
|---|---|
| Baume du Pérou | 30 |
| Résine de labdanum | 30 |
| Mousse de chêne (absolu) | 30 |
| Essence de bois de santal (Indes orient.) | 30 |
| Acétate de linalyl | 40 |
| Acétate de terpinyle | 40 |
| Essence de géranium (Bourbon) | 50 |
| Musc ambrette | 50 |
| Coumarine | 60 |
| Salicylate d'amyle | 60 |
| Méthyl Ionone | 70 |
| Essence de bois de cèdre (américaine) | 80 |
| Essence de girofle (Zanzibar) | 80 |
| Essence de vétiver (Bourbon) | 80 |
| Essence de patchouli allongée | 130 |
| Essence de lavandin | 140 |
| | 1000 |

Les matières parfumées contenant un diluant et une huile essentielle utilisées dans l'invention, en plus de leur utilisation pour augmenter ou exalter l'arôme de matières parfumées, eaux de Cologne et objets parfumés, amélioreront aussi les propriétés désodorisantes des matières de consommation de ce genre, quand elles seront présentes en proportion appropriées.

*Description des figures:*

— La figure 1 est le profil GLC du produit obtenu suivant l'exemple 1, fraction 19.

— La figure 2 est le spectre infra rouge du produit obtenu suivant l'exemple 1, fraction 19.

— La figure 3 est le spectre NMR du produit obtenu suivant l'exemple 1, fraction 19.

— La figure 4 est le profil GLC du produit obtenu suivant l'exemple 1, fraction 3.

— La figure 5 est le spectre IR du produit obtenu suivant l'exemple 1, fraction 3, ainsi que du produit de l'exemple III.

— La figure 6 est le spectre NMR du produit obtenu suivant l'exemple 1, fraction 3, ainsi que du produit obtenu suivant l'exemple III.

— La figure 7 est le spectre de masse du produit obtenu suivant l'exemple 1, fraction 3, ainsi que du produit obtenu suivant l'exemple III.

— La figure 8 est le profil GLC du produit obtenu suivant l'exemple III, fractions 9 à 12, où l'on a utilisé un Filtrol comme catalyseur pour l'opération de dimérisation.

— La figure 9 est le profil GLC du produit de la dimérisation de l'alpha-pinène obtenu suivant l'exemple IV (A).

— La figure 10 est le profil GLC du produit de la dimérisation du camphène obtenu suivant l'exemple IV (B).

— La figure 11 est le spectre NMR du produit obtenu suivant l'exemple IV (A).

— La figure 12 est le spectre infra rouge du produit obtenu suivant l'exemple IV (A).

— La figure 13 est le profil GLC du produit obtenu suivant l'exemple IV (C), produit de la dimérisation du limonène.

— La figure 14 est le spectre NMR du produit obtenu suivant l'exemple IV (C), dimère du limonène.

— La figure 15 est le spectre infra rouge du produit obtenu suivant l'exemple IV (C), dimère du limonène.

**Revendications**

1. Composition pour parfumerie combinée, en phase liquide unique, caractérisée en ce qu'elle comprend (i) une substance utilisée en parfumerie, sous forme non diluée et, en variante, seul ou en mélange, (ii) un diterpène ou un diterpène hydrogéné, ou un mélange d'un diterpène et d'un diterpène hydrogéné, et/ou (iii) un produit de la dimérisation de l'alpha-méthylstyrène et/ou un produit de la dimérisation de l'alpha-méthylstyrène hydrogéné et/ou un produit de la dimérisation d'un homologue méthylé de l'alpha méthylstyrène et/ou un produit de la dimérisation d'un homologue méthylé de l'alpha-méthylstyrène hydrogéné, ces produits de dimérisation (ii) et/ou (iii) étant présents dans une proportion comprise entre 1 et 30 parties en poids pour 100 parties en poids de la composition pour parfumerie combinée, en phase liquide unique.

2. Composition pour parfumerie suivant la revendication 1, caractérisée en ce qu'elle contient une essence de parfumerie naturelle ou une essence de parfumerie synthétique ou un produit chimique odorant synthétique, ou un mélange d'au moins deux essences de parfumerie naturelles, ou un mélange d'au moins deux essences de parfumerie synthétiques, ou un mélange d'au moins deux produits chimiques odorants, ou un mélange d'au moins une essence de parfumerie naturelle, au moins une essence de parfumerie synthétique et au moins un produit chimique odorant auxquels il est mélangé intimement un produit de réaction choisi dans le groupe constitué par, (A) un ou plusieurs produits, contenant vingt atomes de carbone, de la dimérisation de, (i) un terpène monocyclique contenant deux doubles liaisons carbone-carbone, ou (ii) deux terpènes monocycliques différents contenant deux doubles liaisons carbone-carbone, ou (iii) un terpène bicyclique contenant une double liaison carbone-carbone, on (iv), deux terpènes bicycliques différents, contenant chacun une double liaison carbone-carbone, ou (v) un terpène monocyclique contenant deux doubles liaisons carbone-carbone et un terpène bicyclique contenant une double liaison carbone-carbone, (B) les produits de l'hydrogénation d'un ou plusieurs produits, contenant vingt atomes de carbone, de la dimérisation de terpènes, et (C) des mélanges d'un ou plusieurs produits, contenant vingt atomes de carbone, de la dimérisation de terpènes, et d'un ou plusieurs produits de l'hydrogénation d'un ou plusieurs produits, contenant vingt atomes de carbone, de la dimérisation de terpènes, ce produit de réaction étant présent dans une concentration de 1 à 30 parties en poids environ, pour 100 parties en poids de la composition pour parfumerie combinée, en phase liquide unique.

3. Composition suivant la revendication 2, caractérisée en ce que le produit de réaction est un produit de dimérisation choisi dans le groupe constitué des:

Produits de la dimérisation de l'alpha-pinène;

Produits de la dimérisation de la bêta-pinène;

Produits de la dimérisation du camphène;
Produits de la dimérisation du d-limonène;
Produits de la dimérisation de la térébenthine;
Produits de la dimérisation de l'alpha-pinène hydrogénés;
Produits de la dimérisation de la bêta-pinène hydrogénés;
Produits de la dimérisation du camphène hydrogénés;
Produits de la dimérisation du d-limonène hydrogénés;
Produits de la dimérisation de la térébenthine hydrogénés;
Mélanges de ces produits de la dimérisation et
Mélanges de ces produits de la dimérisation, hydrogénés.

4. Procédé pour allonger un produit de parfumerie choisi dans le groupe constitué par les essences naturelles de parfumerie, les essences synthétiques de parfumerie, les produits chimiques synthétiques odorants, les mélanges d'essences naturelles et d'essences synthetiques, les mélanges d'essences naturelles, d'essences synthétiques et de produits chimiques odorants, et les mélanges d'essences synthétiques et de produits chimiques odorants, sans altérer sensiblement leur arôme, caractérisé en ce qu'on mélange intimement, avec 70 à 99 parties en poids du produit de parfumerie, de 1 jusqu'à 30 parties en poids d'un produit de réaction qui est soit en mélange, soit en variante séparée constitué par (A) des produits de la dimérisation de (i) des terpènes monocycliques semblables ou différents contenant dix atomes de carbone et contenant deux doubles liaisons carbone-carbone, ou (ii) des terpènes bicycliques semblables ou différents, contenant dix atomes de carbone et contenant une double liaison carbone-carbone, ou (iii) un terpène monocyclique contenant deux doubles liaisons carbone-carbone et un terpène bicyclique contenant une double liaison carbone-carbone, (B) des produits de l'hydrogénation de ces produits de la dimérisation, (C) des produits de la dimérisation de l'alpha-méthylstyrène, et/ou des produits de la dimérisation de l'alphaméthylstyrène hydrogénés, ces produits de la dimérisation de l'alphaméthylstyrène étant définis par au moins une structure générique choisie dans le groupe constitué par les produits répondant aux formules:

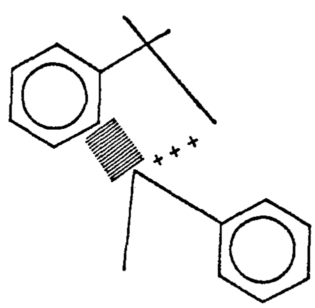

et

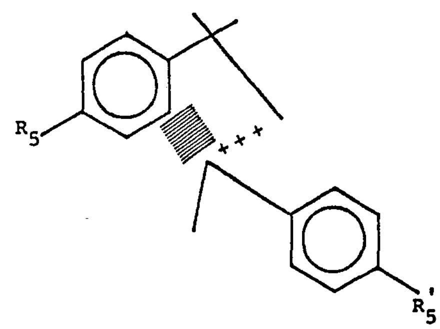

où l'un de $R_5$ et $R'_5$ est une méthyle, l'autre étant un hydrogène, ou $R_5$ et $R'_5$ sont, tous deux, des méthyles, où la ligne |||||||||||| représente une simple liaison carbone-carbone ou aucune liaison, et où la ligne +++++++ représente une simple liaison carbone-carbone ou une double liaison carbone-carbone, avec cette condition que si la ligne +++++++ est une double liaison carbone-carbone, la ligne |||||||||||| ne représente aucune liaison, et si la ligne +++++++ est une simple liaison carbone-carbone, la ligne |||||||||||| est alors une simple liaison carbone-carbone, les produits de la dimerisation de l'alpha-méthylstyrène hydrogénés possédant au moins une des structures génériques choisies dans le groupe des produits répondant aux formules:

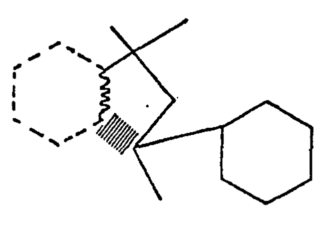

et

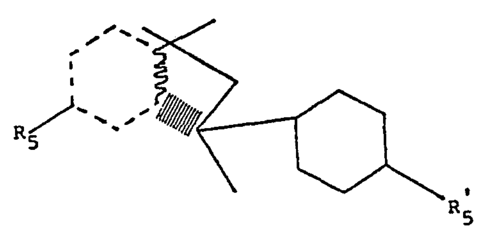

où l'un de $R_5$ et $R'_5$ est un méthyle et l'autre un hydrogène, où $R_5$ et $R'_5$ sont, tous deux; un méthyle, où les lignes en tirets et la ligne ondulée représentent des simples liaisons carbone-carbone ou des doubles liaisons carbone-carbone, avec cette condition que si une double liaison se trouve sur le cycle contenant une ligne ondulée et des lignes en tirets, seule le ligne ondulée pourra être cette double liaison et que s'il existe plus d'une double liaison sur le cycle contenant les lignes en tirets et la ligne ondulée, ce cycle contenant les lignes en tirets et la ligne ondulée est un noyau benzénique, enfin, dans ces formules la ligne |||||||||||| représente soit une simple liaison carbone-carbone, soit aucune liaison.

5. Composition suivant la revendication 1, caractérisé en ce qu'elle comprend une essence de parfumerie naturelle ou une essence de parfumerie synthétique ou un mélange d'essence de parfumerie naturelle et d'essence de parfumerie synthétique ou d'un produit chimique odorant, avec lesquels on a mélangé intimement de 1 à 30 parties en poids d'au moins un dilutif inodore pour parfumerie qui est un produit de dimérisation choisi dans le groupe constitué par les produits de la dimérisation de l'alpha-méthylstyrène, les produits de la dimérisation d'un homologue méthylé de l'alpha-méthylstyrène, les produits de la dimérisation de l'alphaméthylstyrène hydrogénés, et les produits de la dimérisation d'un homologue méthylé de l'alpha-méthylstyrène hydrogénés, pour 100 parties en poids d'essence de parfumerie ou de produit chimique odorant la produit de la dimérisation de l'alpha-méthylstyrène ayant au moins une structure choisie dans le groupe formé par les structures suivantes:

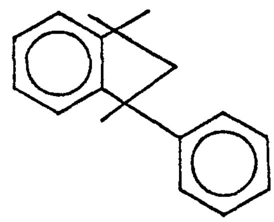

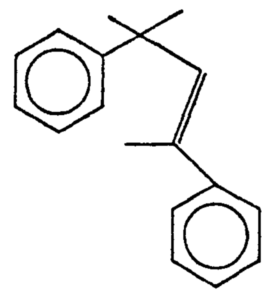

le produit de la dimérisation de l'alpha-méthylstyrène hydrogéné présentant au moins une structure qui se définisse par la structure générique:

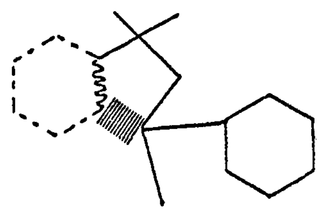

le produit de la dimérisation de l'homologue méthylé de l'alpha-méthylstyrène présentant au moins une des structures choisie dans le groupe constitué par les formules:

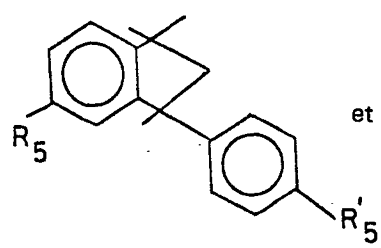

et

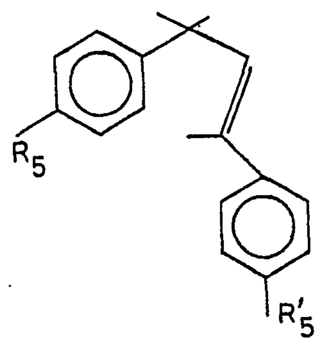

le produit de la dimérisation de l'homologue méthylé de l'alpha-méthylstyrène hydrogéné présentant au moins une des structures répondant aux formules suivantes:

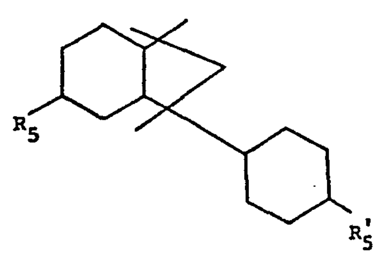

,

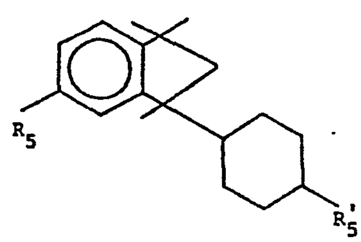

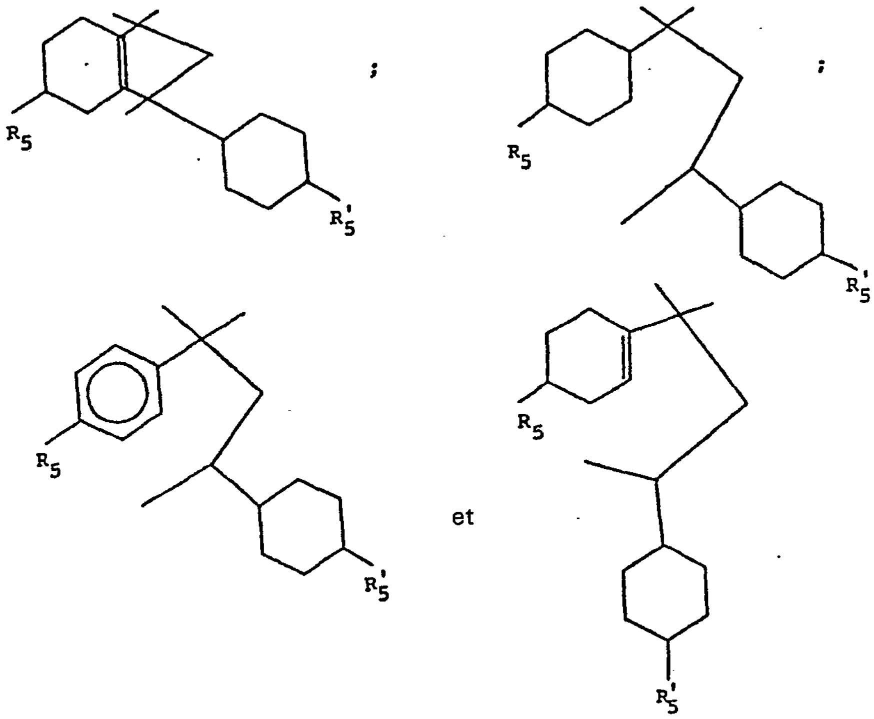

;

;

et

où l'un de $R_5$ ou $R'_5$ est un méthyle, l'autre étant un hydrogéne, ou $R_5$ et $R'_5$ sont tous deux un groupe méthyle, où les lignes en tirets et la ligne ondulée représentent des simples liaisons carbone-carbone ou des doubles liaisons carbone-carbone, avec cette condition que, s'il se trouve une double liaison dans le cycle contenant les lignes en tirets et la ligne ondulée, seule la ligne ondulée représentera une double liaison, et s'il existe plus d'une double liaison dans ce cycle contenant les lignes en tirets et la ligne ondulée, ce cycle est un noyau benzénique, la ligne ||||||||||||| représentant, soit une simple liaison carbone-carbone, soit aucune liaison.

6. Composition suivant la revendication 5 caractérisée en ce que le produit de la dimérisation est un alpha-méthylstyrène dimère répondant à la formule:

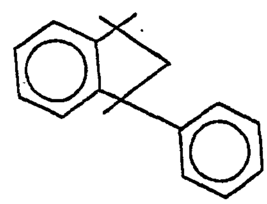

7. Composition suivant la revendication 5 caractérisée en ce que le produit de la dimérisation est un produit de dimérisation hydrogené présentant au moins une structure choisie dans le groupe constitué par les produits répondant aux formules suivantes:

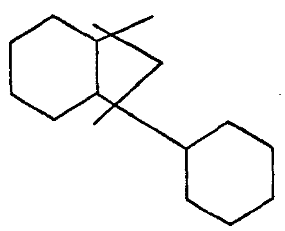

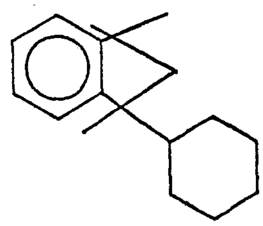

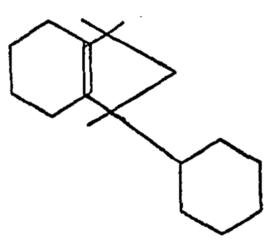

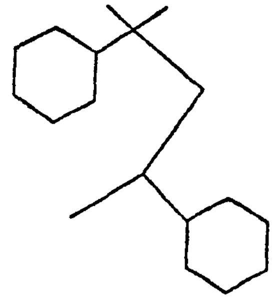

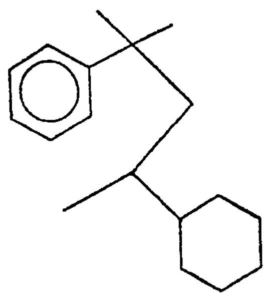

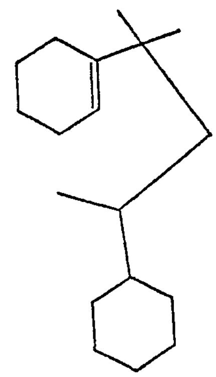

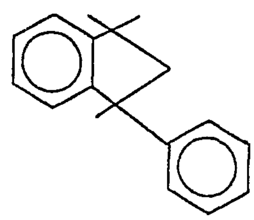

8. Mélange caractérisé en ce qu'il comprend, (A) un produit de la dimérisation d'un alpha-méthylstyrène ou d'un homologue méthylé ou alcoylé avec un autre alcoyle en $C_2$ à $C_4$ de ce produit ou un mélange des précédents ayant une des structures génériques suivantes:

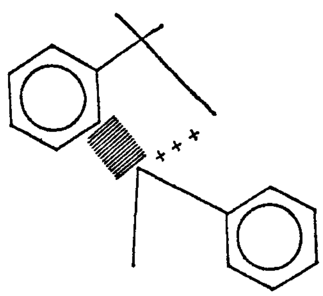

et

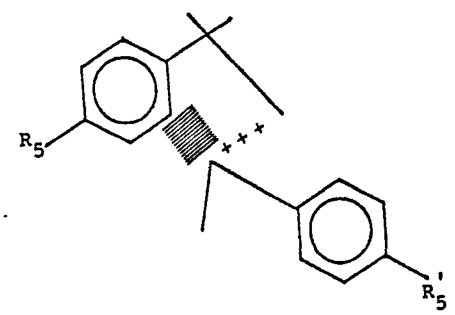

ou leurs dérivés hydrogénés, ou un mélange des précédents dans lequel un de $R_5$ et $R'_5$ est un alcoyle en $C_1$ à $C_4$, l'autre étant un hydrogène, ou $R_5$ et $R'_5$ sont tous deux un alcoyle en $C_1$ à $C_4$, dans lequel la ligne |||||||| représente une simple liaison carbone-carbone ou aucune liaison et dans lequel la ligne +++++++ représente une simple liaison carbone-carbone ou une double liaison carbone-carbone avec cette condition que si la ligne ++++++ est une double liaison carbone-carbone, la ligne |||||| ne représente alors aucune liaison et si la ligne +++++ est une simple liaison carbone-carbone, la ligne |||||||| est une simple liaison carbone-carbone, ce produit de la dimérisation de l'alpha-méthylstyrène hydrogéné présentant au moins une structure définie suivant au moins une des structures génériques choisies dans le groupe constitué par les formules:

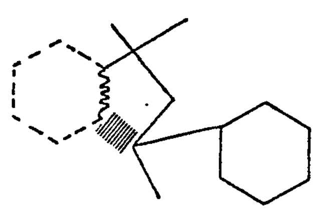

et

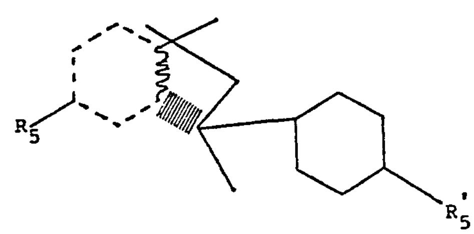

où l'un de $R_5$ et $R'_5$ est un alcoyle en $C_1$ à $C_4$, l'autre étant un hydrogène, ou $R_5$ et $R'_5$ représentent chacun un alcoyle en $C_1$ à $C_4$, où les lignes en tirets et la ligne ondulée représentent des simples liaisons carbone-carbone ou des doubles liaisons carbone-carbone, avec cette condition que s'il se trouve une double liaison présente sur le cycle contenant la ligne ondulée et les lignes en tirets, seule la ligne ondulée représente une double liaison, et s'il se trouve plus d'une double liaison dans le cycle contenant les lignes en tirets et la ligne ondulée, ce cycle contenant les lignes en tirets

et la ligne ondulée est un noyau benzénique, et où la ligne |||||||||| représente une simple liaison carbone-carbone ou aucune liaison, et (B) un produit de réaction choisi dans le groupe constitué par (I) un ou plusieurs produits, contenant vingt atomes de carbone, de la dimérisation de, (i) un terpène monocyclique contenant deux doubles liaisons carbone-carbone, ou (ii) deux terpènes monocycliques différents contenant deux doubles liaisons carbone-carbone, ou (iii) un terpène bicyclique contenant une double liaison carbone-carbone, ou (iv) deux terpènes bicycliques différents, contenant chacun une double liaison carbone-carbone, ou (v), un terpène monocyclique contenant deux doubles liaisons carbone-carbone et un terpène bicyclique contenant une double liaison carbone-carbone, (II) de produits de l'hydrogénation d'un ou plusieurs produits, contenant vingt atomes de carbone, provenant de la dimérisation de terpènes, et un ou plusieurs produits de l'hydrogénation d'un ou plusieurs produits de la dimérisation de terpènes contenant vingt atomes de carbone, ledit mélange étant destiné à être incorporé dans une essence de parfumerie naturelle ou une essence de parfumerie synthétique ou un produit chimique odorant synthétique, ou un mélange d'au moins deux essences de parfumerie naturelles, ou un mélange d'au moins deux essences de parfumerie synthétiques, ou un mélange d'au moins deux produits chimiques odorants, ou un mélange d'au moins une essence de parfumerie naturalle, d'au moins une essence de parfumerie synthétique et d'au moins un produit chimique odorant pour former une composition pour parfumerie combinée en phase liquide unique, dans une proportion comprise entre 1 et 30 parties en poids pour 100 parties en poids de la composition pour parfumerie en phase liquide unique.

9. Mélange suivant la revendication 8 caractérisé en ce que le produit de réaction (B) est un produit de dimérisation choisi dans le groupe constitué par les produits suivants:
Produits de la dimérisation de l'alpha-pinène;
Produits de la dimérisation de la bêta-pinène;
Produits de la dimérisation du camphène;
Produits de la dimérisation du d-limonène;
Produits de la dimérisation de la térébenthine;
Produits de la dimérisation de l'alpha-pinène hydrogénés;
Produits de la dimérisation de la bêta-pinène hydrogénés;
Produits de la dimérisation du camphène hydrogénés;
Produits de la dimérisation du d-limonène hydrogénés;
Produits de la dimérisation de la térébenthine hydrogénès;
Mélanges de ces produits de la dimérisation et
Mélanges de ces produits de la dimérisation, hydrogénés.

10. Composition pour parfumerie combinée, à phase unique liquide, caractérisée en ce qu'elle contient au moins une essence naturelle de parfumerie, une essence de parfumerie synthétique ou un produit chimique odorant, ou un mélange d'essences de parfumerie naturelles et d'essences de parfumerie synthétiques, ou un mélange d'essence de parfumerie synthétiques et de produits chimiques odorants, avec lesquels on mélange intimement de 1 â 30 parties en piods environ d'au moins un dilutif inodore pour parfumerie qui est constitué par un mélange suivant la revendication 8 pour 100 parties èn poids de la composition pour parfumerie combinée à phase unique liquide.

**Patentansprüche**

1. Kombinierte Parfüm-Komposition in einer einzigen flüssigen Phase, dadurch gekennzeichnet, daß sie
(i) eine in der Parfümerie verwendete Substanz in unverdünnter Form und als Variante einzeln oder in Mischung
(ii) ein Diterpen oder ein hydriertes Diterpen oder eine Mischung aus einem Diterpen und einem hydrierten Diterpen und/oder
(iii) ein Produkt der Dimerisation von $\alpha$-Methylstyrol und/oder ein hydriertes Produkt der Dimerisation von $\alpha$-Methylstyrol und/oder ein Produkt der Dimerisation eines Methylhomologen von $\alpha$-Methylstyrol und/oder ein hydriertes Produkt der Dimerisation eines Methylhomologen von $\alpha$-Methylstyrol enthält, wobei die Dimerisationsprodukte (ii) und/oder (iii) in einer Menge zwischen 1 und 30 Gew.-Teilen pro 100 Gew.-Teile der kombinierten Parfüm-Komposition in einer einzigen flüssigen Phase vorhanden sind.

2. Parfum-Komposition nach Anspruch 1, dadurch gekennzeichnet, daß sie ein natürliches Parfümöl oder ein synthetisches Parfümöl oder einen synthetischen chemischen Riechstoff oder eine Mischung aus mindestens zwei natürlichen Parfümölen oder eine Mischung aus mindestens zwei synthetischen Parfümölen oder eine Mischung aus mindestens zwei chemischen Riechstoffen oder eine Mischung aus mindestens einem natürlichen Parfümöl, mindestens einem synthetischen Parfümöl und mindestens einem chemischen Riechstoff enthält, mit welchen Bestandteilen ein Reaktionsprodukt innig vermischt ist, das aus der Gruppe ausgewählt ist, die
(A) eines oder mehrere Produkte mit 20 Kohlenstoffatomen der Dimerisation
(i) eines monocyclischen Terpens mit zwei Kohlenstoff-Kohlenstoff-Doppelbindungen oder

(ii) zweier verschiedener monocyclischer Terpene mit zwei Kohlenstoff-Kohlenstoff-Doppelbindungen oder

(iii) eines bicyclischen Terpens mit einer Kohlenstoff-Kohlenstoff-Doppelbindung oder

(iv) zweier verschiedener bicyclischer Terpene, die jeweils eine Kohlenstoff-Kohlenstoff-Doppelbindung aufweisen, oder

(v) eines monocyclischen Terpens mit zwei Kohlenstoff-Kohlenstoff-Doppelbindungen und eines bicyclischen Terpens mit einer Kohlenstoff-Kohlenstoff-Doppelbindung,

(B) die Produkte der Hydrierung eines oder mehrerer Produkte mit 20 Kohlenstoffatomen der Dimerisation von Terpenen und

(C) Mischungen aus einem oder mehreren Produkten mit 20 Kohlenstoffatomen der Dimerisation von Terpenen und einem oder mehreren Produkten der Hydrierung eines oder mehrerer Produkte mit 20 Kohlenstoffatomen der Dimerisation von Terpenen, umfaßt,

wobei dieses Reaktionsprodukt in einer Konzentration von etwa 1 bis 30 Gew.-Teilen pro 100 Gew.-Teile der kombinierten Parfüm-Komposition in einer einzigen flüssigen Phase vorhanden ist.

3. Komposition nach Anspruch 2, dadurch gekennzeichnet, daß das Reaktionsprodukt ein Dimerisationsprodukt ist, das aus der Gruppe ausgewählt ist, die

Produkte der Dimerisation von $\alpha$-Pinen;
Produkte der Dimerisation von $\beta$-Pinen;
Produkte der Dimerisation von Kamphen;
Produkte der Dimerisation von d-Limonen;
Produkte der Dimerisation von Terebenthin;
hydrierte Produkte der Dimerisation von $\alpha$-Pinen;
hydrierte Produkte der Dimerisation von $\beta$-Pinen;
hydrierte Produkte der Dimerisation von Kamphen;
hydrierte Produkte der Dimerisation von d-Limonen;
hydrierte Produkte der Dimerisation von Terebenthin;
Mischungen dieser Dimerisationsprodukte und
Mischungen dieser hydrierten Dimerisationsprodukte umfaßt.

4. Verfahren zum Strecken eines Parfümerieprodukts, ausgewählt aus der Gruppe, die natürliche Parfümöle, synthetische Parfümöle, synthetische chemische Riechstoffe, Mischungen aus natürlichen und synthetischen Parfümölen, Mischungen aus natürlichen Parfümölen, synthetischen Parfümölen und chemischen Riechstoffen und Mischungen aus synthetischen Parfümölen und chemischen Riechstoffen umfaßt, ohne wesentliche Veränderung ihres Duftes, dadurch gekennzeichnet, daß man 70 bis 99 Gew.-Teile des Parfümerieprodukte innig mit 1 bis 30 Gew.-Teilen eines Reaktionsprodukts vermischt, welches entweder in Form einer Mischung oder in getrennter Form besteht aus

(A) Produkten der Dimerisation von

(i) ähnlichen oder verschiedenartigen monocyclischen Terpenen mit 10 Kohlenstoffatomen und zwei Kohlenstoff-Kohlenstoff-Doppelbindungen oder

(ii) ähnlichen oder verschiedenartigen bicyclischen Terpenen mit 10 Kohlenstoffatomen und einer Kohlenstoff-Kohlenstoff-Doppelbindung oder

(iii) eines monocyclischen Terpens mit zwei Kohlenstoff-Kohlenstoff-Doppelbindungen und eines bicyclischen Terpens mit einer Kohlenstoff-Kohlenstoff-Doppelbindung,

(B) Produkten der Hydrierung dieser Dimerisationsprodukte und

(C) Produkten der Dimerisation von $\alpha$-Methylstyrol und/oder hydrierten Produkten der Dimerisation von $\alpha$-Methylstyrol, wobei diese Produkte der Dimerisation von $\alpha$-Methylstyrol durch mindestens eine allgemeine Struktur definiert sind, die aus der Gruppe ausgewählt ist, welche die Produkte der nachstehenden Formeln umfaßt:

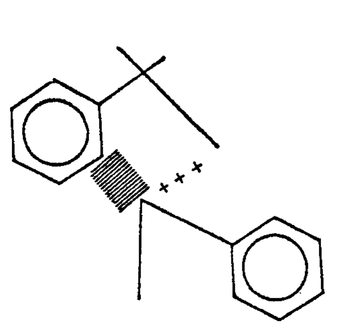

und

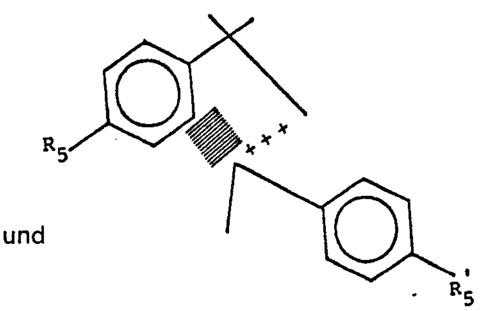

worin eine der Gruppen $R_5$ und $R'_5$ eine Methylgruppe und die andere ein Wasserstoffatom oder beide Gruppen $R_5$ und $R'_5$ Methylgruppen bedeuten, die Linie |||||||||| für eine Kohlenstoff-Kohlenstoff-Einfachbindung oder keine Bindung und die Linie +++++++++ für eine Kohlenstoff-Kohlenstoff-Einfachbindung oder eine Kohlenstoff-Kohlenstoff-Doppelbindung mit der Maßgabe

stehen, daß, wenn die Linie ++++++++ eine Kohlenstoff-Kohlenstoff-Doppelbindung bedeutet, die Linie |||||||||||| keine Bindung darstellt, und wenn die Linie ++++++++ eine Kohlenstoff-Kohlenstoff-Einfachbindung darstellt, die Linie |||||||||||| eine Kohlenstoff-Kohlenstoff-Einfachbindung bedeutet, wobei die hydrierten Produkte der Dimerisation von $\alpha$-Methylstyrol mindestens eine allgemeine Struktur aufweisen, die aus der Gruppe ausgewählt ist, die die Produkte der nachstehenden Formeln umfaßt:

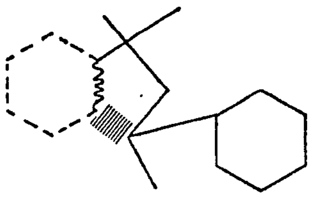

und

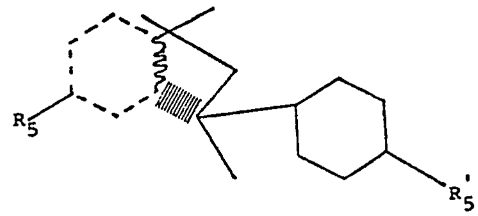

worin eine der Gruppen $R_5$ und $R'_5$ eine Methylgruppe und die andere ein Wasserstoffatom oder beide Gruppen $R_5$ und $R'_5$ Methylgruppen bedeuten und die gestrichelte Linie und die Wellenlinie für Kohlenstoff-Kohlenstoff-Einfachbindungen oder Kohlenstoff-Kohlenstoff-Doppelbindungen mit der Maßgabe stehen, daß, wenn in den eine Wellenlinie und gestrichelte Linien umfassenden Ringen eine Doppelbindung vorliegt, nur die Wellenlinie für diese Doppelbindung steht, und daß, wenn mehr als eine Doppelbindung in dem die gestrichelten Linien und die Wellenlinie umfassenden Ring vorhanden ist, dieser gestrichelte Linien und die Wellenlinie umfassende Ring ein Benzolkern ist und wobei in diesen Formeln die Linie |||||||||||||| entweder für eine Kohlenstoff-Kohlenstoff-Einfachbindung oder für keine Bindung steht.

5. Komposition nach Anspruch 1, dadurch gekennzeichnet, daß sie ein natürliches Parfümöl oder ein synthetisches Parfümöl oder eine Mischung aus einem natürlichen Parfümöl und einem synthetischen Parfümöl oder einen chemischen Riechstoff unfaßt, mit welchen pro 100 Gew.-Teilen des Parfümöls oder des chemischen Riechstoffs 1 bis 30 Gew.-Teile mindestens eines geruchlosen Parfümerieverdünnungsmittels innig vermischt worden sind, bei dem es sich um ein Dimerisationsprodukt handelt, das aus der Gruppe ausgewählt ist, welche Produkte der Dimerisation von $\alpha$-Methylstyrol, Produkte der Dimerisation eines Methylhomologen von $\alpha$-Methylstyrol, hydrierte Produkte der Dimerisation von $\alpha$-Methylstyrol und hydrierte Produkte der Dimerisation eines Methylhomologen von $\alpha$-Methylstyrol umfaßt, wobei das Produkt der Dimerisation von $\alpha$-Methylstyrol mindestens eine Struktur aufweist, die aus der Gruppe der folgenden Strukturen ausgewählt ist:

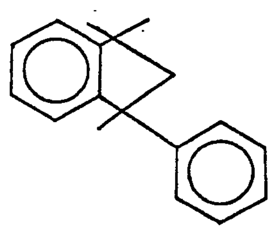

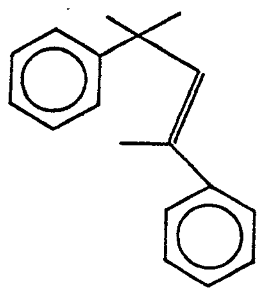

wobei das hydrierte Produkt der Dimerisation von $\alpha$-Methylstyrol mindestens eine Struktur aufweist, die durch die folgende allgemeine Struktur definiert ist:

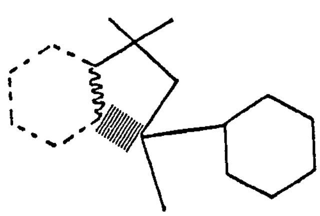

wobei das Produkt der Dimerisation des Methylhomologen von $\alpha$-Methylstyrol mindestens eine Struktur aus der Gruppe aufweist, die die folgenden Formeln umfaßt:

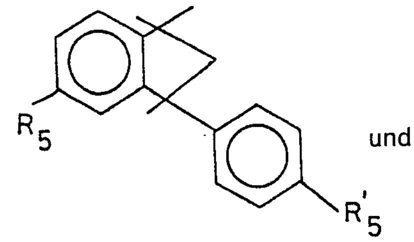

und

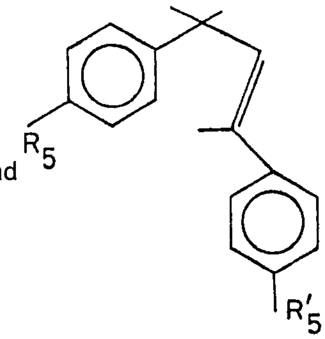

und

wobei das hydrierte Produkte der Dimerisation des Methylhomologen von $\alpha$-Methylstyrol mindestens eine Struktur der nachstehenden Formeln aufweist:

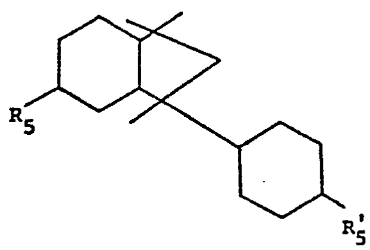

;

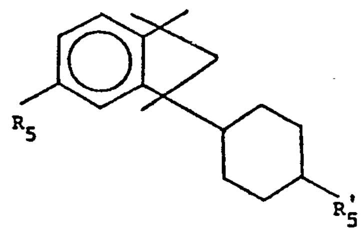

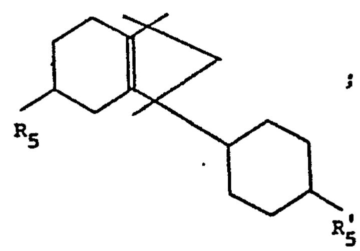

;

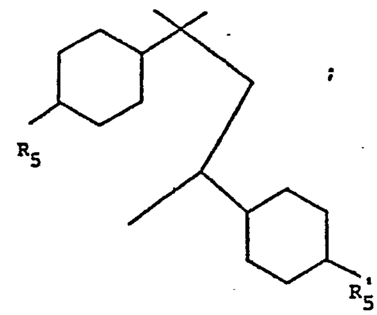

;

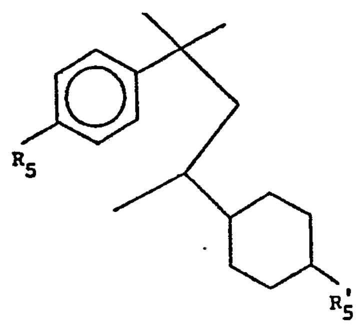

und

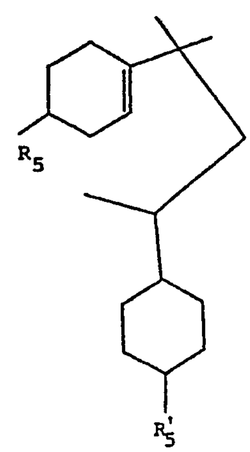

worin eine der Gruppen $R_5$ und $R'_5$ eine Methylgruppe und die andere ein Wasserstoffatom oder beide Gruppen $R_5$ und $R'_5$ Methylgruppen bedeuten, die gestrichelten Linien und die Wellenlinie für Kohlenstoff-Kohlenstoff-Einfachbindungen oder Kohlenstoff-Kohlenstoff-Doppelbindungen mit der Maßgabe stehen, daß, wenn in dem gestrichelte Linien und die Wellenlinie aufweisenden Ring eine Doppelbindung vorliegt, lediglich die Wellenlinie für eine Doppelbindung steht und wenn mehr also eine Doppelbindung in diesem gestrichelte Linien und die Wellenlinie aufweisenden Ring vorhanden ist, dieser Ring ein Benzolkern ist, und die Linie ||||||||| für entweder eine Kohlenstoff-Kohlenstoff-Einfachbindung oder für keine Bindung steht.

6. Komposition nach Anspruch 5, dadurch gekennzeichnet, daß das Dimerisationsprodukt ein dimeres $\alpha$-Methylstyrol der Formel

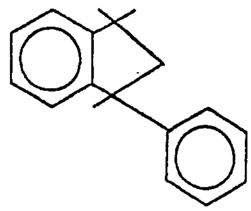

ist.

7. Komposition nach Anspruch 5, dadurch gekennzeichnet, daß das Dimerisationsprodukt ein hydriertes Dimerisationsprodukt ist, das mindestens eine Struktur aufweist, die aus der Gruppe ausgewählt ist, die die Produkte der folgenden Formeln umfaßt:

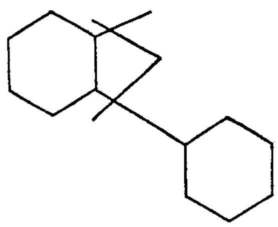

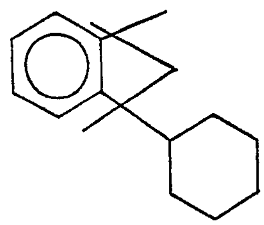

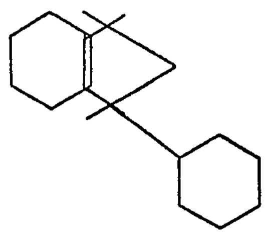

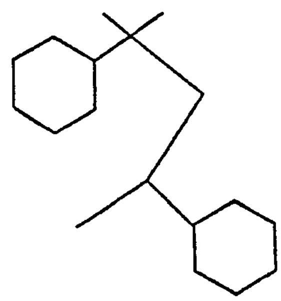

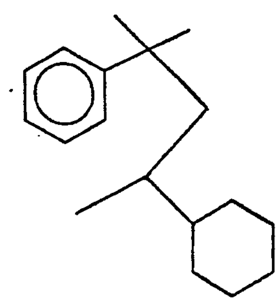

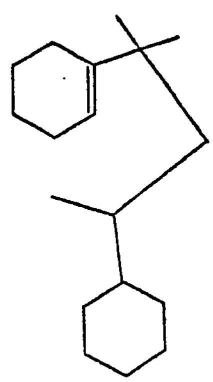

und

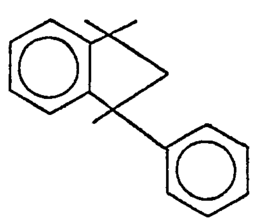

8. Mischung, dadurch gekennzeichnet, daß sie

(A) ein Produkt der Dimerisation eines $\alpha$-Methylstyrols oder eines Methylhomologen oder Alkylhomologen mit einer anderen $C_2$—$C_4$-Alkylgruppe dieses Produkts oder eine Mischung dieser Verbindungen mit einer der nachstehenden allgemeinen Strukturen:

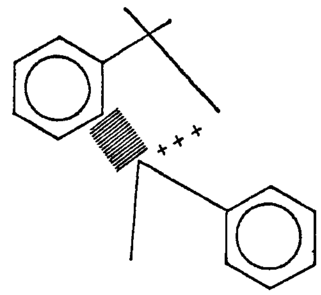 und 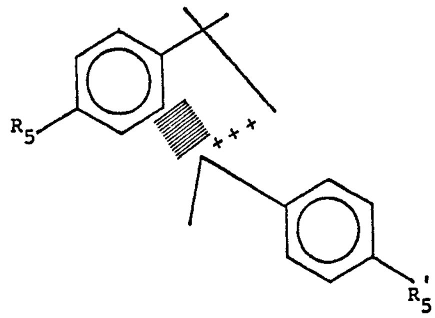

oder deren hydrierte Derivate oder eine Mischung dieser Verbindungen, wobei eine der Gruppen $R_5$ und $R'_5$ eine $C_1$—$C_4$-Alkylgruppe und die andere ein Wasserstoffatom oder beide Gruppen $R_5$ und $R'_5$ eine $C_1$—$C_4$-Alkylgruppe bedeuten, die Linie |||||||||| für eine Kohlenstoff-Kohlenstoff-Einfachbindung oder keine Bindung steht und die Linie +++++++ für eine Kohlenstoff-Kohlenstoff-Einfachbindung oder eine Kohlenstoff-Kohlenstoff-Doppelbindung mit der Maßgabe steht, daß, wenn die Linie ++++++ eine Kohlenstoff-Kohlenstoff-Doppelbindung darstellt, die Linie |||||||||| für keine Bindung steht und wenn die Linie ++++++ eine Kohlenstoff-Kohlenstoff-Einfachbindung darstellt, die Linie |||||||||| für eine Kohlenstoff-Kohlenstoff-Einfachbindung steht, wobei dieses hydrierte Produkt der Dimerisation von $\alpha$-Methylstyrol mindestens eine Struktur aufweist, die durch mindestens eine der allgemeinen Strukturen definiert ist, welche aus der Gruppe ausgewählt ist, die die folgenden Formeln

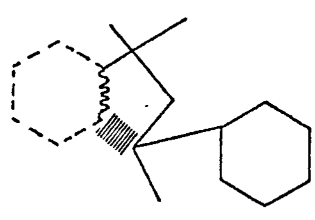 und 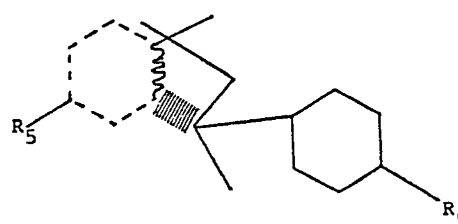

umfaßt, worin eine der Gruppen $R_5$ und $R'_5$ eine $C_1$—$C_4$-Alkylgruppe und die andere ein Wasserstoffatom oder beide Gruppen $R_5$ und $R'_5$ $C_1$—$C_4$-Alkylgruppen bedeuten und die gestrichelten Linien und die Wellenlinie für Kohlenstoff-Kohlenstoff-Einfachbindungen oder Kohlenstoff-Kohlenstoff-Doppelbindungen mit der Maßgabe stehen, daß, wenn der die Wellenlinie und die gestrichelten Linien umfassende Ring eine Doppelbindung aufweist, nur die Wellenlinie für eine Doppelbindung steht und wenn der die gestrichelten Linien und die Wellenlinie umfassende Ring mehr als eine Doppelbindung aufweist, dieser die gestrichelten Linien und die Wellenlinien umfassende Ring ein Benzolkern ist, und die Linie |||||||||| für eine Kohlenstoff-Kohlenstoff-Einfachbindung oder für keine Bindung steht, und

(B) ein Reaktionsprodukt, ausgewählt aus der Gruppe, die
(I) eines oder mehrere Produkte mit 20 Kohlenstoffatomen der Dimerisation
(i) eines monocyclischen Terpens mit zwei Kohlenstoff-Kohlenstoff-Doppelbindungen oder
(ii) zweier verschiedener monocyclischer Terpene mit zwei Kohlenstoff-Kohlenstoff-Doppelbindungen oder
(iii) eines bicyclischen Terpens mit einer Kohlenstoff-Kohlenstoff-Doppelbindung oder
(iv) zweier verschiedener bicyclischer Terpene mit jeweils einer Kohlenstoff-Kohlenstoff-Doppelbindung oder
(v) eines monocyclischen Terpens mit zwei Kohlenstoff-Kohlenstoff-Doppelbindungen und eines bicyclischen Terpens mit einer Kohlenstoff-Kohlenstoff-Bindung und
(II) Produkte der Hydrierung eines oder mehrere Produkte mit 20 Kohlenstoffatomen der Dimerisation von Terpenen und eines oder mehrerer Produkte der Hydrierung eines oder mehrerer Produkte mit 20 Kohlenstoffatomen der Dimerisation von Terpenen, umfaßt,

enthält, welche Mischung in ein natürliches Parfümöl oder ein synthetisches Parfümöl oder einen synthetischen chemischen Riechstoff oder eine Mischung aus mindestens zwei natürlichen Parfümölen oder eine Mischung aus mindestens zwei synthetischen Parfümölen oder eine Mischung aus mindestens zwei chemischen Riechstoffen oder eine Mischung aus mindestens einem natürlichen Parfümöl, mindestens einem synthetischen Parfümöl und mindestens einem chemischen Riechstoff unter Bildung einer kombinierten Parfüm-Komposition in einer einzigen flüssigen Phase in einer Menge zwischen 1 und 30 Gew.-Teilen pro 100 Gew.-Teile der Parfüm-Komposition in einer einzigen flüssigen Phase eingemischt werden kann.

9. Mischung nach Anspruch 8, dadurch gekennzeichnet, daß das Reaktionsprodukt (B) eine

Dimerisationsprodukt ist, das aus der Gruppe ausgewählt ist, welche die folgenden Produkte umfaßt:
Produkte der Dimerisation von $\alpha$-Pinen;
Produkte der Dimerisation von $\beta$-Pinen;
Produkte der Dimerisation von Kamphen;
Produkte der Dimerisation von d-Limonen;
Produkte der Dimerisation von Terebenthin;
hydrierte Produkte der Dimerisation von $\alpha$-Pinen;
hydrierte Produkte der Dimerisation von $\beta$-Pinen;
hydrierte Produkte der Dimerisaiton von Kamphen;
hydrierte Produkte der Dimerisation von d-Limonen;
hydrierte Produkte der Dimerisation von Terebenthin;
Mischungen dieser Dimerisationsprodukte und
Mischungen dieser hydrierten Dimerisationsprodukte.

10. Kombinierte Parfüm-Komposition mit einer einzigen flüssigen Phase, dadurch gekennzeichnet, daß sie mindestens ein natürliches Parfümöl, ein synthetisches Parfümöl oder einen chemischen Riechstoff oder eine Mischung aus natürlichen Parfümölen und synthetischen Parfümölen oder eine Mischung aus synthetischen Parfümölen und chemischen Riechstoffen enthält, mit denen etwa 1 bis 30 Gew-Teile mindestens eines geruchlosen Parfümerie-Verdünnungsmittels, bei dem es sich um eine Mischung nach Anspruch 8 handelt, pro 100 Gew.-Teile der kombinierten Parfüm-Komposition mit einer flüssige Phase innig vermischt sind.

**Claims**

1. A compounded single liquid phase perfumery composition, characterized in that it comprises (i) a perfumery used substance in undiluted form and in the alternative, alone or in admixture, (ii) a diterpene or a hydrogenated diterpene, or a mixture of a diterpene and a hydrogenated diterpene, and/or (iii) an alpha methyl styrene dimerization product and/or an hydrogenated alpha methyl styrene dimerization product and/or a dimerization product of an alpha methyl styrene methylated homologue and/or a dimerization product of an hydrogenated alpha methyl styrene methylated homologue, said dimerization products (ii) and/or (iii) being present in a proportion comprised between 1 and 30 parts by weight per 100 parts by weight of the compounded single liquid phase perfumery composition.

2. A perfumery composition according to claim 1, characterized in that it contains a natural perfumery essence or a synthetic perfumery essence or a synthetic olfactory chemical product or a mixture of at least two natural perfumery essences, or a mixture of at least two synthetic perfumery essences, or a mixture of at least two olfactory chemical products, or a mixture of at least one natural perfumery essence, at least one synthetic perfumery essence and at least one olfactory chemical product with which there is intimately admixed a reaction product selected from the group consisting of (A) one or more twenty carbon atom containing products of the dimerization of (i) one monocyclic terpene containing two carbon-carbon double bonds, or (ii) two different monocyclic terpenes containing two carbon-carbon double bonds, or (iii) one bicyclic terpene containing one carbon-carbon double bond, or (iv) two different bicyclic terpenes, each containing one carbon-carbon double bond, or (v) one monocyclic terpene containing two carbon-carbon double bonds and one bicyclic terpene containing one carbon-carbon double bond; (B) the products of the hydrogenation of one or more twenty carbon atom containing products of the dimerization of terpenes, and (C) mixtures of one or more twenty carbon atom containing products of the dimerization of terpenes, and of one or more products of the hydrogenation of one or more twenty carbon atom containing products of the dimerization of terpenes, said reaction product being present in a concentration of from about 1 up to 30 parts by weight, per 100 parts by weight of the compounded single liquid phase perfumery composition.

3. A composition according to claim 2, characterised in that the reaction product is a dimerization product selected from the group consisting of:
Dimerization products of alpha-pinene;
Dimerization products of beta-pinene;
Dimerization products of camphene;
Dimerization products of d-limonene;
Dimerization products of turpentine;
Hydrogenated dimerization products of alpha-pinene;
Hydrogenated dimerization products of beta-pinene;
Hydrogenated dimerization products of camphene;
Hydrogenated dimerization products of d-limonene;
Hydrogenated dimerization products of turpentine;
Mixture of these dimerization products and
Mixtures of said hydrogenated dimerization products.

4. A process for extending a perfumery product selected from the group consisting of natural perfumery essences, synthetic perfumery essences, synthetic olfactory chemical products, mixtures of

natural essences and synthetic essences, mixtures of natural essences, synthetic essences and olfactory chemical products and mixtures of synthetic essences and olfactory chemical products, without substantially altering the aroma thereof, characterised in that one admixes intimately, with from 70 up to 99 parts by weight of the perfumery product, from 1 up to 30 parts by weight of a reaction product which is either in admixture, or in a separate alternative formed by (A) dimerization products of (i) similar or different monocyclic ten carbon atom containing terpenes containing two carbon-carbon double bonds, or (ii) similar or different bicyclic ten carbon atom containing terpenes containing one carbon-carbon double bond, or (iii) a monocyclic terpene containing two carbon-carbon double bonds and a bicyclic terpene containing one carbon-carbon double bond, (B) hydrogenation products of said dimerization product, (C) products of alpha methyl styrene dimerization and/or hydrogenated products of alpha methyl styrene dimerization, said alpha methyl styrene dimerization products being defined by at least one generic structure selected from the group consisting of products having the formulas:

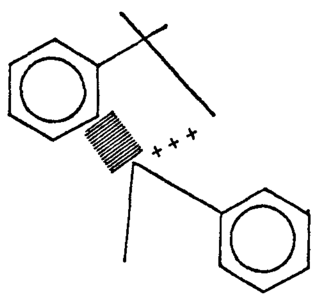

and

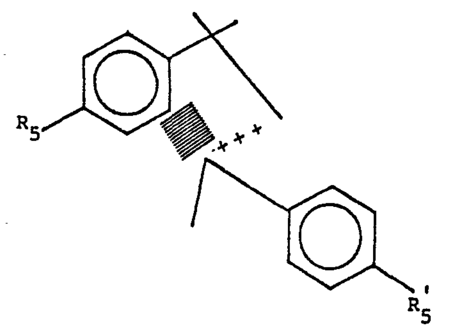

wherein one of $R_5$ and $R'_5$ is a methyl, the other being a hydrogen or $R_5$ and $R'_5$ are both methyl, wherein the line |||||||||||||||||| represents a carbon-carbon single bond or no bond, and wherein the line +++++ represents a carbon-carbon single bond or a carbon-carbon double bond, with the proviso that if the line +++++ is a carbon-carbon double bond, the line |||||||||||||||||| represents no bond, and if the line +++++ is a carbon-carbon single bond, the line |||||||||||||||||| is then a carbon-carbon single bond, the hydrogenated products of alpha methyl styrene dimerization having at least one of the generic structures selected from the group of products having the formulas:

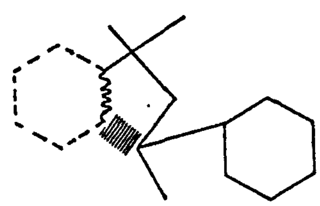

and

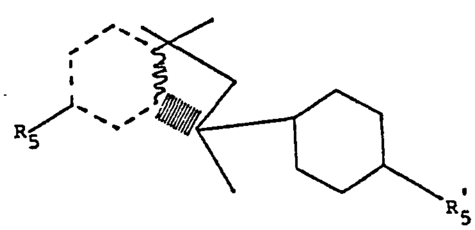

wherein one of $R_5$ and $R'_5$ is a methyl and the other a hydrogen, or $R_5$ and $R'_5$ are both a methyl, wherein the dashed lies and the wavy line represent carbon-carbon-single bonds or carbon-carbon double bonds, with the proviso that if there is one double bond present in the ring having a wavy line and dashed lines, only the wavy line is a double bond and if there exists more than one double bond in the ring containing the dashed lines and the wavy line, this ring containing the dashed lines and the wavy line is a benzene group, at least in those formulas the line |||||||||||||| represents either a carbon-carbon single bond, or no bond.

5. A composition according to claim 1, characterised in that it comprises a natural perfumery essence or a synthetic perfumery essence or a mixture of natural perfumery essence and synthetic perfumery essence or an olfactory chemical product with which there was intimately admixed from 1 to 30 parts by weight of at least one odorless perfumery diluant which is a dimerization product selected from the group consisting of products of the dimerization of alpha methyl styrene, products of the dimerization of a methyl homologue of alpha methyl styrene, hydrogenated products of the dimerization of alpha methyl styrene, and hydrogenated products of the dimerization of a methyl homologue of alpha methyl styrene, per 100 parts by weight of perfumery essence or olfactory chemical product, the products of the dimerization of the alpha methyl styrene having at least one structure selected from the group consisting of the following structures:

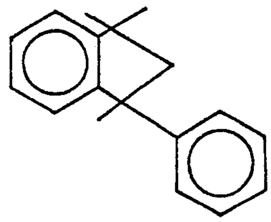

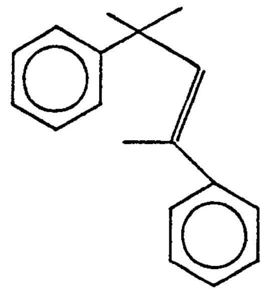

the hydrogenated product of the dimerization of alpha methyl styrene presenting at least one structure defined by the generic structure:

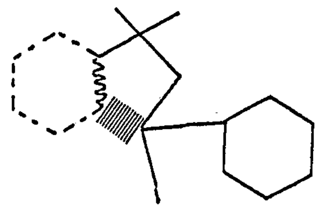

the product of the dimerization of the methyl homologue of alpha methyl styrene presenting at least one of the structures selected from the group consisting of the formulas:

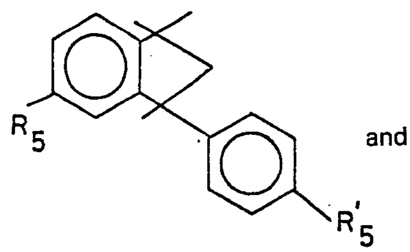

and

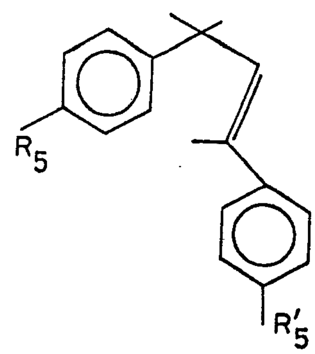

the hydrogenated product of the dimerization of the methyl homologue of alpha methyl styrene presenting at least one of the structures having the following formulas:

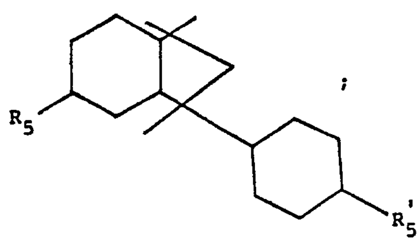

;

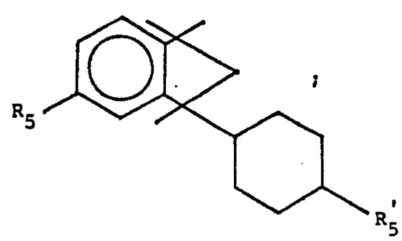

;

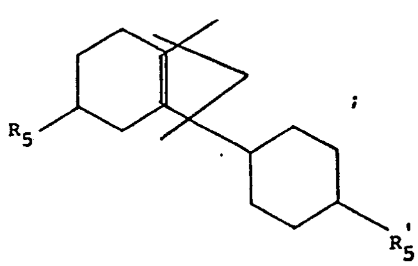

;

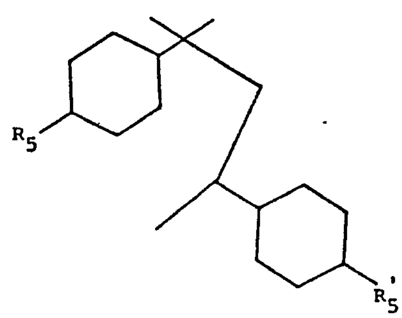

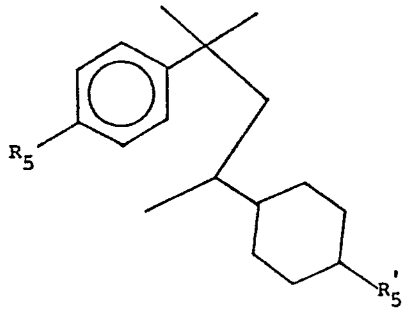

and

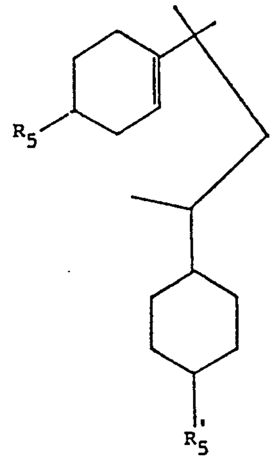

wherein one of $R_5$ or $R'_5$ is a methyl, the other being a hydrogen or $R_5$ and $R'_5$ are both a methyl group, wherein the dashed lines and wavy line represent carbon-carbon single bonds or carbon-carbon double bonds, with the proviso that if there exists one double bond in the ring having the dashed lines and the wavy line, only the wavy line represents a double bond, and if there exists more than one double bond in this ring containing the dashed lines and the wavy line, this ring is a benzene group, the line representing either a carbon-carbon single bond, or no bond.

6. A composition according to claim 5, characterised in that the dimerization product is an alpha methyl styrene dimer having the formula:

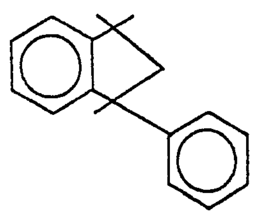

7. A composition according to claim 5, characterised in that the dimerization product is a hydrogenated dimerization product presenting at least one structure selected from the group consisting of products having the following formulas:

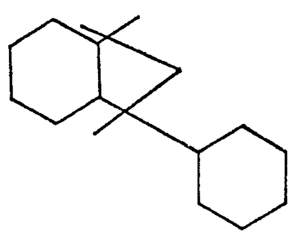

;

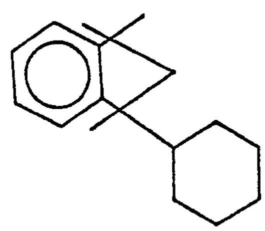

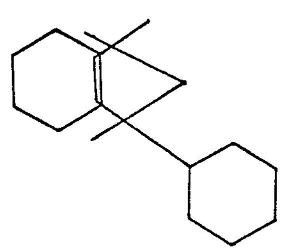

;

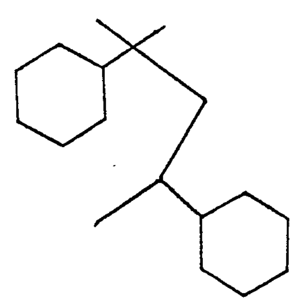

;

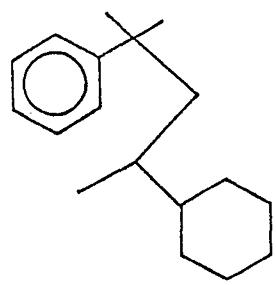

;

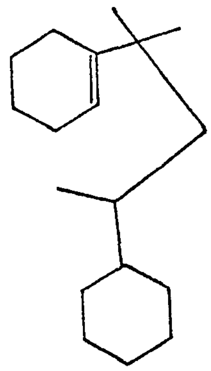

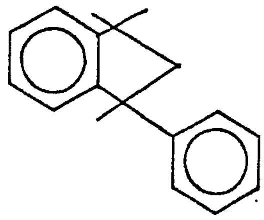

8. A mixture characterised in that it comprises (A) a product of the dimerization of an alpha methyl styrene or of a methyl or alkylated homologue with an other $C_2$—$C_4$ alkyl of this product or a mixture of the foregoing having one of the generic following structure:

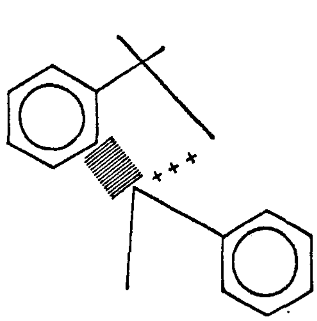

and

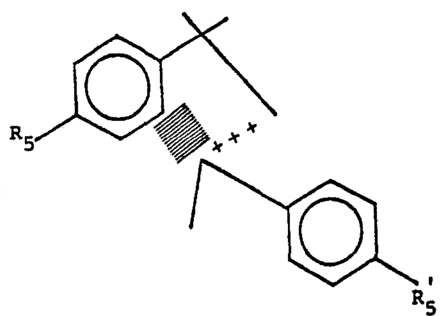

or hydrogenated derivatives thereof, or a mixture of the foregoing wherein one of $R_5$ and $R'_5$ is a $C_1$—$C_4$ alkyl, the other being a hydrogen, or $R_5$ and $R'_5$ are both a $C_1$—$C_4$ alkyl, wherein the line |||||||||||||| represents a carbon-carbon single bond or no bond and wherein the line +++++ represents a carbon-carbon single bond or a carbon-carbon double bond with the proviso that if the line +++++ is a carbon-carbon double bond, the line |||||||||||||| represents then no bond and if the line ++++ is a carbon-carbon single bond, the line |||||||||||||| is a carbon-carbon single bond, this hydrogenated product of the dimerization of alpha methyl styrene presenting at least one structure defined according to at least one of the generic structures selected from the group consisting of the formulas:

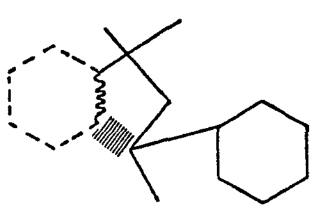

and

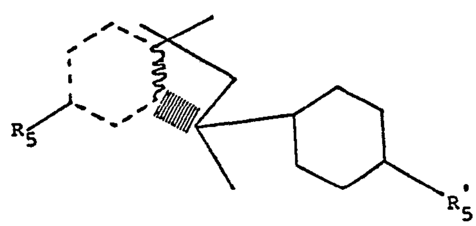

wherein one of $R_5$ and $R'_5$ is a $C_1$—$C_4$ alkyl, the other being a hydrogen, or each of $R_5$ and $R'_5$ represents a $C_1$—$C_4$ alkyl, wherein the dashed lines and the wavy line represent carbon-carbon single bonds or carbon-carbon double bonds, with the proviso that if there exists one double bond present in the ring having the wavy line and the dashed lines, only the wavy line represents a double bond, and if there exists more than one double bond present in the ring containing the dashed lines and the wavy line, this ring containing the dashed lines and the wavy line is a benzene group, and wherein the line represents either a carbon-carbon single bond or no bond, and (B) a reaction product selected from the group consisting of (I) one or more twenty carbon atom containing products of the dimerization of, (i) one monocyclic terpene containing two carbon-carbon double bonds, or (ii) two different monocyclic

terpenes containing two carbon-carbon double bonds, or (iii) one bicyclic terpene containing one carbon-carbon double bond, or (iv) two different bicyclic terpenes, each containing one carbon-carbon double bond, or (v) one monocyclic terpene containing two carbon-carbon double bonds and one bicyclic terpene containing one carbon-carbon double bond; (II) products of the hydrogenation of one or more twenty carbon atom containing products of the dimerization of terpenes, and one or more products of the hydrogenation of one or more products of the dimerization of twenty carbon atom containing terpenes, said mixture being intended to be incorporated in a natural perfumery essence or a synthetic perfumery essence or a synthetic olfactory chemical product, or a mixture of at least two natural perfumery essences, or a mixture of at least two synthetic perfumery essences, or a mixture of at least two olfactory chemical products, or a mixture of at least one natural perfumery essence, at least one synthetic perfumery essence and at least one olfactory chemical product to form a single liquid phase compounded perfumery composition, in a proportion included between 1 and 30 parts by weight per 100 parts by weight of the single liquid phase perfumery composition.

9. A mixture according to claim 8, characterised in that the reaction product (B) is a dimerization product selected from the group consisting of the following products:

Dimerization products of alpha-pinene;
Dimerization products of beta-pinene;
Dimerization products of camphene;
Dimerization products of d-limonene;
Dimerization products of turpentine;
Hydrogenated dimerization products of alpha-pinene;
Hydrogenated dimerization products of beta-pinene;
Hydrogenated dimerization products of camphene;
Hydrogenated dimerization products of d-limonene;
Hydrogenated dimerization products of turpentine;
Mixtures of said dimerization products; and
Mixtures of said hydrogenated dimerization products.

10. A single liquid phase compounded perfumery composition, characterised in that it contains at least one natural perfumery essence, one synthetic perfumery essence or one olfactory chemical product or a mixture of natural perfumery essences and synthetic perfumery essences, or a mixture of synthetic perfumery essences and olfactory chemical products, with which there is intimately admixed from about 1 up to 30 parts by weight of at least one odorless perfumery diluant which is formed by a mixture according to claim 8 per 100 parts by weight of said compounded single liquid phase perfumery composition.

FIG.I

PROFIL G L C DE L'EXEMPLE I, FRACTION 19

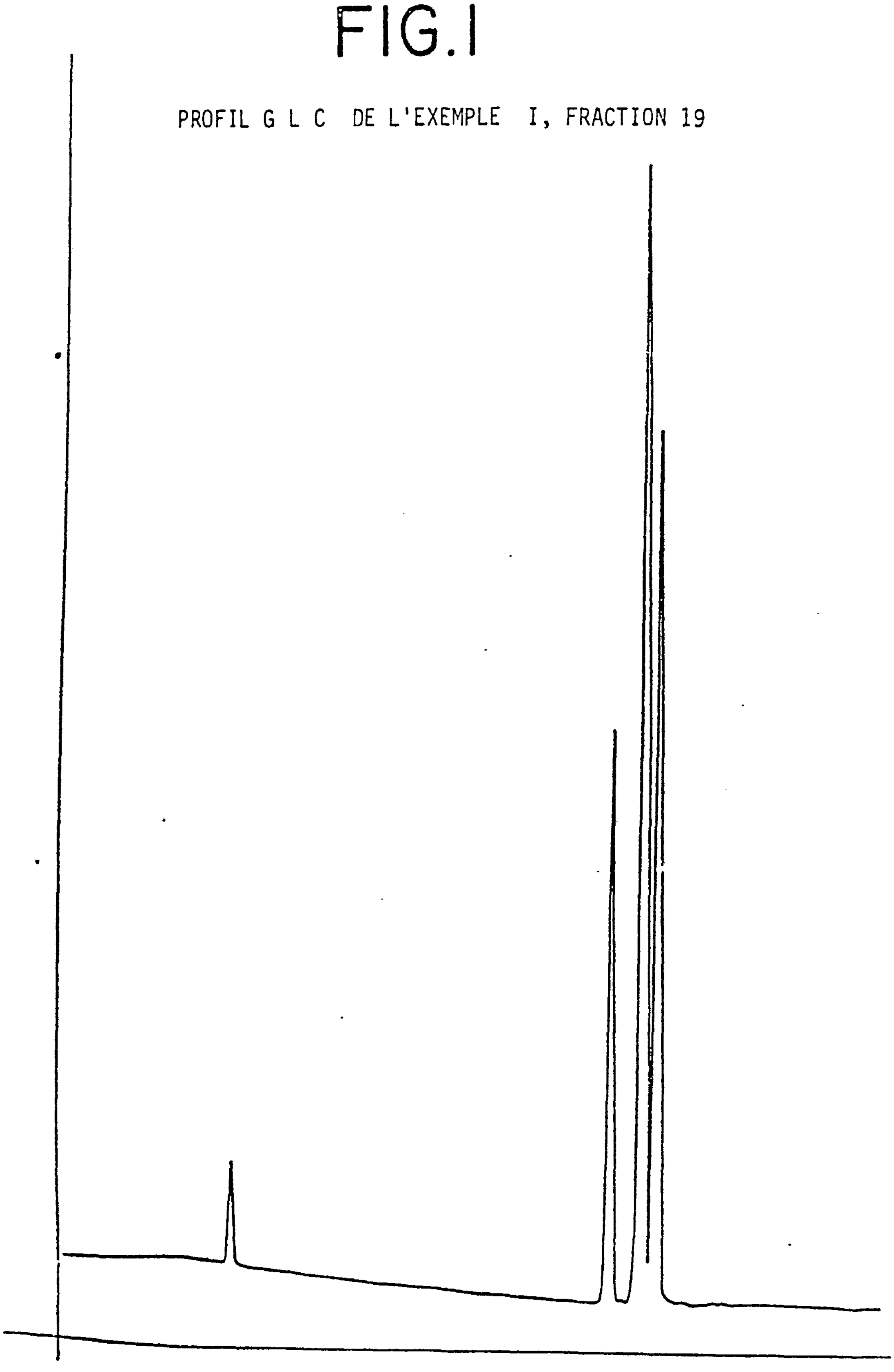

# FIG.2

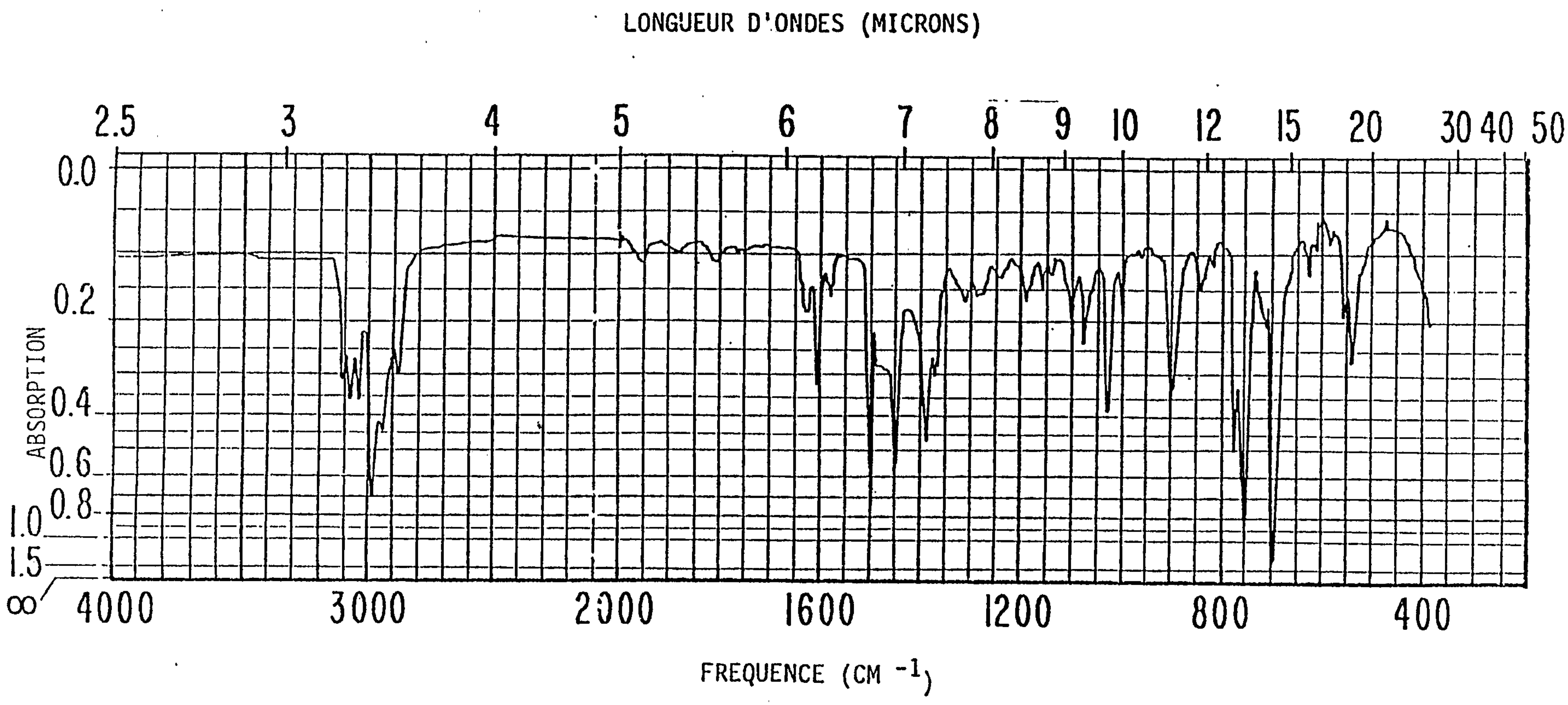


SPECTRE IR DE L'EXEMPLE I, FRACTION 19

FIG. 3

SPECTRE N M R DE L'EXEMPLE I, FRACTION 19

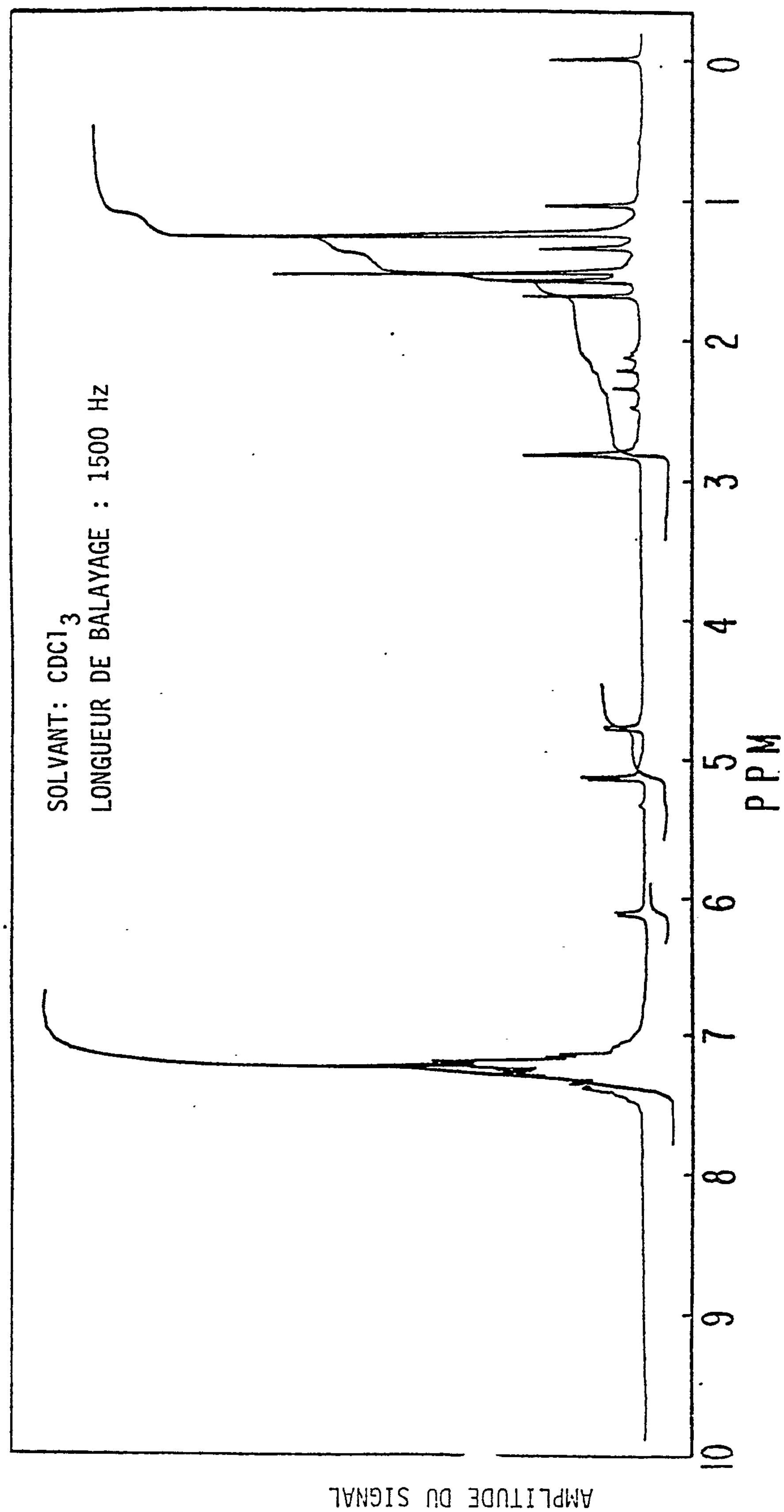

FIG. 4

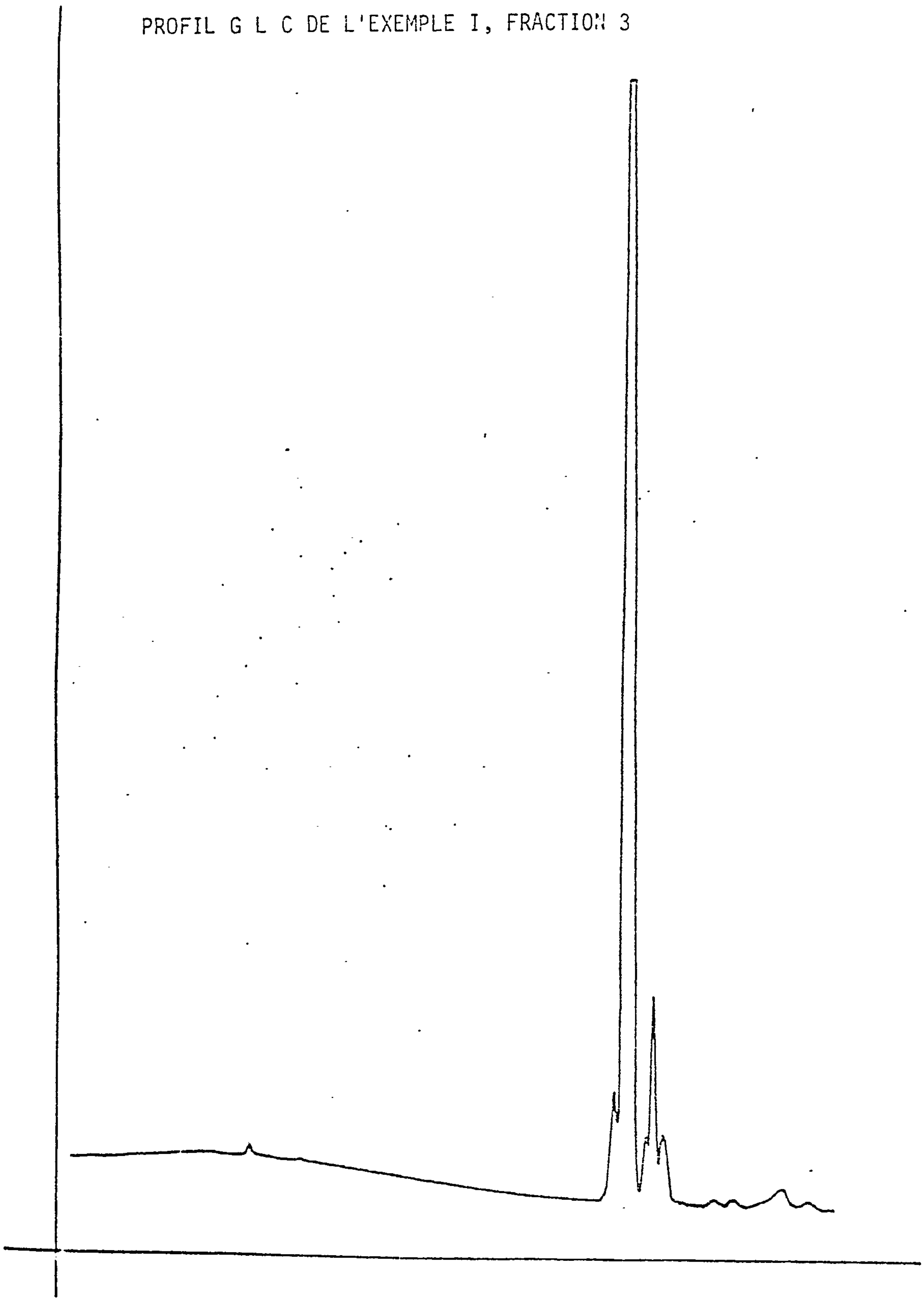

FIG.5

LONGUEUR D'ONDES (MICRONS)

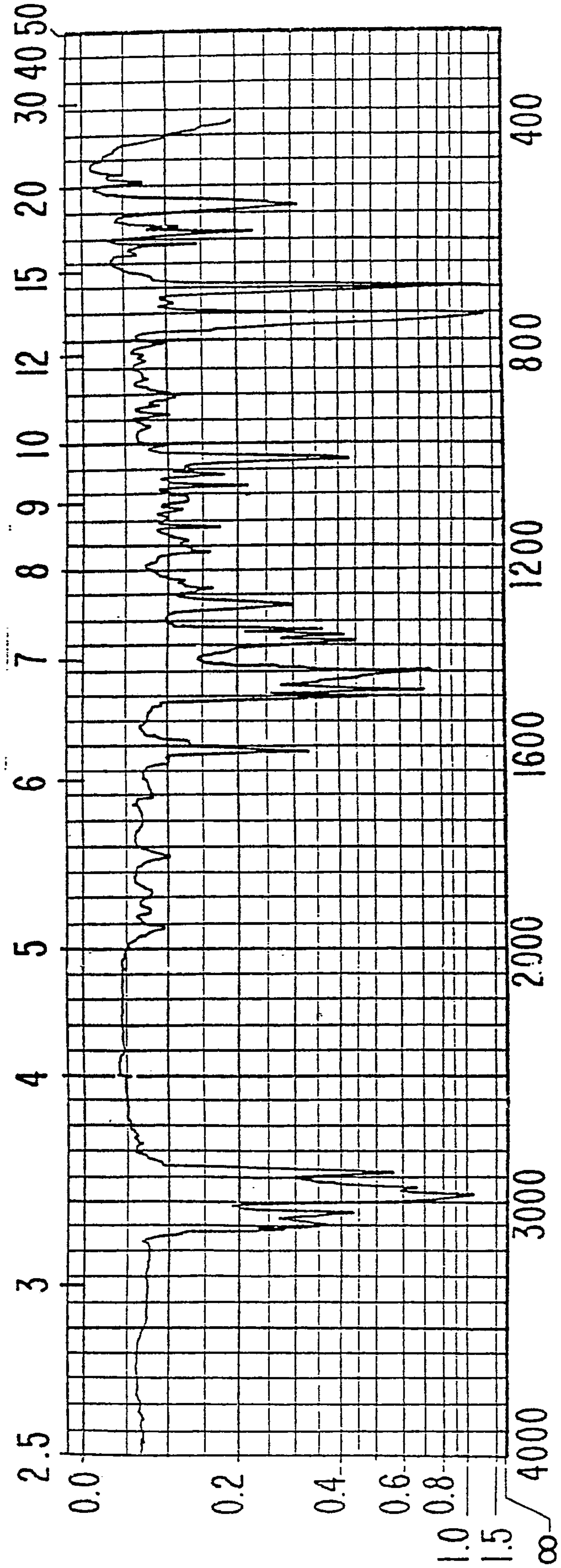


FREQUENCE ($CM^{-1}$)

SPECTRE IR DE L'EXEMPLE I, FRACTION 3 ET DE L'EXEMPLE III

FIG.6

SPECTRE N M R DE L'EXEMPLE 1, FRACTION 3 ET DE L'EXEMPLE III

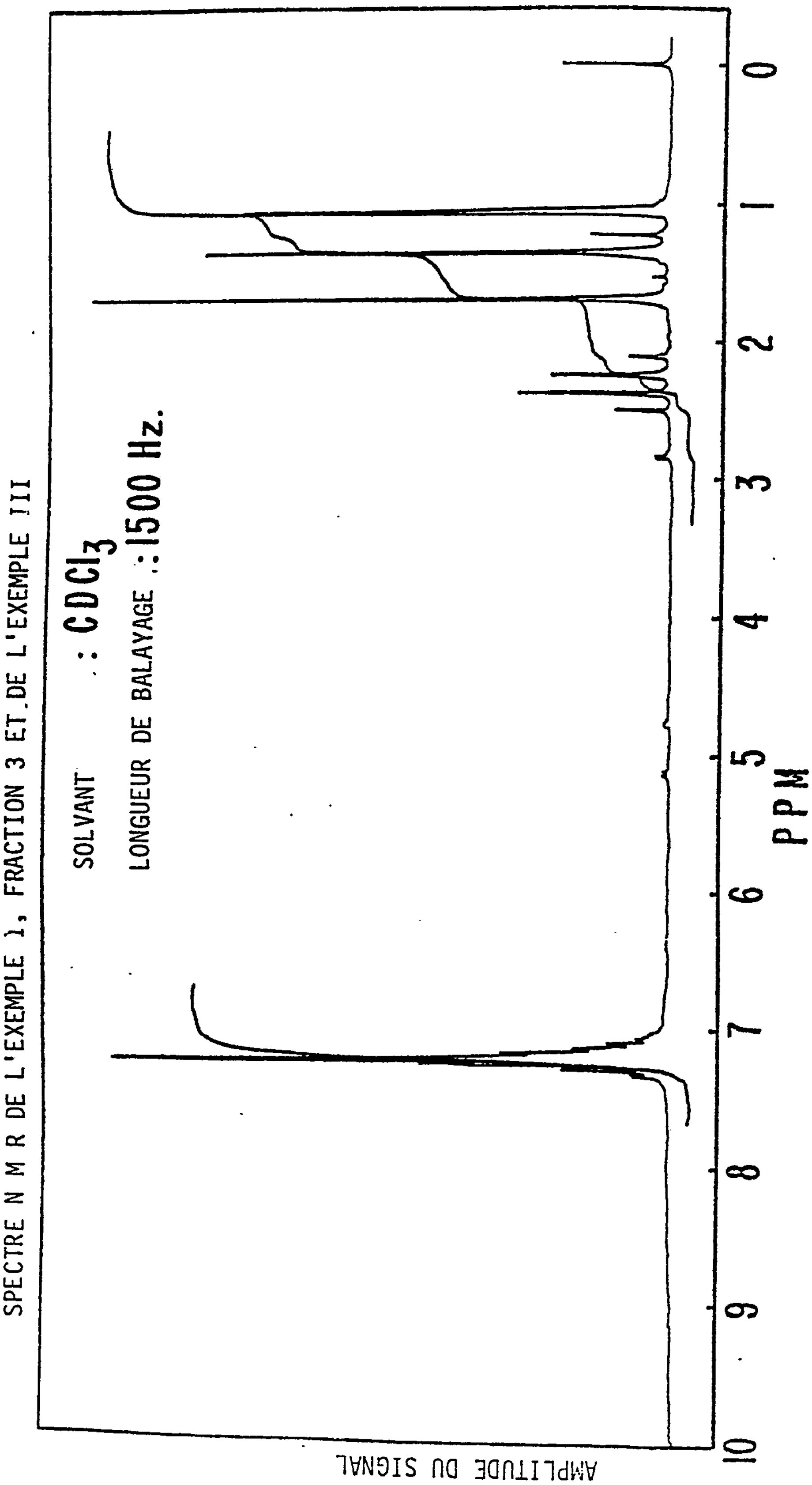

FIG. 7

SPECTRE DE MASSE DE L'EXEMPLE I, FRACTION 3 ET DE L'EXEMPLE III

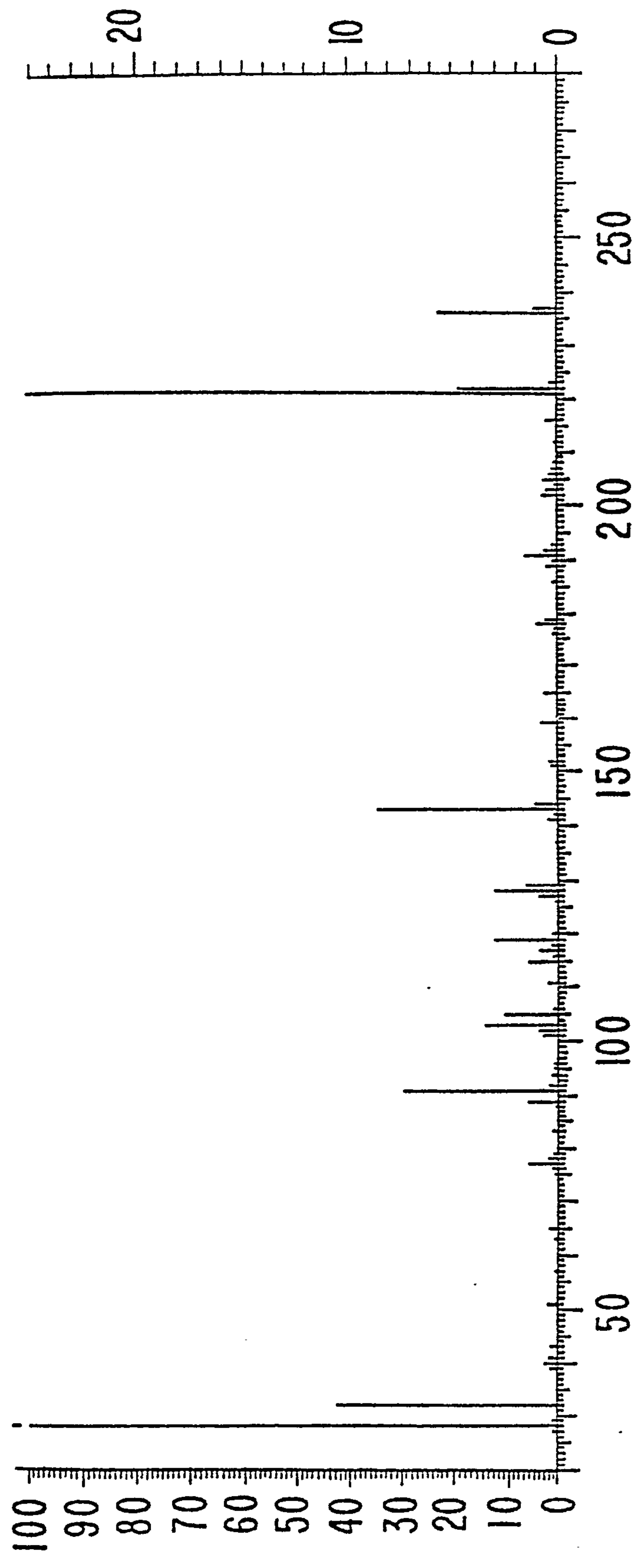

FIG. 8

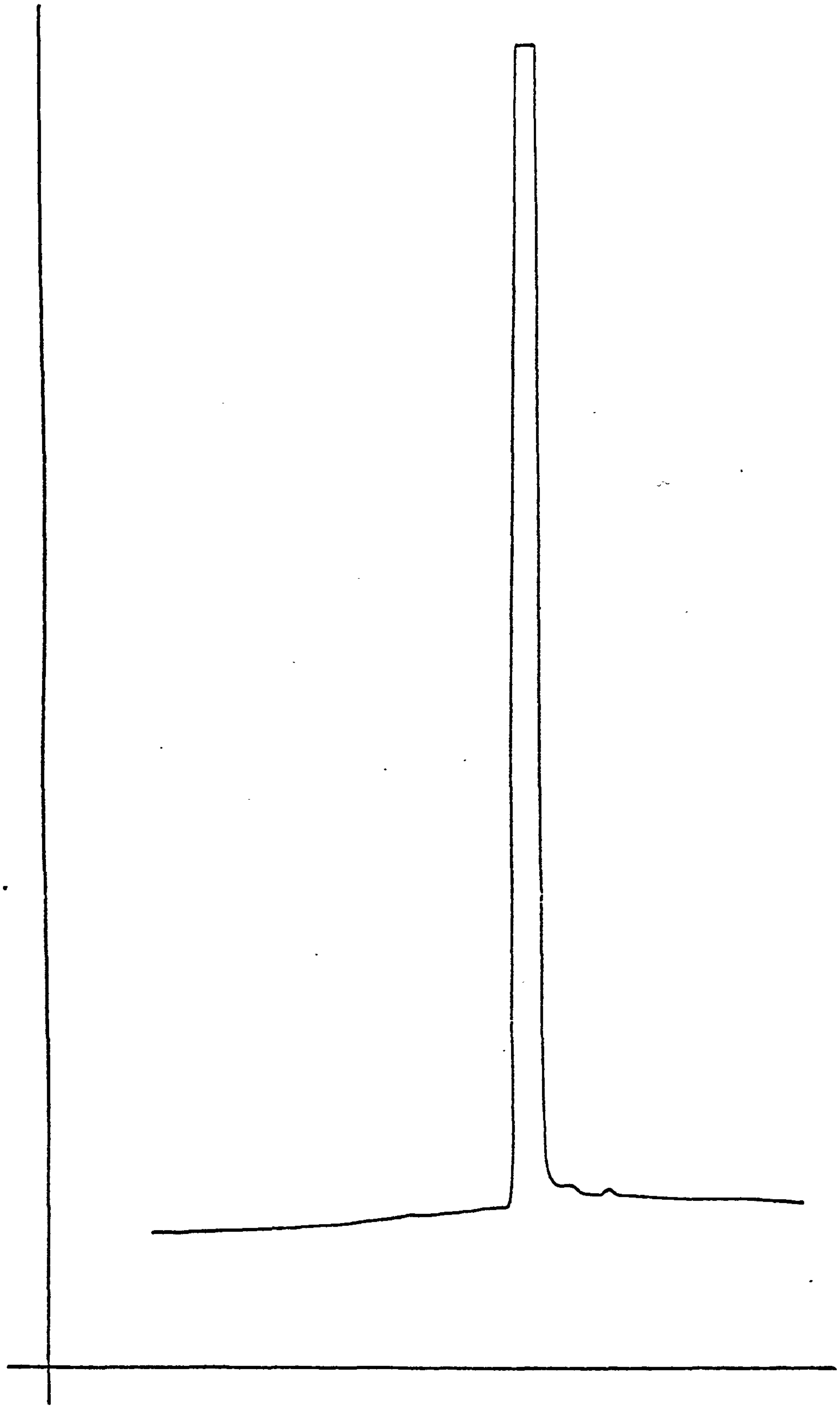

PROFIL G L C DE L'EXEMPLE III, FRACTIONS 9-12

FIG.9

PROFIL G L C DE L'EXEMPLE IV (A)

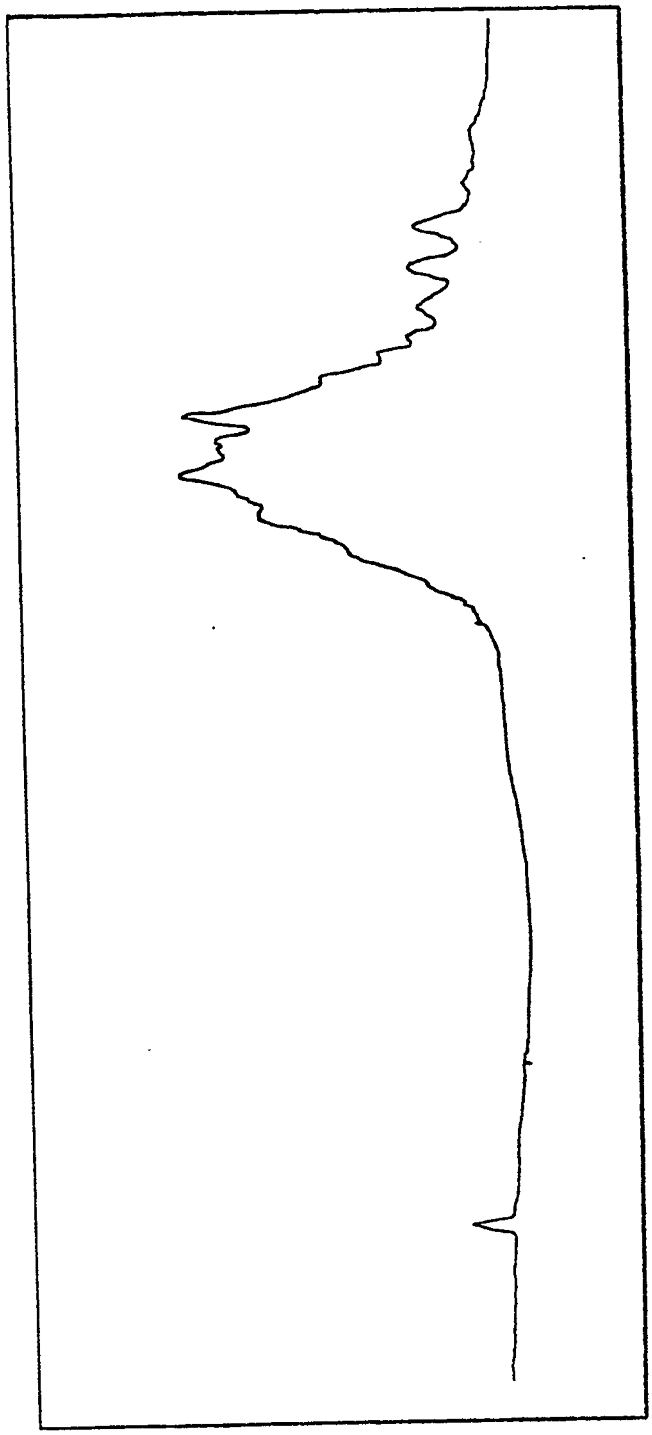

FIG.10

PROFIL G L C DE L'EXEMPLE IV (B)

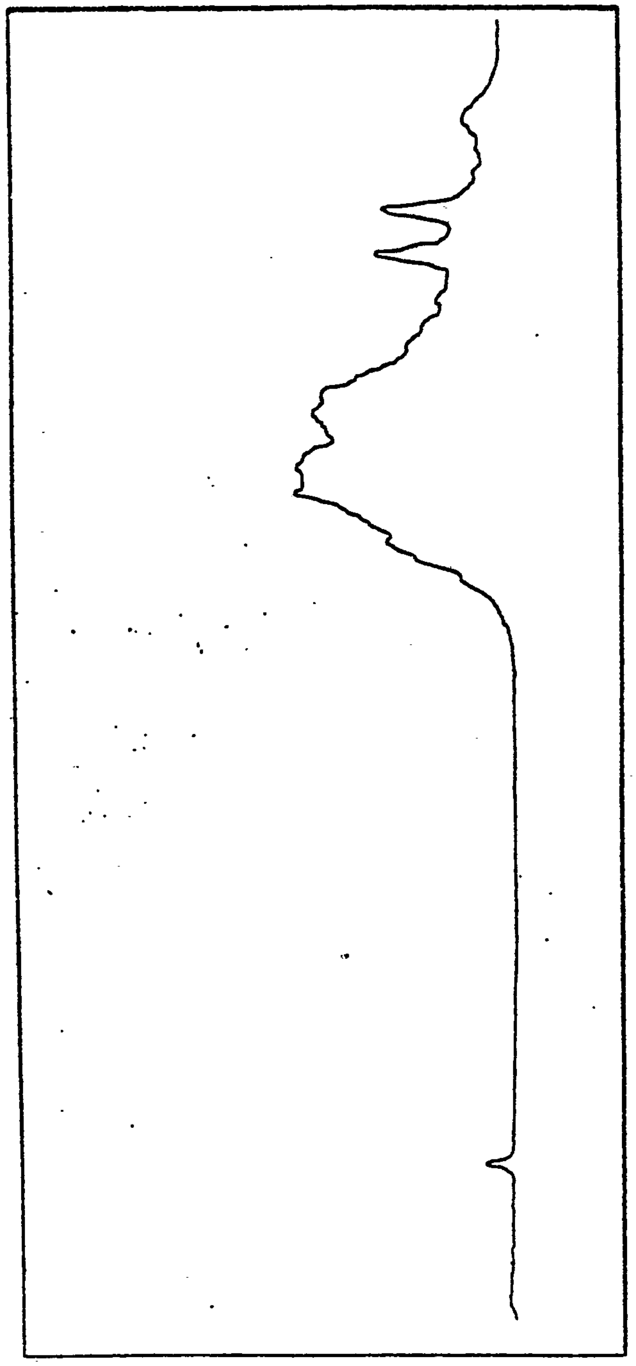

FIG.II

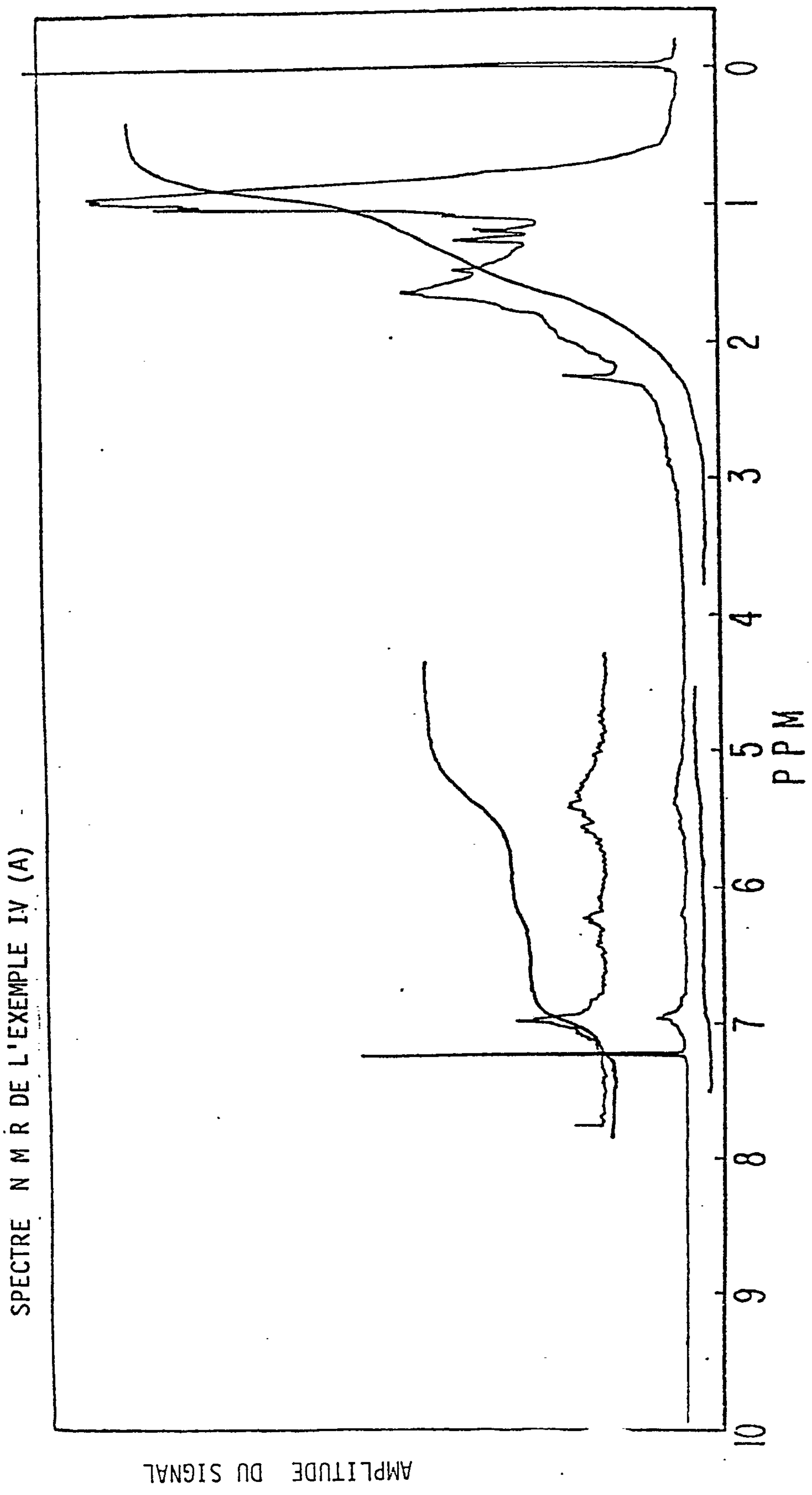
SPECTRE N M R DE L'EXEMPLE IV (A)
0
1
2
3
4
5
6
7
8
9
10
P P M
AMPLITUDE DU SIGNAL

FIG.12

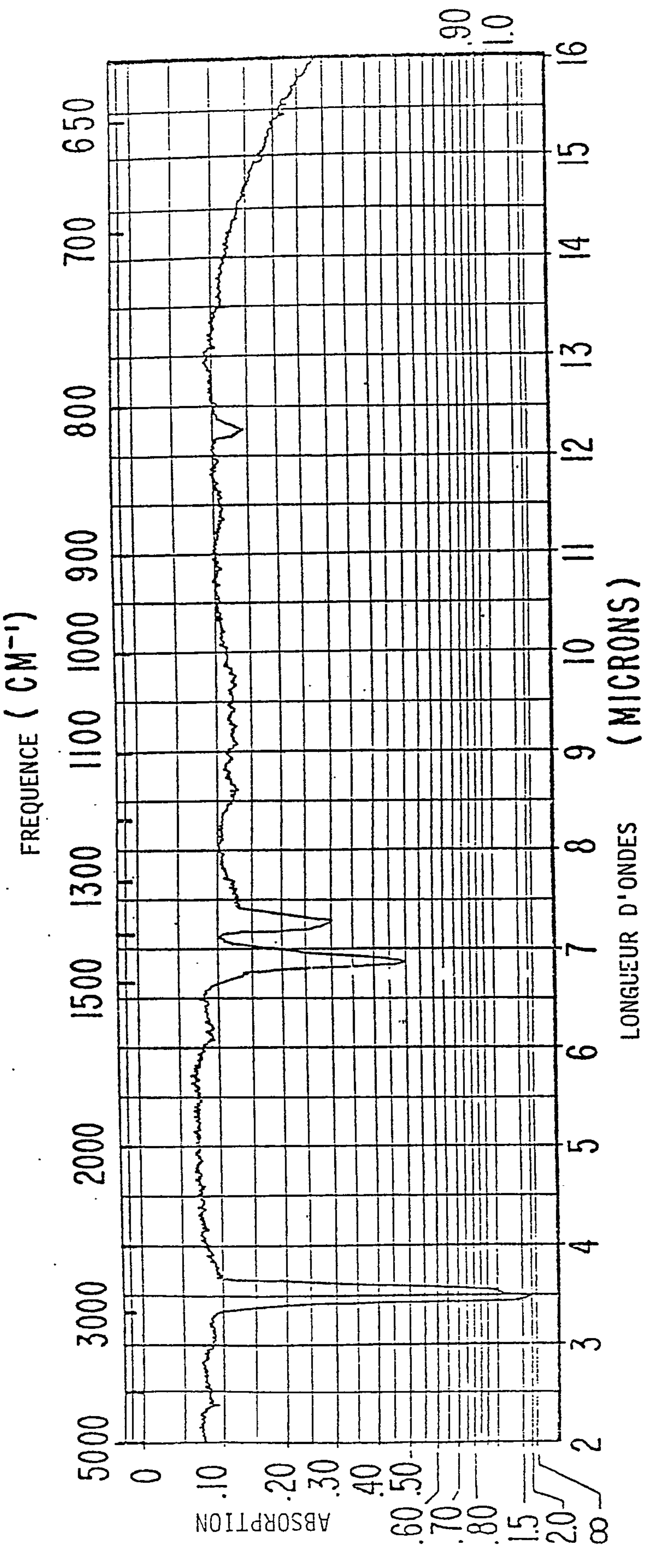


SPECTRE I R DE L'EXEMPLE IV (A)

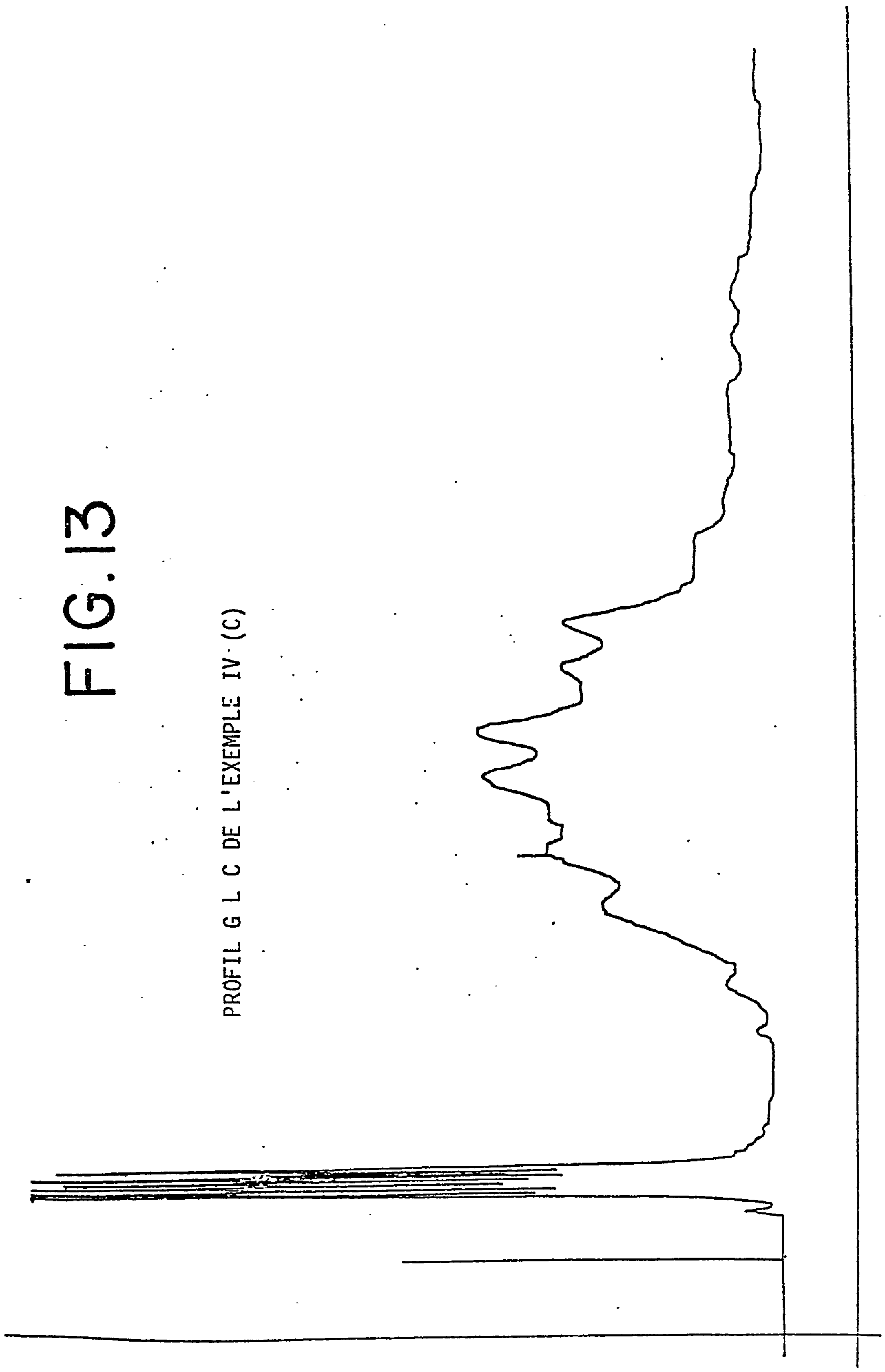

FIG. 13

PROFIL G L C DE L'EXEMPLE IV (C)

FIG.14

SPECTRE N M R DE L'EXEMPLE IV (C)

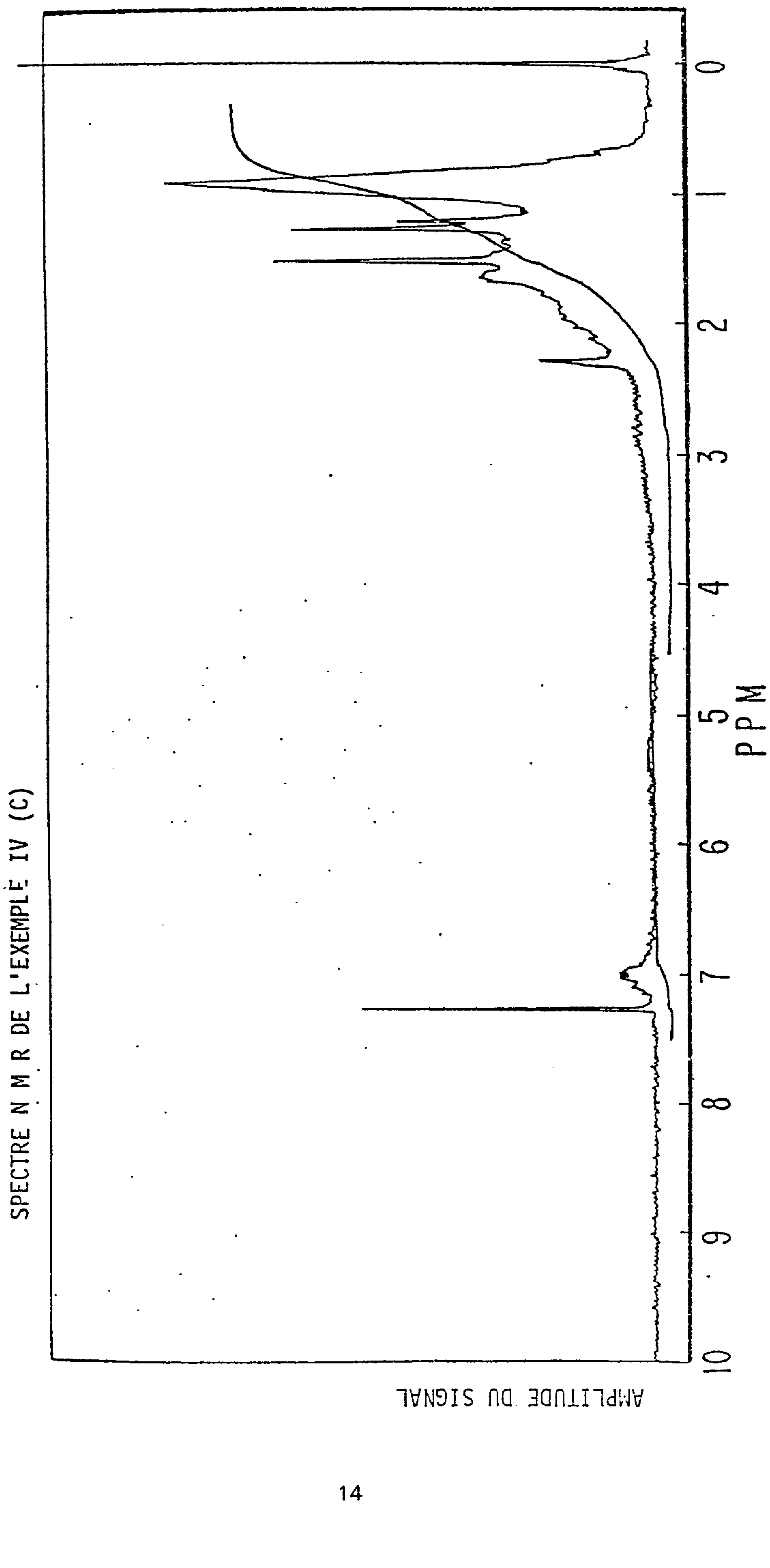

FIG.15

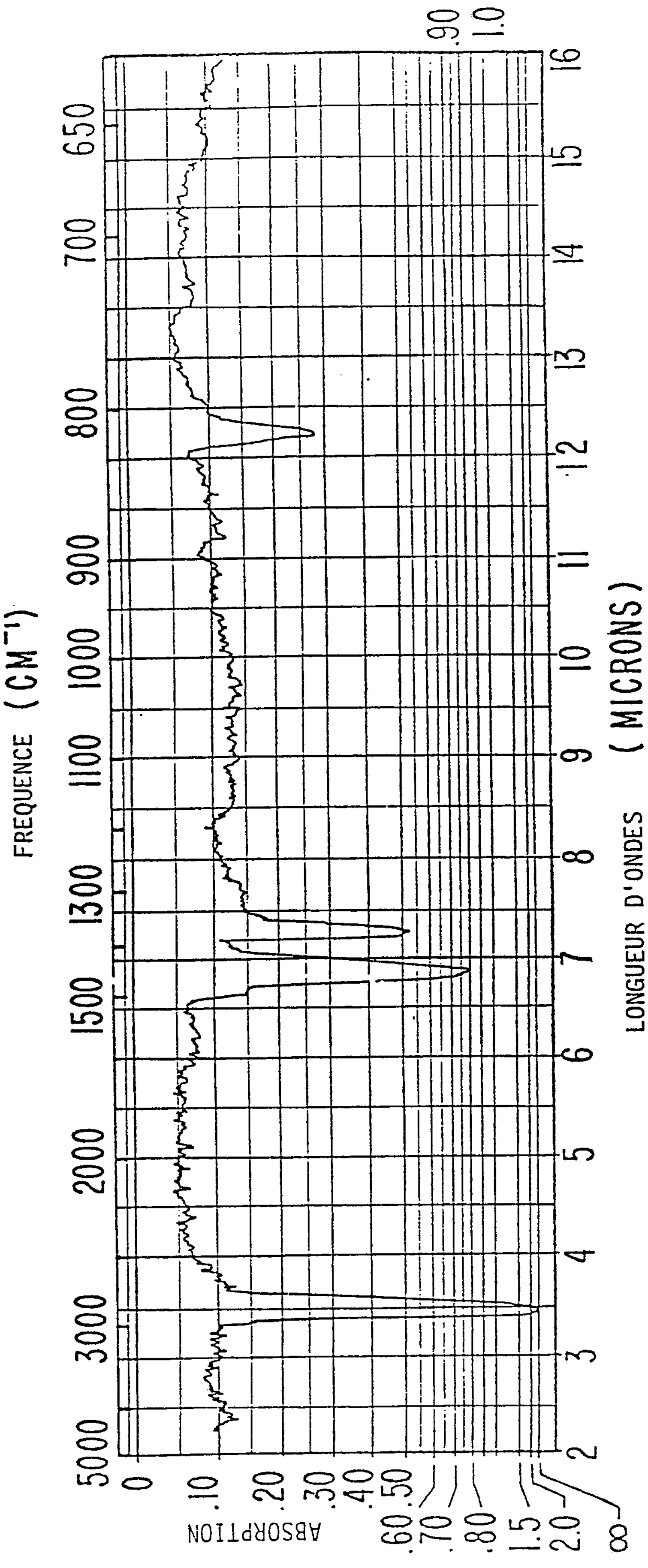


SPECTRE I R DE L'EXEMPLE IV (C)